# EUROPEAN PATENT APPLICATION

(11) **EP 4 846 968 A1**
(43) Date of publication of application: **02.12.2026**
(21) Application number: 25178894.9
(22) Date of filing: 26.05.2025
(51) Int. Cl.: A61B 18/20, A61B 18/22, A61B 18/00

(54) **LASER DEVICE**

(71) Applicant: BTL Medical Solutions a.s., Staré Mesto 110 00 Praha 1 (CZ)
(72) Inventor: SCHWARZ, Tomas, 169 00 Prague (CZ); CHLOUBA, Martin, 147 00 Prague (CZ); VOZAR, Michal, 902 01 Pezinok (SK); CERNY, Pavel, 250 66 Zdiby-Premysleni (CZ); REHAKOVA, Veronika, 506 01 Jicin (CZ)
(74) Representative: K&L Gates LLP

(57) **Abstract**

The present disclosure refers to a laser apparatus for treatment of patient tissue, comprising a plurality of laser sources for generating a plurality of laser beams, the plurality of laser sources comprising a first laser source configured to generate a first laser beam of a first wavelength configured to provide non-ablation of a tissue of the patient and a second laser source configured to generate a second laser beam of a second wavelength configured to provide ablation of the tissue of the patient, wherein the first wavelength is different than the second wavelength. Moreover, the disclosure further refers to a method for irradiating a target zone.

## Description

### Background of the invention

The field of medicine, e.g. aesthetic, dentistry, ophthalmology and so on, has witnessed rapid advancements over the past few decades, with a significant focus on the development of non-invasive and minimally invasive techniques, for example for use in dermatology. Among these technologies, devices based on electromagnetic radiation (EMR) stand out due to their versatility and efficacy. Such treatments include, but are not limited to, skin rejuvenation, body contouring, cosmetic enhancement, unwanted hair removal, removal of vascular lesions, acne treatment, treatment of cellulite, treatment of pigmented lesions, treatment of psoriasis, skin ablation, skin coagulation, other procedure for improvement of visual appearance, and others.

Electromagnetic radiation is often used in the form of radiofrequency, microwaves, or optical spectrum. Optical treatment involves irradiating a target area to be treated with radiation at one or more wavelengths preferentially absorbed by chromophores in a target area.

Devices often contain a coherent or a non-coherent form of radiation, e.g. light. An advantage of treating a body using electromagnetic radiation is the ability to treat almost any part of the body non-invasively and non-surgically. These technologies offer promising alternatives to traditional surgical procedures, aiming to reduce recovery times, minimize side effects, and cater to the nuanced demands of a diverse patient demographic.

One type of EMR device is a laser device. A laser emits light radiation through a process known as stimulated emission. The type of radiation produced by a laser is part of the electromagnetic spectrum and is typically in the visible, infrared, or ultraviolet range. The main characteristics of laser are monochromaticity, coherence and directionality.

Laser light is monochromatic, meaning it consists of a single color or wavelength. This is in contrast to white light, which contains a broad spectrum of wavelengths. The monochromatic nature of laser light is crucial for targeting specific types of tissues in medical and cosmetic procedures, as different tissues absorb specific wavelengths differently.

The light emitted by a laser is coherent, with all the light waves having a constant phase difference between them. This coherence allows laser beams to be focused to a very tight spot, enabling high precision in applications ranging from cutting and engraving materials in industrial processes to targeting small areas in medical treatments.

Laser light is highly directional, meaning it spreads out very little compared to other light sources. This allows laser beams to remain narrow over great distances, which is why lasers can be used for applications requiring precise targeting, such as in surgical procedures or in industry.

Due to their coherence and directionality, lasers can concentrate a lot of energy into a very small area. This high intensity is beneficial for applications requiring significant amounts of energy, for example to cut, burn, ablate, or vaporize tissue or materials.

One advantage of laser treatment is its precision and selectivity, precise control of the penetration depth, the predictability and consistency of its results, efficiency, and versatility.

Lasers can be highly precise, targeting specific depths and structures within the skin without affecting surrounding tissues. This precision is due to the laser's ability to produce a coherent, monochromatic beam of light that can be focused to a very fine point. Different lasers can be selected based on their wavelength to specifically target melanin, water, or hemoglobin, making them highly effective for treatments like pigment correction, skin resurfacing, and vascular lesions, respectively.

The wavelength of laser light determines its depth of penetration into the skin, allowing for targeted treatments of conditions at specific layers of the skin. This level of control is crucial for procedures like laser skin resurfacing, where the ability to precisely remove layers of skin can significantly affect the outcome and recovery time. Due to the precise control over the laser parameters (wavelength, pulse duration, energy), treatments can be highly reproducible, leading to more predictable and consistent results. This predictability is especially important for treatments that require precision, such as facial rejuvenation and scar reduction.

A wide range of laser types (e.g., CO₂, Nd:YAG, Er:YAG, Er:Glass, Alexandrite) allows for a broad spectrum of treatments with applications in skin rejuvenation, pigmentation correction, vascular treatments, hair removal, and acne treatment. This versatility is augmented by the ability to adjust laser parameters to suit specific treatment goals and patient needs.

Certain laser treatments can effectively stimulate the production of collagen and elastin without the need for direct contact or mechanical action on the skin, as is necessary with some RF and ultrasound treatments. This stimulation can lead to improvements in skin texture and firmness over time.

While these characteristics are useful for many dermatological therapies, there are still some significant limitations of the known devices.

One disadvantage is that a laser beam delivering only a single wavelength at a time might not be capable of achieving the desired result. There are no devices available in the current market that can deliver a combination of different laser wavelengths.

Another disadvantage is that laser sources take time to settle at their final power and become consistent. When a laser source ramps up to its target power, it initially emits fluctuating or unstable energy. The energy delivered to tissue or a target may be non-uniform, leading to under-treatment (ineffective therapy), over-treatment (tissue damage, burns), or a patient discomfort. Thus, it is necessary to devise a method of controlling laser sources such that they deliver almost constant laser beams throughout the whole treatment.

In view of the aforementioned technical challenges, the present devices and methods provide a laser apparatus configured to improve therapeutic outcomes. The device comprises dual laser chambers to generate both coagulation and ablation laser beams, which are then combined into a single combined, multi-wavelength beam. This single combined beam may be delivered in a fractional treatment mode.

Additionally, the laser chambers may be operated in a pre-heating or heating mode to ensure the performance of the laser beams stays consistent throughout the treatment.

### Summary of the invention

The present invention relates to an advanced dermatological laser apparatus that significantly improves upon existing technologies by providing a multifunctional, highly adaptable, and efficient system for aesthetic, medical, and surgical treatments. The apparatus addresses several shortcomings of the prior art by integrating multiple laser sources into a single device, capable of producing both ablative and non-ablative laser beams. These beams are combined into a spatially coordinated, multi-wavelength output, allowing for simultaneous, successive, or overlapping operation modes. This provides unprecedented flexibility in tailoring treatments to various skin conditions, tissue types, and clinical goals.

In one aspect, the disclosed device may have the ability to deliver laser beam pulses in fractional or full-field modes using interchangeable applicators. In fractional mode, the device subdivides the laser beam into multiple micro beams, forming micro treatment/thermal zones (MTZs) surrounded by unaffected tissue. This approach promotes faster healing, reduces posttreatment downtime, and minimizes the risk of adverse effects. The fractional energy distribution is achieved using micro-lens arrays, diffractive optics, or custom reflectors, depending on the clinical application. In full-field mode, the laser beam is delivered uniformly across a treatment area, suitable for example for resurfacing and deep tissue therapies.

The apparatus may also include intelligent control systems, such as a user interface and control unit capable of adjusting fluence, pulse duration, repetition rate, and treatment protocols in real-time. The system may be further enhanced with preheating and heating mechanisms for maintaining energy consistency, optical output measurement units for real-time calibration, and a collimated beam architecture to ensure precise targeting. Quick-connect applicators, integrated vacuum and cooling systems, and an ergonomic waveguide enable user-friendly operation and patient comfort.

By combining flexibility, precision, and efficiency, the present invention sets a new standard in laser-based dermatological and medical treatments. It is designed for a wide range of applications including skin rejuvenation, scar and pigmentation treatment, vascular and pigmented lesions, tattoo removal, and even surgical, dental, gynecological, and ophthalmological procedures, providing an all-in-one solution for modern clinical practice.

In one aspect, a laser apparatus for treatment of patient tissue may comprise a plurality of laser sources for generating a plurality of laser beams. The plurality of laser sources comprises a first laser source configured to generate a first laser beam of a first wavelength configured to provide non-ablation of a tissue of the patient and a second laser source configured to generate a second laser beam of a second wavelength configured to provide ablation of the tissue of the patient with the first wavelength being different than the second wavelength. The laser apparatus may further comprise a laser beam combiner coupled to the plurality of the laser sources, said laser beam combiner being configured to combine the plurality of laser beams of the predetermined wavelengths into a single combined laser beam of multiple wavelengths.

In one aspect, the single combined laser beam may mean coaxial beams of multiple wavelengths that share the same optical axis. This means that the plurality of laser beams are aligned concentrically, such that they follow the same (optical) path through space (at least from the beginning of the single combined laser beam). This enables precise overlapping delivery of different wavelengths to the exact same point, minimizes spatial misalignment and ensures energy from multiple sources is focused on the same tissue region (target zone), which may maximize combined effects. The plurality of laser beams may be provided in single combined laser beam simultaneously, in overlap, or sequentially.

In another aspect, the single combined laser beam may mean beam superposition referring to the overlapping of two or more laser beams in the same physical space, so that their intensities and energy profiles add up at the point of contact with tissue (e.g. at the target zone).

Yet in another aspect, the single combined laser beam may mean at least partially coaxial and at least partially superposed beams.

Furthermore, the laser apparatus may comprise a collimator, configured to collimate (e.g. to align and maintain in an at least substantially parallel form or in a parallel form) the single combined laser beam. The apparatus may further comprise a movable waveguide coupled (physically or optically coupled) to the collimator on a first side and configured to deliver (transmit) the single combined laser beam to an applicator coupled to a second side of the waveguide (opposite side of the first side). The applicator may be configured to deliver the single combined laser beam to a target zone of the patient tissue, wherein the plurality of the laser beams in the single combined laser beam irradiates the same area of the target zone or irradiates the target zone such that the centers of the plurality of laser beams of the single combined laser beam are aligned to a common point on the target zone and their cross-sections areas overlaps on the target zone. Lastly, the laser apparatus may comprise a control unit in communication with the plurality of laser sources, said control unit being configured to control the plurality of laser sources in a manner that the plurality of laser beams combined into the single combined laser beam are delivered to the target zone of tissue in a simultaneous or overlapping method, wherein each laser source of the plurality of the laser sources is configured to generate a pulsed laser beam, and wherein the delivery of the plurality of laser beams in the simultaneous or overlapping method facilitates a combined effect of the effects of each individual laser beam of the plurality of laser beams.

In another aspect, a laser apparatus for treatment of patient tissue may comprise a first laser source configured to generate a non-ablative laser beam, a second laser source configured to generate an ablative laser beam, an optical assembly, and a control unit.

The first laser source may comprise a first flash lamp charger electrically coupled to a first flash lamp and a first laser chamber comprising the first flash lamp and a first laser rod. The first flash lamp may be configured to generate a first heat flash and a first therapeutic flash when energized by the first flash lamp charger, wherein the first heat flash and the first therapeutic flash are broad-spectrum, and wherein the first heat flash is different than the first therapeutic flash. The first laser rod may be configured to generate a therapeutic non-ablative laser beam having a wavelength in a range of 1200 nm to 1650 nm, when energized by the first therapeutic flash.

**The** second laser source may comprise a second flash lamp charger electrically coupled to a second flash lamp and a second laser chamber comprising the second flash lamp and a second laser rod. The second flash lamp may be configured to generate a second therapeutic flash when energized by the second flash lamp charger, wherein the second therapeutic flash is broad-spectrum. The second laser rod may be configured to generate a therapeutic ablative laser beam having a wavelength in a range of 1700 nm to 4000 nm, when energized by the first therapeutic flash.

The optical assembly may be coupled to the first laser source and second laser source configured to substantially combine the ablative laser beam and non-ablative laser beam and deliver them to the desired target zone on the patient tissue.

The control unit may be configured to control the first flash lamp charger and the second flash lamp charger to deliver:
a. a first heat current pulse to the first flash lamp configured to energize the first flash lamp to generate the first heat flash, wherein the first heat flash is configured to energize the first laser rod below a laser activation threshold, thus keeping the first laser rod thermally primed and not producing the therapeutic non-ablative laser beam; and
b. a first therapeutic current pulse to the first flash lamp configured to energize the first flash lamp to generate the first therapeutic flash, wherein the first therapeutic flash is configured to energize the solid state first laser rod above the laser activation threshold, producing the therapeutic non-ablative laser beam; and
c. a second therapeutic current pulse to the second flash lamp configured to energize the second flash lamp to generate the second therapeutic flash, wherein the second therapeutic flash is configured to energize the solid state second laser rod above the laser activation threshold, producing the therapeutic ablative laser beam.

The control unit may be further configured to control the first flash lamp charger and second flash lamp charger such that one or more first heat current pulses precede one or more first therapeutic current pulses ensuring the one or more first therapeutic non-ablative laser beams are substantially consistent, and one or more therapeutic ablative laser beams are generated simultaneously or in overlap with the therapeutic non-ablative laser beams.

### Description of the drawings

Fig. 1 is an illustration of one aspect of the apparatus.
Fig. 2 is a schematic view of a block diagram of one aspect of the apparatus.
Fig. 3A is a schematic view of a laser chamber with a multiple flowtube assembly.
Fig. 3B is a schematic view of a laser chamber with a single flowtube assembly.
Fig. 4A is a graph laser powered produced by two laser sources when the apparatus is operated in the overlapping method.
Fig. 4B is a graph laser powered produced by two laser sources when the apparatus is operated in the successive method.
Fig. 4C is a graph laser powered produced by two laser sources when the apparatus is operated in the simultaneous method.
Fig. 5A is a graph showing the laser power of the laser beam pulses when the laser rod is not heated.
Fig. 5B is a graph showing the laser power of the laser beam pulses when the laser rod is heated.
Fig. 6 is a graph of current supplied to a flash lamp for heating and subsequent laser beam pulses.
Fig. 7 is a graph of two different current slopes calibrated such that the energy delivered to the flash lamp is the same for both.
Fig. 8 is a schematic view of one aspect of the optical paths of the laser beams inside of the main unit.
Fig. 9 is a schematic view of one aspect of the apparatus with a focus on a fractional applicator.
Fig. 10 is a view of one possible array of plurality of laser spots on skin with potential movement of an applicator.
Fig. 11A is a schematic side view of an array of reflecting elements.
Fig. 11B is a schematic top view of an array of reflecting elements.
Fig. 11C is a schematic top view of an array of reflecting elements.
Fig. 11D is a schematic top view of an array of reflecting elements.
Fig. 11E is a schematic top view of an array of reflecting elements.
Fig. 11F is a schematic top view of an array of reflecting elements.
Fig. 11G is a schematic top view of an array of reflecting elements.
Fig. 11H is a schematic top view of an array of reflecting elements.
Fig. 11J is a schematic top view of an array of reflecting elements.
Fig. 12 is a schematic view of one possible reflecting element.
Fig. 13 is a schematic view of one aspect of the apparatus with a focus on a full field applicator.
Fig. 14A is a schematic view of one aspect of a spacer comprising a contact pad.
Fig. 14B is a schematic view of another aspect of a spacer without a contact pad.

### Detailed description

The disclosed methods and devices may be utilized for treatment of a patient, specifically a treatment area on a patient tissue. In one aspect for example, for removal of unwanted hair, skin rejuvenation, removal of vascular lesions, vascular dyschromia such as telangiectasia and spider veins, treatment of epidermal nevi, fine lines, furrows, keratoses, melasma, skin lesions, skin tags, striae (stretch marks), periorbital rejuvenation, treatment of photo aging, acne treatment, treatment of cellulite, pigmented lesions and psoriasis, tattoo removal, treatment of skin and other cancers or sun damage. Furthermore, the proposed methods and devices may be used for skin resurfacing, resurfacing treatment of textural irregularities, resurfacing treatment of wrinkles, rhinophyma, rhytides, treatment of scars (e.g. surgical or acne scars), hair transplantation, treatment of non-pigmented benign epidermal or dermal lesions (warts, xenthalasma, syringoma, milium...) or laser-assisted drug delivery.

In one aspect, the treatment area may comprise various regions of the human body that are commonly targeted in aesthetic medicine procedures, e.g. for improvement of visual appearance. These include the entire face or parts of the face, with particular attention to the forehead, temples, glabella, periorbital area (including upper and lower eyelids), cheeks, nose, nasolabial folds, upper and lower lips, chin, and jawline. The treatment area may further extend to the earlobes, scalp (for potential applications in hair stimulation or rejuvenation), neck, neckline, nape, submentum, and décolleté (cleavage) or chest. Additional commonly treated areas include the shoulders, upper arms, elbows, forearms, hands (including the dorsal surfaces and fingers), and underarms. The abdomen, flanks, hips, upper back, lower back, buttocks, thighs (inner, outer, and posterior), knees, calves, feet (e.g. dorsal surface and toes) and ankles may also be included.

In another aspect, the treatment area may also comprise oral mucosa, gingiva, dental tissues, the periorbital region, eye or intraocular structures, ear, vaginal mucosa, external genital tissues and localized areas of the scalp or peripheral nerves.

In addition, the proposed methods and devices may be utilized for treatment of verruca, warts and other hard growth on the skin caused by a viral infection, specifically by certain strains of the human papillomavirus (HPV).

Furthermore, the proposed methods and devices may be used in dentistry, for example for hard tissue ablation (cavity preparation, enamel and dentin treatment), soft tissue procedures (gingivectomy, periodontal treatments), bone surgery (osteotomy, implant site preparation), soft tissue surgeries (gingivectomy, frenectomy, and lesion removal), peri-implantitis treatment, or endodontic disinfection.

In another aspect, the proposed methods and devices may be used in vaginal applications, e.g. treatment of vaginal atrophy, vaginal laxity, stress urinary incontinence, lesion of the external genitalia, vaginal rejuvenation (improving elasticity and hydration), and cervical lesion removal and so on.

In another aspect, the proposed methods and devices may be used for surgery, e.g. ablation of soft tissues with minimal thermal damage, microsurgery (ENT, ophthalmology) general soft tissue surgery (ENT, neurosurgery, gynecology, urology), or treatment of tumors and dysplastic lesions.

In another aspect, the proposed methods and devices may be used in ophthalmology, for example for eyelid surgery (blepharoplasty), vision correction (refractive surgery), treatment of retinal diseases (Diabetic Retinopathy, Retinal Tears or Detachments, Macular Edema), glaucoma treatment, cataract surgery, treatment of corneal disorders, removal of eye tumors or lesions, or pleryglium removal.

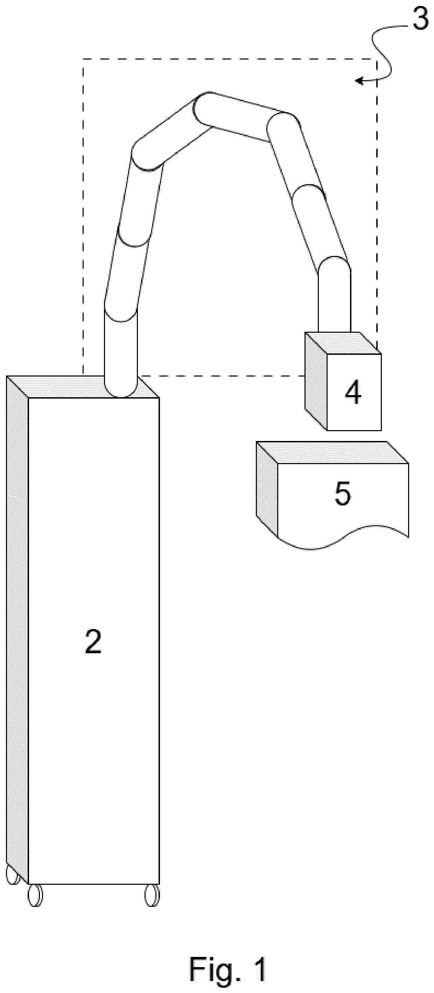

Fig. 1 shows a diagram of one aspect of a laser apparatus 1 for a treatment of a patient. The apparatus 1 may comprise a main unit 2, a waveguide 3, and an applicator 4, which can be in contact, coupled to, adjacent to or spaced from the tissue 5.

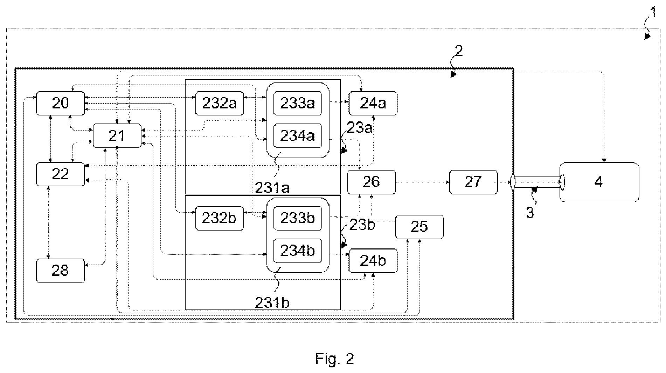

The main unit 2, of which an exemplary diagram is shown in Fig. 2, may comprise a power source 20, one or more laser sources (e.g. laser sources 23a and 23b), a control unit 21, a user interface 22, one or more optical output measurement units (e.g. optical output units 24a and 24b), an aiming beam source 25, a beam combiner 26, one or more collimators 27 and a foot switch 28.

In another aspect, the foot switch 28 is external to the main unit 2, but may be connected to the main unit 2, preferably in communication with the control unit 21.

The main unit 2 may comprise a power source 20, which may include a transformer, disposable battery, rechargeable battery, power plug or standard power cord. The output power of the power source 20 may be in a range of 10 W to 4000 W, or in a range of 500 W to 3000 W, or in a range of 800 W to 2000 W.

In one aspect, the main unit 2 may comprise only one laser source, e.g. the laser source 23a, configured to produce a laser beam.

In another aspect, the main unit 2 may comprise multiple laser sources, e.g. in a range of 2 to 20, or 2 to 10 or 2 to 5 or 2 to 4 or 3 to 10 or 3 to 5 laser sources, configured to produce a respective number of laser beams.

In one aspect, the main unit 2 may comprise two laser sources, e.g. the laser source 23a and 24b, configured to produce a first laser beam and a second laser beam, respectively, and the aiming beam source 25.

Throughout the description, the laser source may mean at least one laser source 23a and/or 23b, a laser chamber 231 may mean at least one of laser chamber 231a and/or 231b, a laser rod 234 may mean at least one of laser rod 234a and/or 234b, a flash lamp 233 may mean at least one of flash lamp 233a and/or 233b, and a flash lamp charger may mean at least one of flash lamp charger 232a and/or 232b.

Each laser source (e.g. laser source 23a or 23b) may comprise a laser chamber (e.g. laser chamber 231a or 231b) and a charger. The charger may comprise an electronic circuit configured to charge the laser chamber (e.g. laser chamber 231a or 231b).

The laser chamber (e.g. laser chamber 231a or 231b) may comprise a laser medium (gain medium, e.g. solid, liquid, gas, or semiconductor), a pumping source (e.g. flash lamp, diode, electric discharge or current source, or chemical pump), an optical resonator (laser cavity/optical cavity, e.g. two mirrors, one fully reflective and one partially reflective - output coupler), a cooling system (water cooling, air cooling, cryogenic cooling), beam focusing and collimating optics, stabilization and alignment mechanism (e.g. alignment screws or vibration isolation), or a shutter or beam blocker (e.g. to safely block the beam when not in use). In one aspect, the laser resonator may comprise a convex mirror. In another aspect, the laser resonator may comprise a concave mirror. In another aspect, the laser resonator may comprise a planar mirror. In another aspect, the laser resonator may comprise a combination of two above-mentioned mirrors (e.g. one planar and one concave).

In one aspect, the laser chamber (e.g. laser chamber 231a or 231b) may comprise a solid-state laser (e.g. a laser rod 234a or 234b) and a flash lamp (e.g. 233a or 233b). The laser charger may comprise a flash lamp charger (e.g flash lamp charger 232a or 232b) configured to charge a flash lamp (e.g a flash lamp 233a or 233b) inside the laser chamber. The flash lamp charger may comprise a capacitor or a capacitor bank configured to store the electric energy provided by the power source 20 and then rapidly discharge it through a flash lamp with a given electric current in a therapeutic current pulse 703 with a substantially rectangular profile (i.e. turns on quickly and turns off quickly) controlled by the control unit 21. When triggered, the flash lamp emits a burst of broad-spectrum light in a therapeutic flash 713, which is absorbed by the laser rod 234. This optical energy excites the atoms in the laser medium to higher energy states, creating a population inversion-where more atoms are in an excited state than in the ground state. Once a sufficient number of atoms is excited, stimulated emission occurs within the optical cavity, where mirrors reflect and amplify the emitted photons, resulting in coherent laser light in the form of a laser beam pulse 723. The process repeats with each therapeutic flash 713 of the flash lamp, enabling the laser to operate in a pulsed mode. In another aspect, the therapeutic flashes may repeat fast enough to keep the laser rod continually excited, which may enable the laser to operate in continuous mode.

In another aspect the laser chamber may comprise a laser rod and a plurality of flash lamps arranged around the laser rod. The flash lamps may be charged by one flash lamp charger or each flash lamp may have its own flash lamp charger.

In another aspect, the laser chamber may comprise a plurality of laser rods and a plurality of flash lamps arranged around the laser rods. The flash lamps may be charged by one flash lamp charger or each flash lamp may have its own flash lamp charger.

In another aspect, the laser chamber may comprise a plurality of laser rods and one flash lamp, wherein the laser rods are arranged around the flash lamp.

In different aspects, the flash lamp/lamps may be replaced by a pumping laser diode/diodes.

In another aspect, the laser chamber may comprise one or more gas lasers (e.g. He-Ne, CO₂, Ar), or semiconductor (diode) lasers (e.g. laser diodes, or VCSEL (Vertical-Cavity Surface-Emitting Laser)), or fiber lasers (Erbium-Doped Fiber Laser, Ytterbium-Doped Fiber Laser), or dye lasers, or free electron lasers (FELs), or any other known type of laser.

In one aspect, the main unit 2 may comprise a first laser source 23a and a second laser source 23b. The first laser source 23a may comprise a first laser chamber 231a and a first flash lamp charger 232a. The second laser source 23b may comprise a second laser chamber 231b and a second flash lamp charger 232b.

In one aspect, the first laser chamber 231a may be configured to generate a non-ablative laser beam, which may have a wavelength in a range of 1000 nm to 1800 nm or in a range of 1200 nm to 1650 nm or in a range of 1520 nm to 1560 nm. In another aspect, the wavelength of the non-ablative laser beam may be in a range of 1000 nm to 3000 nm. Alternatively, the wavelength of the non-ablative laser beam may be in a range of 9000 nm to 11000 nm or in a range of 1700 nm to 4000 nm.

In one aspect, the first laser chamber 231a may generate a laser beam with an output power in a range of 10 W to 3000 W or in a range of 50 W to 2500 W or in a range of 100 W to 1500 W. Alternatively, the first laser chamber 231a may generate a laser beam with an output power in a range of 10 W to 375 W.

In another aspect, the maximum pulse energy (i.e. maximum energy released in a single pulse) of the non-ablative laser beam may be in a range of 1 J to 5 J or in a range of 2 J to 4 J or in a range of 2.5 J to 3.5 J or in a range of 2.6 J to 3.4 J or in a range of 2.7 J to 3.3 J.

In one aspect, the first laser chamber 231a may comprise a first flash lamp 233a and a first laser rod 234a. In one aspect the first laser rod 234a may comprise a solid state Er:glass rod (capable of producing a laser beam at approximately 1540 nm). In another aspect, the first laser rod 234a may comprise a solid-state Nd:YAG or Ho:YAG rod or Er:YAG or Tm:YAG rod. The first flash lamp 233a may have an output power of a broad-spectrum light in a range of 10 W to 3000 W or in a range of 50 W to 2500 W or in a range of 100 W to 1500 W. The first flash lamp 233a flash duration may be in a range of 0.5 ms to 10 ms or in a range of 1 ms to 9 ms or in a range of 2 ms to 8 ms. The first flash lamp 233a inside the first laser chamber 231a may be powered by the first flash lamp charger 232a. The first flash lamp charger 232a may comprise a capacitor bank charged by the power source 20. In another aspect, the first laser chamber 231a may comprise a different type of laser, e.g. a CO₂ laser, dye laser or a diode laser.

In one aspect, the second laser chamber 231b may be configured to generate an ablative laser beam, which may have a wavelength in a range of 2500 nm to 3500 nm or in a range of 2700 nm to 3200 nm or in a range of 2900 nm to 2980 nm. Alternatively, the wavelength of the ablative laser beam may be in a range of 9000 nm to 11000 nm or in a range of 1700 nm to 4000 nm.

In one aspect, the second laser chamber 231b may generate a laser beam with an output power in a range of 10 W to 20000 W or in a range of 50 W to 19000 W or in a range of 100 W to 17500 W. Alternatively, the second laser chamber 231b may generate a laser beam with an output power in a range of 10 W to 2500 W.

In another aspect, the maximum pulse energy (i.e. maximum energy released in a single pulse) of the ablative laser beam may be in a range of 1 J to 5 J or in a range of 2 J to 4.5 J or in a range of 3 J to 4 J or in a range of 3.1 J to 3.9 J or in a range of 3.2 J to 3.8 J.

In one aspect, the second laser chamber 231b may comprise a second flash lamp 233b and a second laser rod 234b. In one aspect the second laser rod 234b may comprise a solid state Er:YAG (capable of producing a laser beam at approximately 2940 nm) rod. The second flash lamp 233b may have an output power in a range of 10 W to 20000 W or in a range of 50 W to 19000 W or in a range of 100 W to 17500 W. The second flash lamp 233b flash duration may be in a range of 0.05 ms to 3 ms or in a range of 0.1 ms to 2 ms or in a range of 0.2 ms to 1.5 ms. The second flash lamp 233b inside the second laser chamber 231b may be powered by the second flash lamp charger 232b. The second flash lamp charger 232b may comprise a capacitor bank charged by the power source 20. In another aspect, the second laser chamber 231b may comprise a different type of laser, e.g. a CO₂ laser or a diode laser.

In another aspect, one or more other laser sources may be present. In one example, the main unit 2 may also comprise a laser chamber configured to generate ultraviolet (UV) laser beams, particularly useful for specific dermatological treatments and disinfection, or dentistry or ophthalmology. In one example, the UV laser may emit a therapeutic wavelength within the ultraviolet spectrum, typically in a range of 190 nm to 400 nm, or in a range of 193 nm to 308 nm. In another example, the UV laser emission may be configured to wavelengths such as 193 nm (e.g. ArF excimer laser), 248 nm (e.g. KrF), or 308 nm (e.g. XeCl excimer laser), selected for their specific interaction with targeted chromophores such as nucleic acids and proteins within the epidermis and superficial dermal layers.

In another example, the main unit 2 may further comprise a laser chamber configured to generate laser beam pulses within the visible spectrum, particularly beneficial for dermatological and aesthetic procedures requiring precise chromophore targeting or for dentistry or ophthalmology. Such a visible-spectrum laser chamber may emit wavelengths in a range of approximately 400 nm to 700 nm, or in a range of 532 nm to 595 nm. Visible wavelength lasers, including those operating at approximately 532 nm (frequency-doubled Nd:YAG), 585 nm or 595 nm (pulsed dye lasers), may be particularly effective for treating vascular lesions, such as telangiectasias, spider veins, and port-wine stains, due to the selective absorption by hemoglobin.

In another example, the main unit 2 may further comprise a laser chamber configured to generate near-infrared laser beams, such as those generated by Nd:YAG lasers, typically operating at wavelengths around 1064 nm. Near-infrared lasers offer deeper penetration into dermal and subdermal layers and may be particularly suitable for deeper vascular lesions, skin tightening, and hair removal treatments. Due to its deeper penetration and selective absorption characteristics, near-infrared laser treatment may be particularly effective for vascular treatments, non-invasive skin tightening, hair removal, and the management of deeper dermal lesions.

In yet another example, the main unit 2 may further comprise a laser chamber utilizing a carbon dioxide (CO₂) laser source, emitting laser beams at wavelengths typically around 10 600 nm. Due to its very high absorption by water, the CO₂ laser may provide highly efficient and controlled ablation and vaporization of superficial skin tissues, making it ideally suited for aggressive skin resurfacing, removal of deep wrinkles, prominent scars, or significant sun damage.

In one aspect the laser chamber (e.g. the first or second laser chambers) may comprise PTFE (Polytetrafluoroethylene) 236, for example the laser chamber cavity may be made of PTFE 236 or may be coated with PTFE 236. PTFE has a refractive index in a range of 1.3 to 1.4 and a high reflectivity, for example a high diffuse reflectivity for a broad spectrum of light. Unlike metallic reflectors, PTFE diffusely scatters light, ensuring that light from the flash lamp is evenly distributed around the laser rod. This may ensure that the broad spectrum light of the flash lamp is maximally absorbed by the laser rod. It is also one of the easiest materials to work with known in the art. PTFE possesses superior mechanical resilience and thermal stability (up to 260°C), thereby enhancing the longevity and reliability of the laser chamber. Unlike polished metallic reflectors that create hot spots due to specular reflection, PTFE's diffuse reflection eliminates concentrated areas of heating. PTFE is chemically inert (e.g. it does not react with coolants, cleaning agents, or gases) and resistant to corrosion, ensuring sustained optical performance without deterioration. The low coefficient of friction of PTFE further facilitates ease of manufacturing and assembly, reducing complexity and cost in device production. PTFE has excellent electrical insulating properties, preventing electrical breakdown in laser systems that use high-voltage components (e.g., flash lamp circuits). This makes it ideal for protecting wiring, connectors, and insulating high-energy discharge components in pulsed laser systems. Accordingly, the use of PTFE in the laser chamber provides a durable, cost-effective, and optically efficient solution for enhancing the performance of the solid-state laser system.

In another aspect the laser chamber may comprise ceramics (e.g. Al₂O₃), BaSO₄, gold or silver. For example, the laser chamber cavity may be made of or may be coated in ceramics (e.g. Al₂O₃), BaSO₄, gold or silver.

In one aspect the laser chamber (e.g. the first laser chamber 231a and the second laser chamber 231b) may be cooled by a cooling fluid 237 circulating in the cooling system of apparatus 1. The cooling system may be a part of the main unit 2. The temperature of the cooling fluid may be in a range of 0°C to 50°C or in a range of 10°C to 45°C or in a range of 18°C to 40°C or in a range of 18°C to 35°C or in a range of 18°C to 25°C. The cooling fluid 237 may comprise a cooling liquid or a cooling gas.

The cooling system may be configured such that the temperature of a laser rod 234 (e.g. the first laser rod 234a or the second laser rod 234b) cooled by the cooling system while in use may be in a range of 0°C to 50°C or in a range of 10°C to 45°C or in a range of 18°C to 40°C. Additionally, the cooling system may be configured such that a flash lamp (e.g. the first flash lamp 233a or the second flash lamp 233b) in use may be cooled to a temperature in a range of 0°C to 50°C or in a range of 10°C to 45°C or in a range of 18°C to 40°C. In one aspect, the cooling system may be configured such that the temperature of laser rod(s) 234 or flash lamp(s) is maintained at ambient or room temperature during use of the apparatus 1.

In one aspect the cooling liquid 237 may comprise water, or deionized water. In another aspect, the cooling liquid may comprise a water-glycerol mixture, dielectric fluid (e.g. fluorinated fluid, mineral oil or silicone oil), thermally conductive oil (e.g. polyalphaolefin fluid), liquid metal (e.g. galinstan), refrigerant (e.g. R134a, R1234y) or other cooling fluids typically used in the art.

In another aspect, the cooling liquid may comprise a cooling gas. The cooling gas may comprise ammonia (NH₃), carbon dioxide (CO₂), propane, isobutane, helium (He), argon (Ar), nitrogen (N₂), difluoromethane (R-32), other refrigerants (e.g. R-410A, R-404A, R-507 or R-407C) or other commonly used cooling gases.

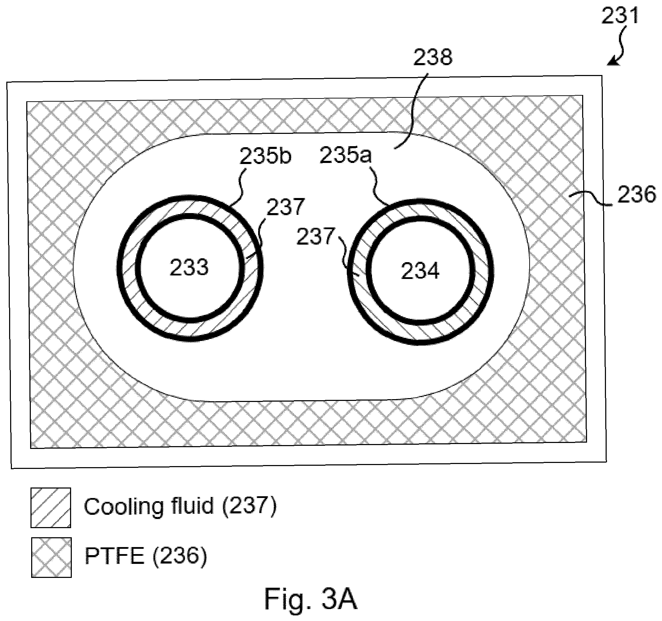

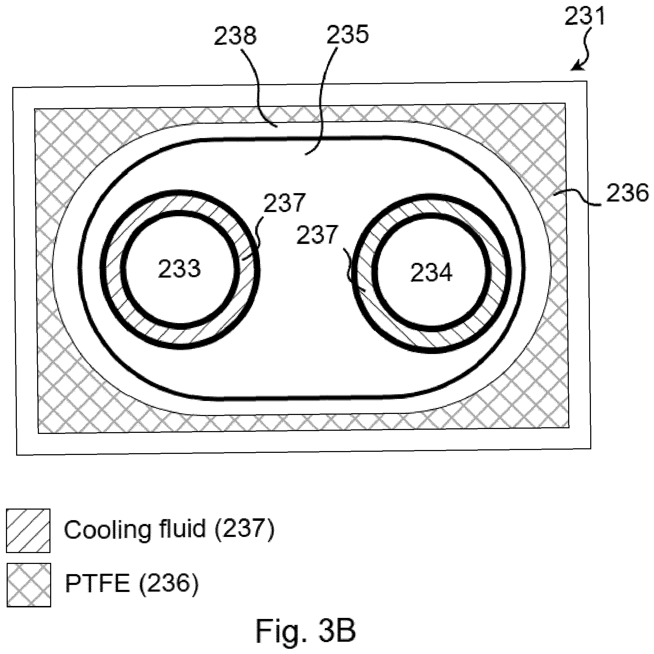

In one aspect, the laser chamber (e.g. the first laser chamber 231a or the second laser chamber 231b) may comprise a flowtube assembly, for example such as shown in Figs. 3A and 3B.

In one aspect, the flowtube assembly may be a multiple flowtube assembly as shown in Fig. 3A. The multiple flowtube assembly may be configured such that multiple flowtubes are inside the cavity 238 of the laser chamber. Each flowtube holds one laser rod 234 or one flash lamp 233 such that space for cooling-flow (e.g. water-flow) 237 may be present adjacent the laser rod 234 or the flash lamp 233 to ensure proper cooling of the laser rods or flash lamps. In one aspect, the laser rod or the flash lamp is physically isolated from the cooling-flow 237 (thus isolated from physical contact with the cooling fluid). In another aspect, the laser rod or the flash lamp is in contact with cooling fluid of the cooling-flow 237. In the multiple flowtube assembly configuration the cavity may take up most of the laser chamber and thus most of the laser chamber may comprise air, other gas, or may be evacuated. This configuration allows for efficient cooling since the cooling fluid can circulate independently around each laser rod and each flash lamp. Furthermore, the majority of cavity space is filled with air, other gas, or is evacuated, which minimizes heat transfer between laser rods and flash lamps. This setup prevents thermal stress on the laser rod, maintaining beam quality and increasing the lifetime of both the rod and the flash lamp. It also provides easy maintenance and replacement of the laser rods or flash lamps without affecting the entire cavity structure. In one example, the multiple flowtube assembly may comprise glass.

In one aspect, the amount of air, gas, or vacuum in the laser chamber comprising the multiple flowtube assembly may be in a range of 10% to 90% or in a range of 20% to 85% or in a range of 50% to 80% of the laser chamber volume.

In one example, shown in Fig. 3A, the multiple flowtube assembly may comprise a first flowtube 235a configured to hold the laser rod 234 and a second flowtube 235b configured to hold the flash lamp 233, wherein the first flow tube comprises a space for cooling-flow (e.g. water-flow) 237 adjacent the laser rod and/or the second flow tube comprises a space for cooling-flow 237 adjacent the flash lamp to ensure proper cooling of the laser rod and/or the flash lamp.

In another aspect, the flowtube assembly may be a single flowtube assembly 235 as shown in Fig. 3B. The single flowtube assembly may be configured such that a single flowtube inside the cavity 238 of the laser chamber 231 holds at least one laser rod 234 and at least one flash lamp 233 and may take up most of the laser chamber 231. A small air gap may be present near the inner edge of the laser chamber 231 close to the outer edge of the single flowtube 235 to ensure the material of the laser chamber is not damaged and to enable the assembly of the chamber. A small space for cooling-flow 237 (e.g. water-flow) may be present near the laser rod 234 and/or the flash lamp 233 to ensure proper cooling of the laser rod(s) or the flash lamp(s). In one aspect, the laser rod or the flash lamp is physically isolated from the cooling-flow 237 (thus isolated from a physical contact with the cooling fluid). In another aspect, the laser rod or the flash lamp is in contact with cooling fluid of the cooling-flow 237. The single flowtube assembly configuration reduces the complexity of the laser system and minimizes the risk of misalignment. Placing both components in the single flowtube may ensure maximum optical coupling. In one example, the single flowtube assembly may comprise glass.

In one aspect, the amount of air, gas or vacuum in the laser chamber comprising the single flowtube assembly may be in a range of 0.1% to 40% or in a range of 0.5% to 30% or in a range of 1% to 20% of the laser chamber volume.

In one example, shown in Fig. 3B, the first laser chamber 231a may comprise a single flowtube assembly while the second laser chamber 231b may comprise a multiple flowtube assembly. In another example, the first laser chamber 231a may comprise a multiple flowtube assembly while the second laser chamber 231b may comprise a single flowtube assembly. In another example, both the first laser chamber 231a and the second laser chamber 231b, may comprise multiple flowtube assemblies. In another example, both the first laser chamber 231a and the second laser chamber 231b, may comprise single flowtube assemblies.

In one aspect, a laser chamber (e.g. the first laser chamber 231a or the second laser chamber 231b) may have a single flash lamp configured to deliver energy to a single laser rod. This arrangement, while having lower power flashes, does simplify the laser chamber.

In another aspect, the number of flash lamps and laser rods inside a laser chamber (e.g. the first laser chamber 231a or the second laser chamber 231b) may be in a range of 2 to 10 or in a range of 2 to 5 or in a range of 2 to 3.

For example, the first laser chamber 231a may comprise a single flowtube assembly holding a single laser rod 234a and a single flash lamp 233a and the second laser chamber 231b may comprise a multiple flowtube assembly holding a single laser rod 234b and a single flash lamp 233b.

The main unit 2 may comprise a control unit 21 and a user interface 22. The user interface 22 may be connected to the control unit 21. The control unit 21 may be configured to control the treatment parameters of the apparatus 1 based on the input from the user interface 22. The parameters of the treatment may include, but are not limited to, the wavelength of the laser beams produced by the laser beam sources, the pulse length of the laser beam pulses, the energy output of the apparatus 1 and the frequency of the laser beam pulses, or the number of pulses produced by respective laser beam sources. The control unit 21 may comprise a microprocessor, PCB, processor and/or a memory. The memory may store treatment protocols, the treatment protocols may include values for one or more treatment parameters. The treatment parameters stored on the memory may include, but are not limited to, the fluence used for each laser source, frequency of the laser beam pulses and the pulse length for each laser source, and the number of repeated pulses (stack) for each laser source.

The user interface 22 may include a display, buttons, a keyboard, a touchpad, a touch panel or other control members enabling an operator to control, check or adjust therapy and device parameters. In one aspect, the operator may be able to control each of the laser beam parameters separately (e.g. the frequency of laser beam pulses or laser beam pulse stacks, the fluence, the pulse length). In another aspect, the operator may be able to control one or more laser beam parameters indirectly - for example, directly adjusting a first laser beam parameter may automatically change a value of a second laser beam parameter based on a rule or relationship established between the parameters.

In one aspect, the operator may be able to adjust the treatment parameters for each of the laser sources (e.g. the first laser source 23a or the second laser source 23b) separately. Thus, the operator may be able to adjust the fluence separately for each laser source, for example, for the first laser source 23a and for the second laser source 23b. And the operator may also be able to adjust the pulse length for each laser source, for example, for the first laser source 23a and for the second laser source 23b, separately.

In one aspect, the fluence of a single pulse of a laser beam at the distal end of the waveguide 3 may be in a range of 0.05 J/cm² to 6 J/cm² or in a range of 0.1 J/cm² to 5 J/cm² or in a range of 0.2 J/cm² to 4 J/cm². Additionally, the maximum fluence may be in a range of 1.8 J/cm² to 4 J/cm².

In another example, the fluence of a single pulse of the non-ablative laser beam at the distal end of the waveguide 3 may be in a range of 0.05 J/cm² to 6 J/cm² or in a range of 0.1 J/cm² to 5 J/cm² or in a range of 0.2 J/cm² to 3.3 J/cm². Additionally, the maximum fluence may be in a range of 2.3 J/cm² to 3.3 J/cm².

In one example, the fluence of a single pulse of the ablative laser beam at the distal end of the waveguide 3 may be in a range of 0.05 J/cm² to 6 J/cm² or in a range of 0.1 J/cm² to 5 J/cm² or in a range of 0.2 J/cm² to 3.7 J/cm². Additionally, the maximum fluence may be in a range of 2.7 J/cm² to 3.7 J/cm².

In another aspect, the energy of a single combined laser beam pulse produced by a laser source may be in a range of 0.1 J to 6 J or in a range of 0.2 J to 5 J or in a range of 0.3 J to 4 J. Additionally, the maximum energy may be in a range of 2 J to 4 J.

In one example, the energy of a single combined laser beam pulse produced by the first (e.g. non-ablative) laser source may be in a range of 0.1 J to 6 J or in a range of 0.2 J to 5 J or in a range of 0.3J to 3.5 J. Additionally, the maximum energy may be in a range of 2.5 J to 3.5 J.

In another example, the energy of a single combined laser beam pulse produced by the second (e.g. ablative) laser source may be in a range of 0.1 J to 6 J or in a range of 0.2 J to 5 J or in a range of 0.3 J to 4 J. Additionally, the maximum energy may be in a range of 3 J to 4 J.

In one example, when using an ablative laser source and a non-ablative laser source in a successive, overlapping or simultaneous method, the fluence of a single repetition of the method (i.e. each laser pulsing once) at the distal end of the waveguide 3 may have a value in a range of 0.1 J/cm² to 12 J/cm² or in a range of 0.2 J/cm² to 10 J/cm² or in a range of 0.4 J/cm² to 7 J/cm².

In another example, when using an ablative laser source and a non-ablative laser source in a successive, overlapping or simultaneous method, the energy of a single repetition of the method (i.e. each laser pulsing once) produced by the laser sources may be in a range of 0.2 J to 12 J or in a range of 0.4 J to 10 J or in a range of 0.6 J to 7.5 J.

In another aspect, the pulse length of any of the plurality of laser sources may be in a range of 0.01 ms to 30 ms or in a range of 0.05 ms to 20 ms or in a range of 0.1 ms to 10 ms.

In one aspect, the apparatus 1 may fire laser beam pulse stacks with a set frequency. A laser beam pulse stack may comprise a plurality of consecutive laser beam pulses from a single laser source (so called stacking). In one aspect, the stack is a setting of a laser in which the total delivered laser output is the sum of several individual pulses, each with a pulse length (e.g. set by the user). For example, 2x stack may mean that the total laser output is the sum of two consecutive laser pulses. The number of stacks represents the number of pulses being delivered consecutively.

In one aspect, when the user of the apparatus sets a first parameter (e.g. fluence) via the user interface and subsequently selects a stacking option (e.g. 2× stack, representing two consecutive pulses), the control unit may automatically recalculate the value of the first parameter to reflect the cumulative effect over the full stack. The user interface may then display the recalculated value (e.g. double the fluence), representing a value of the first parameter (e.g. fluence) for the whole selected pulse stack.

In one aspect, the laser beam pulse stack may comprise a plurality of consecutive laser beam pulses in a range of 1 to 100 pulses or in a range of 1 to 50 pulses or in a range of 1 to 10 pulses or in a range of 2 to 100 pulses or in a range of 2 to 50 pulses or in a range of 2 to 10 pulses.

In one aspect, the frequency of firing of laser beam pulse stacks may be in a range of 0.1 Hz to 50 Hz or in a range of 1 Hz to 40 Hz or in a range of 1.5 Hz to 30 Hz or in a range of 0.1 Hz to 10 Hz or 0.5 Hz to 7 Hz or 1 Hz to 5 Hz.

In one aspect, the frequency of firing the individual laser beam pulses inside of a laser beam pulse stack may be in a range of 1 Hz to 20 Hz or in a range of 5 Hz to 15 Hz or in a range of 7 Hz to 13 Hz. Thus, the duration of one laser beam pulse stack may be in a range of 0.01 ms to 10 s or in a range of 0.05 ms to 5 s or in a range of 0.1 ms to 800 ms or in a range of 0.01 ms to 110 s.

The user interface 22 may further comprise an emergency stop button. The emergency stop button may be connected to the control unit 21. The control unit 21 may be configured such that when the emergency stop button is pressed, the therapy is immediately stopped. The control unit 21 may also be programmed to stop the therapy, if any of the treatment parameters are outside of predetermined safe ranges for the given protocol or parameter ranges set by the operator.

In one aspect, the control unit 21 may control the plurality of laser beam sources (e.g. first laser beam source 23a and the second laser beam source 23b) such that the laser beams may be delivered in a pulse mode.

In one aspect, the pulse length of the laser beam produced by the first laser beam source 23a (e.g. the non-ablative laser beam) may be in a range of 0.5 ms to 10 ms or in a range of 1 ms to 9 ms or in a range of 2 ms to 8 ms.

In another aspect, the pulse length of the laser beam produced by the second laser beam source 23b (e.g. the ablative laser beam) may be in a range of 0.05 ms to 3 ms or in a range of 0.1 ms to 2 ms or in a range of 0.2 ms to 1.5 ms.

The shapes of the pulses may include, but are not limited to, Gaussian pulses, characterized by a smooth intensity distribution ideal for uniform energy delivery; square pulses, which provide consistent peak power throughout the pulse duration, reducing thermal variability; super-Gaussian pulses, which offer a flatter central intensity profile for controlled energy dispersion; and exponentially decaying pulses, designed to deliver an initial high-energy burst followed by a gradual decline, thereby reducing residual heat build-up. Additionally, chirped pulses may be employed, wherein the frequency or phase of the laser light varies over the pulse duration to enhance nonlinear effects or improve penetration depth. The system may also incorporate composite or modulated pulses, where multiple sub-pulses of varying intensity or duration are combined to achieve tailored photo-thermal or photo-mechanical effects. Furthermore, the pulses may have a flattop shape.

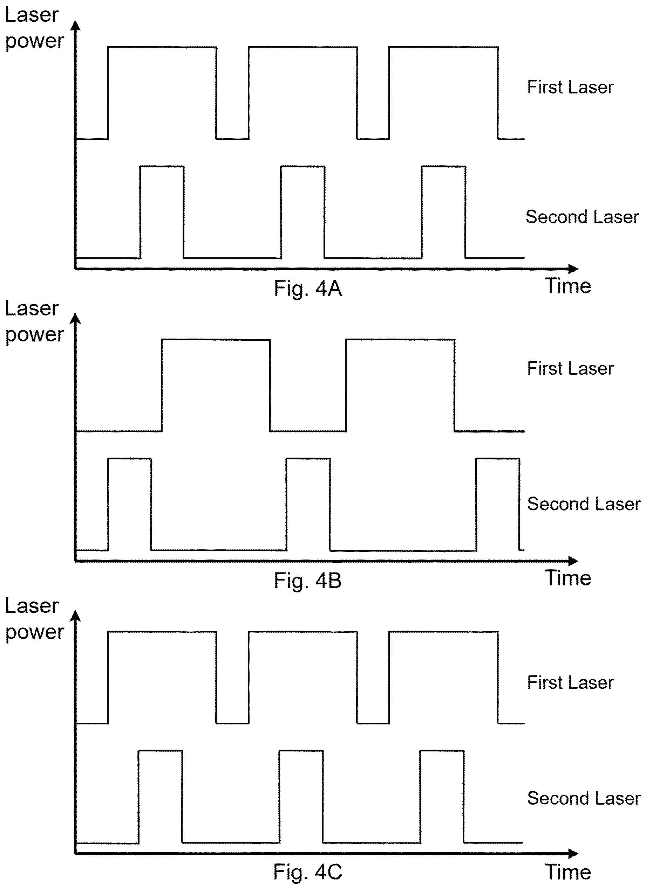

In one aspect, a plurality of laser sources, e.g. the first laser source 23a and the second laser source 23b, may be operated in a simultaneous method. For example, all (e.g. both) laser sources are used simultaneously and the laser beams are effective during a time interval e.g., 1-2 ms. An exemplary graph of laser power for two laser sources operated simultaneously is shown in Fig. 4C.

In another aspect, the laser sources may be operated in a successive method. For example, in the case of two laser sources, the first laser source is activated and the first laser beam is effective during a first time interval e.g., from 1 to 3 ms. The second laser source is then activated such that the second laser beam is effective in a subsequent time interval e.g., from 3 to 4 ms. Such a sequence of two or more successive steps may be repeated. In one aspect, the second laser source may start right after the end of the first laser source. In another aspect, the second laser source may start after a pause time, which follows the end of the operation of the first laser source. The pause time may be in a range of 0.1 ms to 5 ms or in a range of 0.2 ms to 3 ms or in a range of 0.3 ms to 1 ms or in a range of 1 µs to 1 s. An exemplary graph of laser power for two laser sources operated sequentially is shown in Fig. 4B.

In yet another aspect the laser sources may be operated in an overlapping method (temporally overlapping method) or partially simultaneously. For example, in the case of two laser sources, the first laser source is activated and a first laser beam is effective (emitted) during the first time interval e.g., from 1 to 3 ms. Then the second laser source is activated such that the second laser beam is effective in a second overlapping time interval e.g., from 2.5 to 3.5 ms. Accordingly, both laser beams are simultaneously active during the overlapping subinterval from 2.5 ms to 3.0 ms, wherein during the second time interval the first laser beam and the second laser beam are overlapping e.g. with total overlapping method time of 0.5 ms. Thus, the total time interval of overlapping of both laser beams (the total period during which either or both beams are active) is from 1 ms to 3.5 ms. The first laser source is activated at time of 1 ms and remains active until time of 3 ms. The second laser source is activated at time of 2.5 ms and remains active until time of 3.5 ms. As a result, both lasers are simultaneously active during the overlapping time interval from time 2.5 ms to time 3 ms, therefore the overlapping duration, during which both laser beams are simultaneously active, is 0.5 ms. Thus the laser sources are operated partially simultaneously for at least a subinterval of operation time, or in other words the, laser sources are operated in a partially simultaneous manner over at least a portion of their respective activation intervals. An exemplary graph of laser power for two laser sources operated in overlap is shown in Fig. 4A.

In one aspect, a plurality of laser rods (e.g. the first laser source and the second laser source) may be excited by a plurality of flash lamps, e.g., the first flash lamp 233a and the second flash lamp 233b. The plurality of flash lamps may be charged by (e.g. receive current from) their respective flash lamps chargers in a simultaneous method. For example, all (e.g., both) flash lamps are charged simultaneously, and the resulting therapeutic flashes are effective during a time interval, e.g., 1-2 ms, thus the laser rods generate laser beams in said time interval.

In another aspect, the plurality of flash lamps may be charged by their respective flash lamp chargers in a successive method. For example, in the case of two flash lamps, the first flash lamp is charged such that the first therapeutic flash is effective during a first time interval, e.g., from 1 to 3 ms, thus the first laser rod generates a laser beam in said interval. The second flash lamp is then charged such that the second therapeutic flash is effective in a subsequent time interval, e.g., from 3 to 4 ms, thus the second laser rod generates a laser beam in said subsequent time interval. Such a sequence of two or more successive steps may be repeated. In one aspect, the second flash lamp may be charged immediately after the first flash lamp stops being charged. In another aspect, the second flash lamp may be charged after a pause time following the first flash lamps end of charging. The pause time may be in a range of 0.1 ms to 5 ms, or in a range of 0.2 ms to 3 ms, or in a range of 0.3 ms to 1 ms, or in a range of 1 µs to 1s.

In yet another aspect, the plurality of flash lamps may be charged by their respective flash lamp chargers in an overlapping method or partially simultaneously. For example, in the case of two flash lamps, the first flash lamp is charged such that a first therapeutic flash is effective during a first time interval, e.g., from 1 to 3 ms. The second flash lamp is then charged such that a second therapeutic flash is effective in a second overlapping time interval, e.g., from 2.5 to 3.5 ms, wherein during the second time interval, the first and second therapeutic flashes overlap, e.g., with a total overlapping method time of 0.5 ms. Thus, the total time interval of overlapping energy supply from both flash lamps is from 1 ms to 3.5 ms. The first flash lamp is activated at 1 ms and remains effective until 3 ms. The second flash lamp is activated at 2.5 ms and remains effective until 3.5 ms. As a result, both flash lamps are active simultaneously during the overlapping interval from 2.5 ms to 3 ms, thus they are operated partially simultaneously for at least a subinterval of the operation time.

In one aspect, the duration of overlapping subinterval - during which at least two laser sources from the plurality of laser sources are simultaneously active - may be defined as a percentage of the shortest pulse length among a plurality of overlapping laser beam pulses. The overlapping subinterval duration may be in a range of 0.1% to 99% or in a range of 0.5% to 95% or in a range of 1% to 90% or in a range of 5 % to 75% or in a range of 10 % to 55% or in a range of 0.5% to 95% of the shortest pulse length from the plurality of pulse lengths corresponding to the plurality of the overlapping laser beam pulses.

In yet another aspect, the plurality of laser sources may create a respective plurality of laser beam pulse stacks. The main unit may operate the plurality of laser sources such that the respective laser beam pulse stacks are fired in a simultaneous method or in a successive method or in an overlapping method. Thus, the individual laser beam pulses from the plurality of the laser sources may be configured to fire in any of the methods described above.

In another aspect the control unit 21 may control at least one laser beam source such that at least one therapeutic laser beam may be delivered in a continuous mode. The continuous mode may be operated for a time interval in a range of 0.05 s to 60 min or in a range of 0.1 s to 45 min or in a range of 0.2 s to 30 min.

In one aspect, the output power of apparatus 1 operated in the continuous mode may be in a range of 10 W to 20000 W or in a range of 50 W to 19000 W or in a range of 100 W to 17500 W. Alternatively, the output power of apparatus 1 operated in the continuous mode may be in a range of 10 W to 2500 W.

In one aspect, when using a solid state laser rod, energy may be delivered to a laser rod 234 inside one of the plurality of laser sources by the respective flash lamp before (called preheating) or in between (called heating) laser beam pulses or stacks. This procedure is done to ensure that the first and following laser (beam) pulses are substantially consistent. Laser-tissue interaction is highly dependent on energy delivery. If energy varies between pulses, the depth and extent of tissue ablation, coagulation, or stimulation will also vary. Inconsistent pulses can lead to uneven results - some areas over-treated and others under-treated. If one pulse is significantly stronger than intended, it might cause excessive tissue damage, burns, or scarring. If a pulse is too weak, it may fail to produce the desired therapeutic effect, leading to the need for more sessions or poor clinical outcomes. Consistent energy also supports maintaining beam quality and coherence, crucial in applications requiring high precision. Preheating or heating the laser rod serves to stabilize the system thermally, ensuring that the first and subsequent pulses deliver consistent, predictable energy, which is a hallmark of professional-grade medical laser devices. Without this, early pulses may be weaker or unstable due to temperature fluctuations in the gain medium.

Preheating may be done before, after or during calibration, or after turning on the device. Heating may be done in between laser beam pulses during therapy, for example, when the foot switch 28 is not activated but the device is turned on, or when the foot switch 28 is not activated but the device is already calibrated.

In one aspect, substantially consistent laser beam pulses means that the energy of the each laser beam pulse produced by the laser rod 234 is higher than 78 % or 80 % or 85 % or 90 % of the energy set by the operator (e.g. via the fluence set on the user interface). In one aspect, the substantially consistent laser beam pulses have the energy in a range of 80 % to 110 % or in a range of 85 % to 105 % or in a range of 90 % to 101 % or in a range of 90 % to 100 % or in a range of 93 % to 100 % or in a range of 78 % to 100 % or in a range of 80 % to 100 % of the energy set by the operator (e.g. via the fluence set on the user interface).

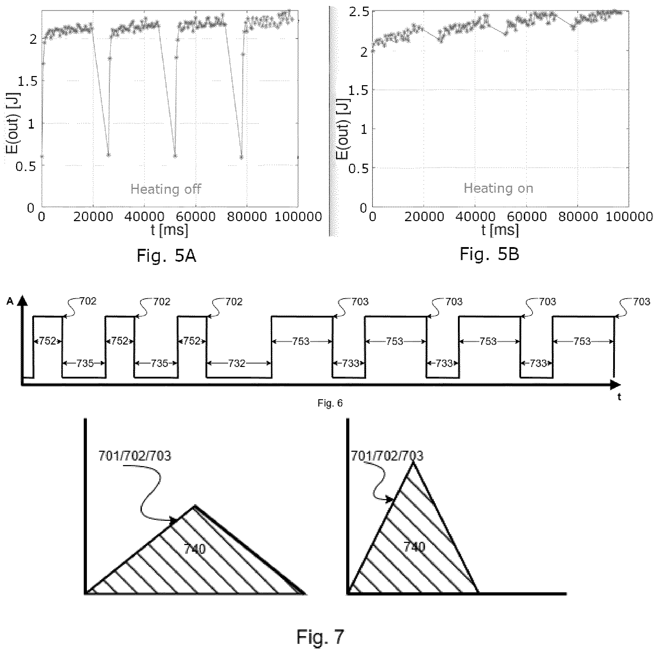

Preheating or heating may work by thermally priming the laser rod, such that the temperature gradient in the laser rod 234 is substantially consistent during laser beam pulses. Without preheating or heating, the temperature gradient inside the solid state laser rod may change during the first few of laser beam pulses due to cooling and flash lamp flashes, which may affect the parameters of the produced laser beam pulses (e.g. energy, fluence, duration and so on). Thus, without preheating or heating of the laser rod, the first few laser beam pulses may have energy or fluence lower than 80 % or 85 % or 90 % of the energy or fluence set by the operator (e.g. via the user interface). Exemplary graphs of laser power throughout multiple pulses of a laser source with and without heating the laser rod are shown in Figs. 5A, 5B. The difference in power for the first few pulses is non-negligible. Fig. 5 shows that without the heating, the energy of first few pulses can be even below 25% of wanted energy. In contrast, in case of heating, the energy of first few pulses is well above 85% of wanted energy as shown in Fig. 5B, which corresponds to the medical regulations.

During preheating, the flash lamp charger 232 delivers current to the respective flash lamp 233 in a preheat current pulse 701, afterward (almost instantaneously) the flash lamp transmits the energy as light in a broad-spectrum preheat flash 711 to the laser rod 234. In one aspect, the flash lamp charger 232 may provide one preheat current pulse 701 to the flash lamp before one or more therapeutic current pulses. Thus, the flash lamp may produce one preheat flash 711 to the laser rod 234 before one or more therapeutic flashes 713. In another aspect, the flash lamp charger 232 may provide plurality of preheat current pulse 701 to the flash lamp before one or more therapeutic current pulses. Thus, the flash lamp may provide plurality of preheat flashes 711 to the laser rod 234 before one or more therapeutic flashes 713.

During heating, the flash lamp charger 232 delivers current to the respective flash lamp 233 in a heat current pulse 702, afterward (almost instantaneously) the flash lamp transmits the energy as light in a broad-spectrum heat flash 712 to the laser rod 234. In one aspect, the flash lamp charger 232 may provide one heat current pulse 702 to the flash lamp before one or more therapeutic current pulses. Thus, the flash lamp may provide one heat flash 712 to the laser rod 234 before one or more therapeutic flashes 713. In another aspect, the flash lamp charger 232 may provide plurality of heat current pulse 702 to the flash lamp before one or more therapeutic current pulses. Thus, the flash lamp may provide plurality of heat flashes 712 to the laser rod 234 before one or more therapeutic flashes 713.

In one aspect, the preheating may be done by the flash lamp 233 delivering to the laser rod 234, in the preheat flash 711, an amount of energy lower than the laser activation threshold of the laser rod 234, thus the laser rod is thermally primed, but not producing laser beam pulses.

In one aspect, the heating may be done by the flash lamp 233 delivering to the laser rod 234, in the heat flash 712, an amount of energy lower than the laser activation threshold of the laser rod 234, thus the laser rod is thermally primed, but not producing laser beam pulses.

In one aspect, the heat current pulse 702 or preheat current pulse 701 may be shorter than the therapeutic current pulse 703. Thus, the heat flash 712 or the preheat flash 711 may be shorter than the therapeutic flash 713, and thus the flash lamp 233 may only deliver an amount of energy to the laser rod 234, that is lower than the laser activation threshold. In another aspect, during preheating, when the laser rod is completely unprimed, the preheat current pulse may be longer than the therapeutic current pulse.

In one aspect, the preheat current pulse length 751 or the heat current pulse length 752 may be adjusted by the main unit 21 and their values may be in a range of 10 % to 125 % or in a range of 20% to 120 % or in a range of 30 % to 115 % of the therapeutic current pulse length 753. Additionally, the length of the heat current pulse 752 may be in a range of 10 % to 99 % or in a range of 20 % to 97% in a range of 30 % to 95 % or in a range of 60 % to 95 % of the length of the therapeutic current pulse 753. An exemplary graph of a few heat current pulses and subsequent therapeutic current pulses delivered to a flash lamp are shown in Fig. 6.

In another aspect, the length of the preheat flash 711 or the heat flash 712 may be in a range of 10 % to 125 % or in a range of 20 % to 120 % or in a range of 30 % to 115 % of the length of the therapeutic flash 713. Additionally, the length of the heat flash 712 may be in a range of 30 % to 95 % of the length of the therapeutic flash 713.

In yet another aspect, the preheat current pulse length 751 or the heat current pulse length 752 may be in a range of 0.001 ms to 33 ms or in a range of 0.002 ms to 30 ms or in a range of 0.003 ms to 24 ms. In yet another aspect, the length of the preheat flash 711 or the heat flash 712 may be in a range of 0.001 ms to 33 ms or in a range of 0.002 ms to 30 ms or in a range of 0.003 ms to 24 ms or in the range of 0.003 ms to 1 ms or in the range of 10 ms to 20 ms.

In another aspect, the flash lamp charger 232 may deliver a current of lower amplitude to the flash lamp 233 during the heat current pulse 702 or preheat current pulse 701 than during the therapeutic current pulse 703 and thus the energy delivered to the laser rod 234 by the preheat flash 711 or the heat flash 712 may be below the laser activation threshold.

In one aspect, the amplitude of the preheat current pulse 701 or the heat current pulse 702 may be adjusted by the main unit 21 and their values may be in a range of 10 % to 99 % or in a range of 20% to 95% or in a range of 30% to 90% of the amplitude of the therapeutic current pulse 703.

In another aspect, the amplitude of the preheat current pulse 701 or the heat current pulse 702 may be in a range of 1 A to 2000 A or in a range of 10 A to 1500 A or in a range of 25 A to 1000 A or in a range of 50 A to 500 A.

In yet another aspect, the preheat current pulse length 751 or the heat current pulse length 752 may be different than the therapeutic current pulse length 753 and the amplitude of the preheat current pulse 701 or the heat current pulse 702 may be different than the amplitude of the therapeutic current pulse 703.

In one aspect, the energy delivered to the flash lamp 233 by the flash lamp charger 232 during the preheat current pulse 701 may be different than the energy delivered to the flash lamp 233 by the flash lamp charger 232 during the heat current pulse 702. In another aspect, the energy delivered to the flash lamp 233 by the flash lamp charger 232 during the preheat current pulse 701 may be the same as the energy delivered to the flash lamp 233 by the flash lamp charger 232 during the heat current pulse 702

Additionally, other parameters that may affect the heating and preheating processes may be the frequency of the preheat current pulses or the heat current pulses and the length of time before the first therapeutic current pulse after the last heat current pulse (heat-shoot delay 732) or the length of time before the first therapeutic current pulse after the last preheat current pulse (preheat-shoot delay 731).

In one aspect, the frequency of the heat current pulses or preheat current pulses may be adjusted. The frequency of the heat current pulses or preheat current pulses may or may not be the same as the frequency of the therapeutic current pulses by the respective flash lamp charger. In one aspect, the frequency of the heat current pulses or preheat current pulses may be in a range of 0.03 Hz to 500 Hz or in a range of 0.5 Hz to 350 Hz or in a range of 1 Hz to 200 Hz. Thus, the amount of time between the preheat current pulses (preheat-preheat delay 734) or the amount of time between the heat current pulses (heat-heat delay 735) may be in a range of 0.001 ms to 34 s or in a range of 1 ms to 20 s or in a range of 1 s to 10 s.

In another aspect, the heat-shoot delay 732 or the preheat-shoot delay 731 may be adjusted by the control unit 21 or by the operator. Their length may depend of the length of time between subsequent laser beam pulses (shoot-shoot delay 733) or may be adjusted freely.

In one aspect, the length of the heat-shoot delay 732 or the preheat-shoot delay 731 may be a multiple of the shoot-shoot delay 733. This multiple may be in a range of 0.1 to 10 times or in a range of 0.2 to 6 times or in a range of 0.3 to 3 times.

In another aspect, the length of the heat-shoot delay 732 or the preheat-shoot delay 731 may be in a range of 0.1 ms to 100 s or in a range of 0.2 ms to 60 s or in a range of 0.3 ms to 30 s or in a range of 0.001 ms to 1 s.

In one aspect, the frequency of the heat current pulses and the heat-shoot delay may be different from the frequency of the preheat current pulses and the preheat-shoot delay. In another aspect, these parameters may be the same for preheat current pulses and heat current pulses.

In one example, the first laser source 234a (e.g. the non-ablative laser source) and the second laser source 234b (e.g. the ablative laser source) may be preheated and heated. The parameters for heating and preheating of both laser sources may correspond to the ranges given above.

In one example, the first laser source 234a (e.g. the non-ablative laser source) or the second laser source 234b (e.g. the ablative laser source) may be preheated and heated. The parameters for heating and preheating of both laser sources may correspond to the ranges given above.

In one aspect, the control unit 21 may have a preheating or heating timer, which turns off preheating or heating to conserve energy and ensure the longevity of the laser rod 234 and flash lamp 233. The preheating and heating timer may have a length in the range of 1 s to 10 min or in a range of 30 s to 5 min or in a range of 2 min to 4 min.

In one aspect, since the substantially rectangular profile of the electric current in the preheat current pulse 701, in the heat current pulse 702 and in the therapeutic current pulse 703 may not be exactly rectangular, there may be edges of the current pulse (701, 702 or 703) with a given slope on both sides of the current profile of the current pulses (rising edge, and falling edge). These slopes may depend on the specific construction of the flash lamp 233 and the flash lamp charger 232 and thus may vary in each device. In one example, when using a laser rod that requires a substantial amount of energy to charge during the flash lamp flash, the time required to charge the flash lamp by the current provided by the flash lamp charger is much higher than the time needed for the current to build up through the (rise) slope. In such examples, the effect of different (rising) slopes is negligible and the heat current pulses, the preheat current pulses and the therapeutic current pulses on each device may be very similar.

In another example, however, when using a laser rod that requires less energy, the current to the flash lamp may have to be cut off while the current is still building up through the (rising) slope. In such examples, due to the possibility of different current slopes in each device, the resulting heat flash, preheat flash or therapeutic flash may be different in each device. Thus, the gradient of the temperature inside the laser rod at the excitation or priming of the laser rod may be different. And, thus, the first few laser beam pulses and the heat and preheat current pulses may also differ in each device. This variability may be prevented by calibrating each device based on its unique current profile slope such that the area underneath the current profile 740 is the same on each device, as shown in Fig. 7. This area represents the energy, if the device is kept at a constant voltage, delivered to the flash lamp and thus the energy delivered to the laser rod. The area under the current profile 740 may be adjusted by adjusting the length of the heat current pulse, preheat current pulse or the therapeutic current pulse.

In another aspect, the preheating or heating procedure may be done by delivering enough energy to the laser rod 234 such that it passes the laser activation threshold and fires a laser beam pulse. This laser beam pulse may then be fired into a built-in shutter such that the waveguide 3 and the applicator 4 and thus the tissue of the patient does not receive the radiation produced by the laser source pulse. However, preheating or heating the laser in such a way is disadvantageous, since it decreases the longevity of the laser rod and the flash lamp and increases energy consumption, since both receive more energy than in the aspects outlined above. Even if absorbed or diverted, optical elements (including the shutter) suffer thermal and mechanical stress, which could degrade them over time. Furthermore, risk of shutter failure or misalignment leading to unintended beam delivery to the patient and the complexity in shutter timing and synchronization increases the risk of unsafe operation.

The main unit 2 may further comprise an aiming beam source 25. An aiming beam may be generated by the aiming beam source 25. In one aspect the aiming beam source 25 may comprise a laser diode. In another aspect, the aiming beam source 25 may comprise a gas laser or a solid state laser or other types of lasers known in the art or any other sources of light known in the art. The aiming beam may have a wavelength in a visible spectrum, for example in a range of 600 nm to 700 nm or in a range of 615 nm to 685 nm or in a range of 630 nm to 670 nm. Alternatively, the aiming beam may have a wavelength in a range of 400 nm to 700 nm. The aiming beam has no clinical effect on the treated tissue and may serve as a tool to mark a target zone in the treatment area to be treated so that the operator knows which exact area will be irradiated.

In one aspect, the apparatus 1 (e.g. the main unit 2) may comprise beam samplers (e.g. low-reflectivity optical window, pellicle, or a beam splitter) used to manipulate the laser beams. In one aspect, the beam reflectance of a beam sampler may be in a range of 0.1% to 50% or in a range of 0.5% to 30% or in a range of 1% to 10%.

In another aspect, the wavelength range of a beam sampler may be in a range of 350 nm to 3500 nm, or in a range of 400 nm to 3250 nm, or in a range of 450 nm to 3000 nm.

In yet another aspect, the reflection efficiency of a beam sampler may be in a range of 30% to 99%, or in a range of 50% to 98%, or in a range of 70% to 95%. The transmission efficiency of a beam sampler may be in a range of 30% to 99%, or in a range of 50% to 98%, or in a range of 70% to 95%.

The main unit 2 may further comprise a plurality of optical output measurement units (e.g. a first optical output measurement unit 24a and a second optical output measurement unit 24b). In one aspect, a part of the laser beam generated by the first laser source 23a (e.g. the non-ablative laser beam) may be split from the main beam using a beam sampler and/or other optical elements and may be directed into the first optical output measurement unit 24a. In one aspect, the first optical output measurement unit 24a may be configured to detect the intensity of the beam at wavelengths in a range of 1300 nm to 1800 nm or in a range of 1400 nm to 1650 nm or in a range of 1520 nm to 1560 nm. Alternatively, the first optical output measurement unit 24a may be configured to detect the intensity of the beam at wavelengths in a range of 1000 nm to 3000 nm.

In another aspect, a part of the laser beam generated by the second laser source 23b (e.g. the ablative laser beam) may be split from the main beam using a beam sampler and/or other optical elements and may be directed into the second optical output measurement unit 24b. In one aspect, the second optical output measurement unit 24b may be configured to detect the intensity of the beam at wavelengths in a range of 2500 nm to 3500 nm or in a range of 2700 nm to 3200 nm or in a range of 2900 nm to 2980 nm. Alternatively, the second optical output measurement unit 24b may be configured to detect the intensity of the beam at wavelengths in a range of 1500 nm to 3500 nm or in a range of 9000 nm to 11000 nm or in a range of 1900 nm to 2000 nm.

The plurality of optical output measurement units (e.g. the first optical measurement unit 24a and the second optical measurement unit 24b) may be connected to the control unit 21. The plurality of optical output measurement units (e.g. the first optical measurement unit 24a and the second optical measurement unit 24b) may supply information about the respective laser beams to the control unit 21. The control unit 21 may adjust the parameters of the treatment based on the information from the plurality of optical output measurement units.

The control unit in connection with one or more optical output measurement units may be configured to perform calibration of one or more laser sources. Laser calibration is the process of adjusting and verifying the output parameters of a laser system to ensure it operates within specified performance standards. It typically involves measuring the laser's power, wavelength, beam alignment, and pulse characteristics using precise instruments such as power meters, wavelength meters, and beam profilers. Calibration helps maintain accuracy, consistency, and safety of the apparatus 1. Regular calibration compensates for variations caused by component aging, thermal effects, or alignment shifts, thereby ensuring reliable and repeatable laser performance. The calibration may be executed when the device 1 is turned on, before, during or after the treatment. In one aspect, the calibration is provided automatically by the control unit.

Dichroic mirrors may be used to manipulate the laser beams. In one aspect, the beam samplers (e.g. beam splitters) may comprise the dichroic mirrors. The dichroic mirror may comprise a substrate material, dielectric-coating layers, anti-reflecting (AR) coating, or protective coating.

The dichroic mirror substrate may comprise high-quality optical glass or crystal that provides mechanical stability and transparency, for example fused silica, glass, sapphire, zerodur or any other suitable material. Dielectric coating layers of dichroic mirror may comprise multiple layers of dielectric materials on the substrate, for example TiO₂ (Titanium dioxide), SiO₂ (Silicon dioxide), Ta₂O₅ (Tantalum pentoxide), Al₂O₃ (Aluminum oxide).

In one aspect, the transmission band of a dichroic mirror may be in a range of 400 nm to 1700 nm, or in a range of 450 nm to 1550 nm, or in a range of 500 nm to 800 nm. Alternatively, the transmission band of a dichroic mirror may be in a range of 400 nm to 3000 nm or in a range of 1500 nm to 3500 nm or in a range of 9000 nm to 11000 nm or in a range of 1560 nm to 3500 nm.

In another aspect, the reflection efficiency of a dichroic mirror may be in a range of 70% to 99.9%, or in a range of 80% to 99.5%, or in a range of 85% to 99%. The transmission efficiency of the dichroic mirror may be in a range of 70% to 99%, or in a range of 75% to 97%, or in a range of 80% to 96%.

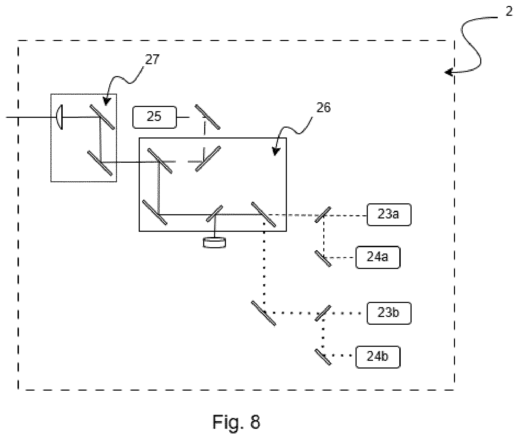

The main unit 2 may comprise a beam combiner 26. In one aspect, the beam combiner 26 may comprise a dichroic mirror beam combiner. The dichroic mirror beam combiner may combine a plurality of laser beams (e.g. the ablative, non-ablative) and/or an aiming beam into a single combined laser beam of multiple wavelengths by using dichroic mirrors and other optical elements. For example, a dichroic mirror that substantially reflects the wavelength of the ablative beam and substantially transmits the wavelength of the non-ablative beam (or vice versa) may be used to combine the ablative and non-ablative beams. Further a dichroic mirror that substantially reflects the wavelengths of the ablative and non-ablative laser beams and substantially transmits the wavelength of the aiming beam (or vice versa) may be used to combine the aiming beam with the already combined ablative and non-ablative beams and thus the result is the combination of all three beams into one. One example of such an optical assembly is shown in a diagram in Fig. 8 including the laser sources 23a and 23b, optical output measurement units 24a and 24b, and a beam combiner 26 as well as a collimator 27.

In another aspect, the beam combiner 26 may utilize different optical elements such as lenses or mirrors or other optical elements as the main way of combining beams.

The beam combiner offers the advantage of having an option to apply a plurality of laser beams (e.g. the ablative, non-ablative) in a spatially coordinated manner, e.g. in a single combined laser beam. The optical elements that make up the beam combiner 26 may be mounted to kinematic or fixed mounts to assure a precise combination of more than one laser beam (e.g. the combination of non-ablative and ablative) and/or aiming beam. In one aspect, 1 to 100 optical elements are mounted to a kinematic mount and 1 to 100 optical elements are mounted to a fixed mount. In another aspect, 2 to 50 optical elements are mounted to a kinematic mount and 2 to 50 optical elements are mounted to a fixed mount. The optical elements may include mirrors, dichroic mirrors, apertures, lenses or other optical elements.

In one aspect, the spatially coordinated manner refers to a configuration in which the central axes of the plurality of laser beams are coincident, such that the respective beam centers are directed toward a common target point within the target zone. In some implementations, this may result in at least a partial overlap of their cross-sectional areas on the target zone.

In another aspect, the spatially coordinated manner may refer to a configuration in which the entire cross-sectional areas of the plurality of laser beams substantially overlap at the target zone, such that the beams are concentrically aligned at the point of incidence. This may mean that the plurality of the laser beams in the single combined beam irradiates the same area of the target zone.

Ensuring precise alignment of the plurality of laser beams (e.g. the ablative, non-ablative) and/or aiming beams can be particularly challenging when they are to be transmitted to the tissue of the patient (e.g. skin) over a non-trivial distance. Kinematic mounts are instrumental in maintaining the collinearity of these beams under such conditions, as they provide stable, repeatable pivot points that can be reliably returned to a known position despite repeated movement or external forces.

The device may comprise a collimator 27, e.g. inside the main unit 2. The collimator 27 may be configured to collimate the laser beam (e.g. align and maintain the single combined laser beam in an at least substantially parallel form or in parallel form). The collimator may include one or more optical elements, such as lenses (e.g. convex or aspheric lenses), fiber collimators, mirrors, or reflective collimation systems, designed to minimize beam divergence and optimize energy delivery. By ensuring a collimated beam, the system enhances targeting precision, reduces unintended dispersion, and improves the safety and efficacy of laser-based medical treatments.

In one aspect, a single collimator may be used to collimate all laser beams from all laser sources, e.g. the non-ablative and ablative, or aiming beam, simultaneously, after they have been combined using the beam combiner 26, e.g. into a single combined laser beam. This arrangement allows for easy tuning of the device and allows the device to stay tuned for a longer period of time. The collimator in this configuration may be configured to ensure that all of the laser beams (e.g. the non-ablative and ablative of the single combined laser beam) have a substantially identical radius at the end of the waveguide 3 (e.g. articulated arm). Using a single collimator is advantageous in reduction of the cumulative optical losses; it reduces thermal drift and mechanical misalignment risks, which is essential in a clinical setting where precision is non-negotiable; and a unified collimation ensures the beam profile and divergence are consistent for all beams within the single combined laser beam, which is crucial when multiple beams (e.g., treatment and aiming) are delivered through a single (uniform) optical path.

In one aspect, the radii differences at the end of the waveguide 3 (e.g. articulated arm) of all of the laser beams in the plurality of combined laser beams (e.g. the non-ablative and ablative beams of the single combined laser beam), which are collimated by a single collimator, may be in a range of 0.1% to 30% or in a range of 0.2% to 25% or in a range of 0.3% to 20%. By providing consistent beam radii, the collimator ensures uniform energy distribution, and improves overall beam quality and performance. Such optimization may be technically challenging due to the different characteristics of each of the plurality of laser beams. The characteristics of each laser beam may change even when switching between different treatment parameters for the given laser source, e.g. the energy produced in each pulse.

In one aspect, the radius of each laser beam in the plurality of laser beams at the end of the waveguide may change when switching between different treatment parameters. The radius may change within a range of 1 % to 20 % or in a range of 5% to 15 % or in a range of 7 % to 13 %.

In one aspect, the parameter characterizing each laser beam (e.g. the non-ablative and ablative) may be a beam propagation ratio (M²). The beam propagation ratio is a measure of laser beam quality, it relates to the divergence of a laser beam. The beam propagation ratio for each laser beam in the plurality of laser beams may be in a range of 1 to 100 or in a range of 3 to 80 or in a range of 6 to 62. By introducing proper configuration of the plurality of laser sources, the beam propagation ratio of each laser beam may be lowered and the use of a single collimator permitted.

In one example the non-ablative laser beam may have a beam propagation ratio in a range of 10 to 100 or in a range of 20 to 80 or in a range of 33 to 62. In another example the ablative laser beam may have a beam propagation ratio in a range of 1 to 50 or in a range of 3 to 40 or in a range of 6 to 27.

In one aspect, the single collimator may comprise a specially coated spherical lens. In one aspect, the radius of curvature of the spherical lens may be in a range of 50 mm to 250 mm or in a range of 75 mm to 225 mm or in a range of 100 mm to 200 mm. In one aspect, the diameter of the spherical lens may be in a range of 10 mm to 50 mm or in a range of 15 mm to 40 mm or in a range of 20 mm to 30 mm. In one aspect, the spherical lens may be made of a material with an index of refraction in a range of 1 to 2 or in a range of 1.2 to 1.8 or in a range of 1.3 to 1.6. In one aspect, the lens may have a focal length in a range of 100 mm to 1 m or in a range of 200 mm to 900 mm or in a range of 300 mm to 500 mm. In one aspect, the spherical lens may have a thickness in a range of 0.1 mm to 10 mm or in a range of 1 mm to 7 mm or in a range of 2.5 mm to 4 mm. These parameters along with the optical distance travelled by each of the plurality of laser beams (e.g. the non-ablative, and ablative) before interacting with the single collimator may, due to the divergent nature of almost all laser beams, affect the radius of the beam at the end of the waveguide (e.g. articulated arm).

In one example, the lens may be plano-convex lens with the flat side being the side which the laser beams interact with first. The lens may have a transmission of the ablative laser greater than 90 % or 95 % or 98 % and the transmission of the non-ablative laser greater than 90 % or 95 % or 99 %. The convex side may have a radius of curvature in a range of 100 mm to 200 mm and the whole lens may have a diameter in a range of 20 mm to 30 mm. The lens may have a focal length in a range of 300 mm to 500 mm. The lens may have a center thickness in a range of 3 mm to 4 mm and an edge thickness in a range of 2.5 mm to 3.5 mm. The lens may have a refractive index in a range of 1.3 to 1.6.

To achieve convergence of beam diameters at the target plane, the axial displacement (i.e. the optical distance) of each laser source from the collimator may be parametrically determined based on the emitted wavelength, the beam waist radius and the beam propagation ratio of the respective laser. In one aspect, the optical distances from the plurality of laser sources to the collimator are adjusted such that the differences in beam radii at the output of the articulated arm is in a range of 0.1% to 30% or in a range of 0.2% to 25% or in a range of 0.3% to 20%.

In one aspect, the axial displacement is increased for longer wavelengths, meaning that the laser source having a longer wavelength has longer optical distance from the collimator than the laser source with shorter wavelength.

In another aspect, the axial displacement is increased for larger beam propagation ratios, meaning that the laser source having a larger beam propagation ratio has longer optical distance from the collimator than the laser source with lower beam propagation ratio.

In another aspect, the axial displacement is increased larger waist radii, meaning that the laser source having a larger waist radii has longer optical distance from the collimator than the laser source with lower waist radii.

In another aspect, the axial displacement is decreased for longer wavelengths, meaning that the laser source having a longer wavelength has shorter optical distance from the collimator than the laser source with shorter wavelength.

In another aspect, the axial displacement is decreased for larger beam propagation ratios, meaning that the laser source having a larger waist radii has shorter optical distance from the collimator than the laser source with lower waist radii.

In another aspect, the axial displacement is decreased for larger waist radii, meaning that the laser source having a larger waist radii has shorter optical distance from the collimator than the laser source with lower waist radii.

In a further aspect, the axial displacement is determined by the product of the beam propagation ratio and the waist radius. In one aspect, a larger value of the product of the beam propagation ratio and the waist radius corresponds to a longer axial displacement. In another aspect, a larger value of the product of the beam propagation ratio and the waist radius corresponds to a shorter axial displacement. Likewise, a smaller value of the product of the beam propagation ratio and the waist radius may result in either a longer or shorter axial displacement. All such variations are within the scope of the invention, allowing precise axial positioning for laser sources with differing beam quality products to achieve uniform collimation.

In one aspect, the optical distance from each of the plurality of laser sources to the collimator may be in a range of 10 mm to 1.5 m or in a range of 100 mm to 800 mm or in a range of 200 mm to 700 mm or in a range of 400 mm to 600 mm.

In one aspect, the difference in the optical distances of two laser sources from the collimator may be in the range of 1 mm to 50 cm or in the range of 1 mm to 25 cm or in the range of 1 mm to 10 cm or in the range of 2 mm to 50 mm or in the range of 2 mm to 14 mm or in the range of or 4 mm to 8 mm.

In one example the optical distance from the first laser source 23a (e.g. the source of the non-ablative laser beam) to the collimator may be in a range of 450 mm to 550 mm. In another example, the optical distance from the second laser source 23b (e.g. the source of the ablative laser beam) to the collimator may be in a range of 450 mm to 500 mm.

In another aspect, the single collimator may comprise other optical elements configured to collimate each of the laser beams in the plurality of laser beams. Such optical elements may include aspheric lenses, gradient-index lenses, cylindrical lenses, parabolic mirrors, diffractive optical elements, prisms, micro-lens arrays and axicons and other optical elements known in the art.

In another aspect, two or more collimators may be used to collimate two or more laser beams, respectively, before they are ultimately combined into a single combined laser beam using the beam combiner 26. For example, to collimate 1 to 5 different laser beams, 1 to 5 different collimators may be used. In another example, a first collimator may be used to collimate a first laser beam (e.g. non-ablative beam), a second collimator may be used to collimate a second laser beam (e.g. ablative beam) and a third collimator may be used to collimate an aiming beam. Each of the two or more collimators may comprise a collimator as mentioned above.

In one aspect, alignment of the laser beam and maintaining it in a substantially parallel form may be achieved by using a beam shaper. In one aspect, the apparatus may use beam expanders or aspheric lenses to shape the input laser beam width before combining.

In one aspect, one or more optical elements within the laser apparatus 1 may be provided with specialized optical coatings configured to enhance the performance of the system. Such coatings may be applied to lenses, mirrors, beam combiners, collimators, or other optical components and are selected to improve key optical characteristics such as transmittance, reflectivity, and spectral selectivity at specific wavelengths. For instance, anti-reflective coatings may be used on transmissive optics to maximize energy throughput and reduce energy losses, while high-reflectivity coatings may be applied to reflective elements to ensure efficient beam guidance and minimal dispersion. These coatings may be dielectric, metallic, or hybrid in composition and may be tailored for the operating wavelengths of the laser beams, such as those in the ranges of 1200 nm to 1650 nm and 1700 nm to 4000 nm. The application of such coatings not only improves overall device efficiency and output consistency but also contributes to the durability and thermal stability of the optical components under repeated high-intensity use.

The main unit 2 may further comprise a foot switch 28. The foot switch 28 may be used to supply information to the control unit 21 from the operator. In one aspect, the control unit 21 may be configured such that when the foot switch is activated, a signal is sent to the one or more flash lamp chargers from the plurality of flash lamp chargers, after which the one or more flash lamp chargers produce a therapeutic current pulse, so that the respective flash lamp 233 or flash lamps create a therapeutic flash 713. This flash then stimulates the respective laser rod 234 to create a laser beam pulse. These laser beam pulses may then be applied to the tissue of the patient (e.g. skin) in a simultaneous, successive or overlapping method.

In another aspect, the control unit 21 may be configured such that, when the foot switch 28 is activated, the apparatus will produce laser beam pulse stacks that happen in a simultaneous, successive or overlapping method with a given frequency. Thus, when the foot switch is activated, the control unit will periodically send signals to the flash lamp chargers and other necessary components to produce laser beam pulses. The frequency of each laser beam pulse stack may be in a range of 0.1 Hz to 50 Hz or in a range of 1 Hz to 40 Hz or in a range of 1.5 Hz to 30 Hz or in a range of 0.1 Hz to 10 Hz or 0.5 Hz to 7 Hz or 1 Hz to 5 Hz.

The main unit 2 may be connected to the applicator 4 by the waveguide 3. The waveguide may be configured such that the operator is able to position the applicator to the required spot on the patient.

In one aspect, the waveguide 3 may be configured such that the collimation of the beam made up of more than one beam (e.g. the non-ablative, ablative, or aiming beam) is conserved during the transfer of the beam to the applicator. Thus, the output of the at least one collimator may be precisely aligned with an input of the waveguide 3.

In another aspect, the waveguide 3 may be configured such that the collimation of the separate beams traversing the waveguide is conserved. The separate beams may include the non-ablative, ablative and aiming beams. Thus, the output of each collimator may be aligned with an input of the waveguide.

In one aspect, the waveguide 3 may have an optical length in a range of 0.1 m to 10 m or in a range of 0.2 m to 5 m or in a range of 0.5 m to 4 m or in range of 1 m to 2.5 m.

In another aspect, the waveguide 3 may have a length in a range of 0.1 m to 10 m or in a range of 0.2 m to 5 m or in a range of 0.5 m to 4 m or in range of 1 m to 2.5 m.

In one aspect, the waveguide 3 may comprise an articulated optical arm. The articulated optical arm may have 1, 2, 3, 4, 5, 6, 7, 8, 9, 10 or up to 20 joints, wherein every joint may contain a 45 degree mirror. In one aspect the articulated optical arm may have 2 to 20, or 3 to 15, or 4 to 12 or 5 to 10 joints, wherein every joint may contain a 45 degree mirror.

In another aspect, the waveguide 3 may comprise one or more optical fibers or a fiber bundle. Each fiber may be a single mode or a multimode fiber with a core diameter in a range of 0.1 to 80 µm or in a range of 0.5 to 50 µm or 1 to 40 µm or 2 to 30 µm. Alternatively, each fiber may have a core diameter in a range of 20 to 2000 µm or in a range of 30 to 1800 µm or in a range of 40 to 1700 µm or in a range of 50 to 1500 µm. The fiber bundle may include 1, 2, 3, 4, 5, 6, 7, 8, 9, 10, 11, 12, 13, 14, 15, 16, 17, 18, 19, 20 or up to 100 above mentioned fibers and may be in straight, bifurcated (Y-cable), or fan-out configurations and may have round or square or linear bundle end configurations.

A distal end of the waveguide 3 may be configured to be connected to the applicator 4.

In one aspect, the applicator 4 is fixed to the waveguide 3. In one example, only a single applicator 4 may be used for the entirety of the therapy.

In another aspect, the applicator 4 is interchangeable. Therefore, a plurality of applicators 4 may be used during the therapy, or the operator of the device 1 may select which applicator 4 will be used for which therapy. The applicators may be configured to be effective for different therapies.

A quick-connect system may be used between the distal end of the waveguide 3 and the applicator 4. Different applicators 4 may be used during the therapy. The different applicators 4 may include a fractional treatment applicator, or a full field applicator, or other types of applicators (e.g. applicator suitable for vaginal treatment, dentistry or ophthalmology).

The quick-connect system may be placed between the distal end of the waveguide (for example, an articulated optical arm) and the proximal end of an applicator. This system may be configured to enable fast, secure, and repeatable attachment or detachment of the applicator.

In one aspect, the quick connect system may be configured to maintain multiple concurrent connections (e.g. an optical connection, an electrical connection and a vacuum hose connection). The optical connection may align the distal end of the waveguide 3 with corresponding optical components in the applicator 4 so that the laser beam or beams are transmitted with minimal loss or misalignment. For example, in an articulated optical arm, a final turning mirror or focusing lens may mate with a corresponding receiving lens or optical interface in the applicator. Alternatively, in fiber-based waveguides, a fiber end ferrule may seat in a corresponding fiber receptacle within the applicator. The electrical connection may provide an interface that may be connected with a corresponding contact array in the applicator. This contact array may include one or more contacts, pins, pads, or pogo pins configured to supply power and data signals to a PCB or other electronic components housed within the applicator. In certain aspects, the interface may be sealed or shielded to reduce the risk of contamination or electromagnetic interference. Via the electrical connection, the control unit may recognize which applicator is connected to the waveguide and provide specific protocols on the user interface. The vacuum or suction hose connection may be configured to accommodate a suction or vacuum hose, which may run alongside or through the waveguide assembly. Upon attachment, this hose may be connected with a corresponding inlet port on the applicator, allowing continuous removal of debris and aerosols generated during laser therapy. In some aspects, the hose connection may include an O-ring or gasket to establish a leak-proof seal.

In one aspect, to ensure the applicator 4 remains firmly attached during operation, the quick connect system may incorporate a locking or latching mechanism.

In one aspect, a recess on the applicator side may get engaged by a latching mechanism on the waveguide side, providing secure coupling. For example, the waveguide distal end may comprise 1 to 10 or 1 to 7 or to 2 to 5 (protruding) balls and the applicator may comprise a respective number of locking grooves. Thus, when the applicator is connected to the waveguide the locking grooves may snap into the (protruding) balls, which provides the lock of the applicator. To release the applicator, the user may pull back tabs on the applicator, which compresses a spring and disengages the locking mechanism. This releases the grip, allowing the applicator to be easily pulled out. The number and the distribution of the balls and grooves may be configured to let the applicator slide in in only one preferred configuration, thus enabling easy connection of all the connectors (optical, electrical or vacuum) between the applicator and the waveguide.

In another example, the waveguide distal end may comprise 1 to 10 or 1 to 7 or to 2 to 5 (protruding) balls and the applicator may comprise a locking groove. Thus, when the applicator is connected to the waveguide the (protruding) balls may engage the locking groove, which provides the lock of the applicator. To release the applicator, the user may pull back tabs on the applicator, which compresses a spring and disengages the locking mechanism. This releases the grip, allowing the applicator to be easily pulled out. Additional pins on the waveguide side and grooves on the applicator side may be configured to let the applicator slide in in only one preferred configuration, thus enabling easy connection of all the connectors (optical, electrical or vacuum) between the applicator and the waveguide.

In another aspect, a quick connect system may comprise a spring loaded collar.

In yet another aspect, other mechanically secure features may be employed, such as bayonet-style fittings, lever or cam locks, or magnetic couplings. These or other suitable designs may be implemented to prevent unintended detachment of the applicator during use, especially under conditions of vibration or repeated handling.

In certain aspects, the quick-connect system may include sealing gaskets around one or more of the connection points to prevent dust, fluids (such as cooling fluid or skin debris), or other contaminants from entering the internal components of the system. This sealing may be particularly useful in maintaining cleanliness and ensuring consistent optical transmission over repeated treatment sessions, and the electrical contacts may likewise be housed within a protective shroud or recessed cavity that is sealed when mated, thereby reducing exposure to moisture or debris.

In another aspect, split or modular connectors may be utilized. For example, the waveguide's distal end may have a primary latch to secure the applicator's optical interface and a secondary or peripheral connection port for the vacuum hose and electrical leads. Although described in conjunction with an articulated optical arm, the same quick-connect system may be adapted for use with fiber-optic cables, rigid waveguides, or hybrid systems having both fiber and articulated segments. By offering an integrated, quick-connect design that addresses optical alignment, power and data exchange, and vacuum hose routing in a single assembly, the disclosed system simplifies clinical workflow, reduces setup time, and ensures robust, repeatable coupling across various applicator types.

The applicator 4, coupled to the waveguide 3, may be configured to deliver the laser beam to the target zone in the treatment area on the patient. In one aspect, the applicator may be a full field applicator for treating large, continuous areas. In another aspect, the applicator may be a fractional applicator, for delivering patterned, localized treatment. In yet another aspect, the applicator may be a spot applicator for highly targeted therapy. In yet another aspect, other applicator types commonly known in the art, such as scanning applicators, or array-based applicators, may also be employed. Additionally, the applicator may be an applicator specialized for a single type of treatment. The applicator may be specialized for, but the specialization is not limited to, gynecological applications, dentistry, ophthalmology applications, treatment of snoring and other specialized applications.

In one aspect, of the full field applicator, the target zone may mean the area irradiated by the laser beam on the tissue (e.g. surface of a skin).

In one aspect, with respect to a full-field applicator, the target zone may refer to the area of tissue (e.g., the surface of the skin) that is directly irradiated by the laser beam during a treatment event. The target zone corresponds to the continuous or contiguous region exposed to laser energy, typically without intervening untreated areas, as contrasted with a fractional pattern. The shape and size of the target zone may be defined by the beam geometry, the optical focusing elements, and the energy delivery parameters of the applicator.

In another aspect, in case of fractional applicator, the target zone may refer to the area on a tissue surface (e.g. skin) that is bounded by the outermost fractional delivery points (e.g. microbeams or micro treatment zones) of a given array or pattern. The target zone encompasses the cumulative lateral extent of the patterned energy delivery in a single (one) pulse or actuation, including both treated and untreated regions within the boundary defined by the outermost points. The target zone may be rectangular, square, circular, hexagonal, or of arbitrary shape depending on the arrangement of the fractional beams.

Yet in another aspect, the target zone may mean the area bounded by a contact pad 461 of a spacer 46.

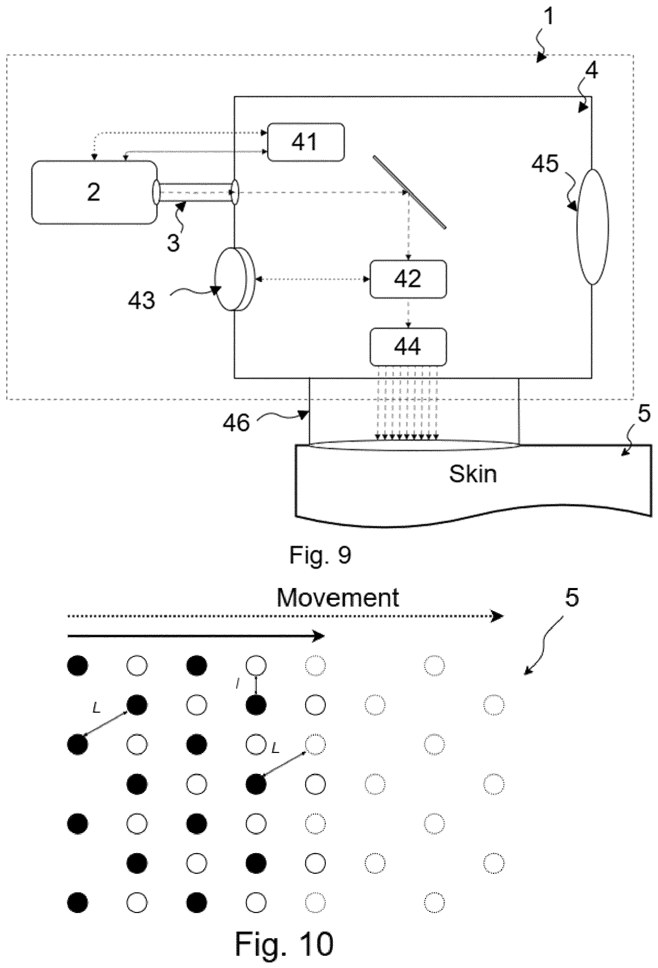

In one aspect, a fractional applicator, having an exemplary diagram of its layout shown in Fig. 9, may comprise a fractional element 44 that is configured to deliver a patterned array of laser micro beams to the treatment site, thereby achieving fractional treatment. In the fractional treatment, MTZs (micro treatment zones or micro thermal zones) are created in places, where the micro laser beams interact with the tissue (e.g. skin). The result of this treatment may be that a part of the target zone in the treatment area, constituting 1% to 99 % or 10 % to 90 % or 20 % to 70 % or 1% to 25 % of the target zone is treated and the rest of the target zone is left untreated. Thus, also 1 % to 99 % or 10 % to 90 % or 20 % to 70 % or 1 % to 25 % of the treatment area may be treated. The fractional element may comprise an array of micro-lenses, diffractive optical elements, and a matrix of micromirrors, a mask, a scanning system or other ways of achieving a fractional arrangement of the delivered energy. The fractional applicator may be configured to deliver the laser beam in a pixelated or grid-like or spot like pattern, creating MTZs while leaving surrounding tissue intact. This may be achieved through the fractional element 44, which splits the impacting laser beam (or laser beams, e.g. ablative or non-ablative laser beams) into a plurality of micro beams or columns. These micro beams may penetrate the skin, triggering collagen remodeling and skin regeneration, while the untreated areas may promote faster healing. The fractional approach minimizes downtime and reduces side effects compared to traditional full-field laser treatments, making it effective for skin resurfacing, scar reduction, and pigmentation correction.

In one aspect the fractional element 44 may comprise a micro-lens element 441 to divide the primary beam into multiple discrete micro beams.

In one aspect, the micro-lens element 441 may comprise an optical element that comprises a top side and a bottom side. In another aspect, the micro-lens element 441 may comprise a first and second optical elements each with a significant side coupled to each other, thus producing a top side and a bottom side.

In one aspect, the top side may comprise multiple cylindrical lenses next to each other and the bottom side may comprise multiple cylindrical lenses next to each other. The lenses on the top side of the element may be rotated 90 degrees compared to the lenses on the bottom side of the element to ensure separate MTZs are created after a laser beam passes through the micro-lens element 441. A cylindrical lens is a lens that has a long side and a short side. In one aspect, the long side may have a curvature, in another aspect, the short side may have a curvature.

In one aspect, the micro-lens element 441 and thus the fractional applicator may be configured such that the laser beam made up of multiple wavelengths produced by the main unit 2 delivered by the waveguide 3 enters the top side of the micro-lens element 441 and multiple micro beams exit at the bottom side of the micro-lens element 441. Thus, the bottom side of the micro-lens element 441 may face the tissue of the patient.

In one aspect, the number of cylindrical lenses on each side may be the same, thus creating a square fractional pattern of micro beams after the laser beams traverse the micro-lens element 441. The number of cylindrical lenses on each side of the micro-lens element 441 may be in a range of 3 to 30 or in a range of 3 to 10 or in a range of 4 to 9 or in a range of 5 to 8.

In another aspect, the number of cylindrical lenses on each side may be different, thus, creating a rectangular fractional pattern of micro beams after the laser beams traverse the micro-lens element 441. The number of cylindrical lenses on each side of the micro-lens element 441 may be in a range of 3 to 30 or in a range of 3 to 10 or in a range of 4 to 9 or in a range of 5 to 8.

In one aspect, the length of the long side of the cylindrical lenses may be in a range of 1 mm to 20 mm or in a range of 3 mm to 15 mm or in a range of 5 mm to 10 mm.

In one aspect, the length of the short side of the cylindrical lenses may be in a range of 0.1 mm to 5 mm or in a range of 0.3 mm to 3 mm or in a range of 0.5 mm to 2 mm.

In another aspect, each of the cylindrical lenses present on each side of the micro-lens element 441 may have a radius of curvature in a range of 5 mm to 30 mm or in a range of 10 mm to 25 mm or in a range of 15 mm to 20 mm.

In one aspect, the focal length of each of the cylindrical lenses may be different based on the wavelength of the laser beam used. In another aspect the focal length of each of the cylindrical lenses may be same for all the wavelengths of the laser beams used. The focal length may be in a range of 30 mm to 55 mm or in a range of 35 mm to 50 mm or in a range of 40 mm to 45 mm.

In one example, a (cylindrical) lens may have a focal length in a range of 30 mm to 55 mm or in a range of 35 mm to 50 mm or in a range of 40 mm to 45 mm for a laser beam with a wavelength 2940 nm (e.g. the ablative laser beam).

In another example, a (cylindrical) lens may have a focal length in a range of 30 mm to 55 mm or in a range of 35 mm to 50 mm or in a range of 40 mm to 45 mm for a laser beam with a wavelength 1540 nm (e.g. the non-ablative laser beam).

In another aspect, the micro-lens element 441 may comprise an array of lenses with radii in a range of 0.1 mm to 4 mm or in a range of 0.2 mm to 3 mm or in a range of 0.5 mm to 2 mm.

In one aspect, the number of micro-lenses in the array may be in a range of 9 to 100 individual lenses or in a range of 20 to 90 individual lenses or in a range of 30 to 80 individual lenses arranged in a regular grid. In another aspect, the number of micro-lenses in the array may be in a range of 10 to 1000. The number of micro-lenses in the array may correspond to the number of micro beams created by the micro-lenses.

In another aspect the micro-lenses may be arranged in a matrix of size in a range of 3 x 3 to 10 x 10 or in a range of 4 x 4 to 9 x 9 or in a range of 5 x 5 to 8 x 8. In another aspect, the micro-lenses may be arranged in a matrix (array) of size in a range of 3 x 3 to 30 x 30 or in a matrix of size 8 x 8.

The micro-lens element 441 material may have an index of refraction in a range of 1 to 2 or in a range of 1.2 to 1.7 or in a range of 1.4 to 1.5, ensuring precise focusing and uniform energy distribution across the target zone in the treatment area.

In one aspect, the micro-lens element 441 may have a square shape with a depth, the length of the side of the square may be in a range of 1 mm to 1000 mm or in a range of 3 mm to 500 mm or in a range of 5 mm to 100 mm.

In another aspect, the fractional applicator may comprise diffractive optical elements (DOEs). These elements may have groove densities in a range of 100 to 3000 lines per millimeter or in a range of 500 to 2500 lines per millimeter or in a range of 1000 to 2000 lines per millimeter, which diffract the incoming laser beam into a well-defined micro beam matrix. The diffraction efficiency may be in a range of 40% and 99% or in a range of 60% to 97% or in a range of 70% to 95% to balance the energy delivered to the treated and untreated areas, optimizing both therapeutic efficacy and safety. However, the DOE approach does not allow for multiple wavelengths to be fractionalized using a single fractional element.

In yet another aspect, the fractional applicator may comprise a micromirror element, which may comprise an array of reflecting elements 442. The single combined laser beam may irradiate the array of reflecting elements to form a plurality of spatially separated micro beams.

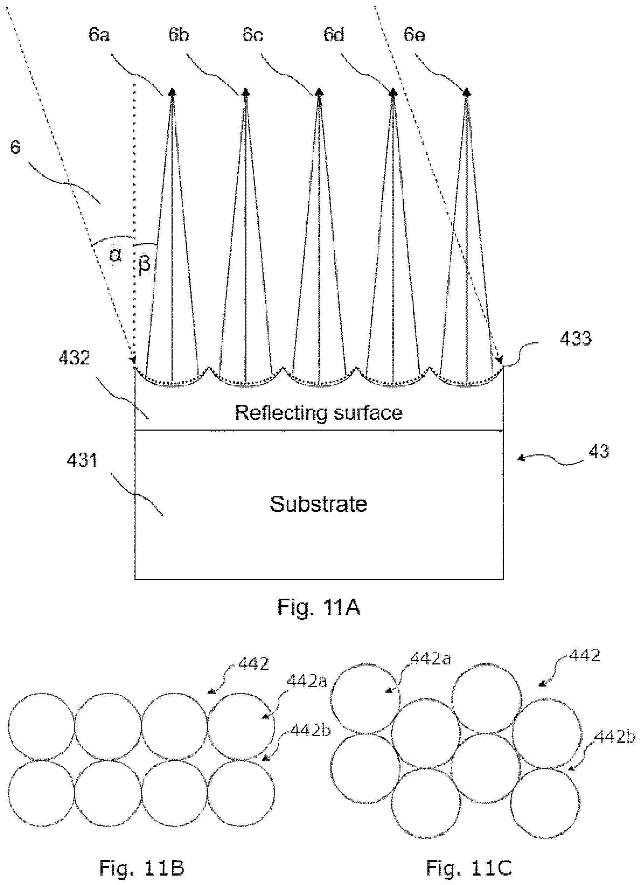

The MTZs on the patient tissue may be created due to the array of reflecting elements 442 which may divide an impacting laser beam into a plurality of micro beams 6a-6e as shown in Fig. 11A, wherein the number of micro beams corresponds to the number of non-planar reflecting elements irradiated by the laser beam. The array of reflecting elements may be formed by several methods. One possible method is shown in Fig. 11A, where on the substrate 443 a thin reflecting surface 444 may be deposited. The substrate may comprise glass, tungsten carbide (WC), aluminum, iron alloy, copper, ceramics, resin, tin, gold or any other material known in the art suitable for a thin layer deposition (e.g. sufficiently stable) and may have a thickness of 1 µm to 200 mm or 2 µm to 150 mm or 5 µm to 130 mm or 10 µm to 100 mm. The suitable material used for deposition of a thin reflecting layer 444 may comprise TiN, Au, Ag, Al, Cu, Cr or any other material known in the art which has high reflectivity in the selected wavelength range of used laser beams. The thickness of the reflecting layer may be in the range of 0.1 nm to 1000 µm or in the range of 0.5 nm to 500 µm or in the range of 0.1 nm to 250 µm or in the range of 1 nm to 100 µm. Alternatively, the substrate 443 and the thin reflecting layer 444 may be one layer of one of the above-mentioned materials, e.g. aluminum. Then the array of non-planar reflecting elements may be created in reflecting layer 444 by optical or mechanical machining. Alternatively, the array of non-planar elements may be created by optical or mechanical machining of a non-planar shape into the substrate 443 and then the thin reflecting layer 444 may be deposited. After that, the protective (also called coating) layer 445 may be deposited on the created array of reflecting elements to cover its surface, because in comparison to reflecting materials in layer 444, the coating layer is more scratch-resistant and may prevent corrosion. The coating layer 445 is depicted in Fig. 11A as dotted arcs and it may have a thickness in the range of 0.01 to 1000 nm or in the range of 0.1 to 500 nm or in the range of 0.5 to 250 nm or in the range of 1 to 100 nm. The suitable materials for a protective layer may include, but are not limited to carbides and nitrides of transition metals (e.g., titanium, zirconium) and of non-metal (e.g. silicon). Furthermore, oxides such as silica, alumina, titania, and zirconia, may also be applied for protective coatings due to their transparency in the field of used laser beam wavelengths. Alternative configurations of the thickness of the substrate or the thickness of the reflecting layer are described in U.S. Provisional Application No. 62/926,371, filed on October 25, 2019, incorporated herein by reference.

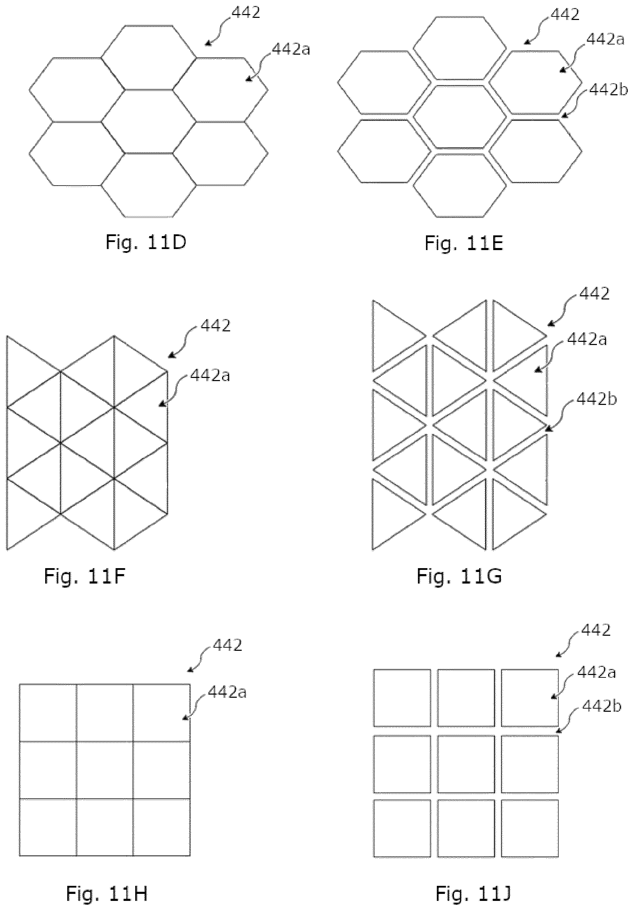

Alternatively, the array of reflecting elements 442 may be created by positioning individual non-planar reflecting elements close together, where one non-planar element may be created on the substrate layer 443, thin reflecting layer 444 or the coating layer 445. By positioning individual reflecting elements together (e.g. by gluing, melting, welding or by mechanical connection), an array of reflecting elements 442 may be created. When the laser beam irradiates the array of reflecting elements 442, it may be reflected due to the reflecting surface 444 and due to the non-planar shape of the reflecting elements it may be focused as a plurality of micro beams, represented in Fig. 11A as 6a-6e. The laser beam 6 may form an angle of incidence a (the angle between a beam incident on a surface of the array of reflecting elements and the line perpendicular to the surface of the array of reflecting elements at the point of incidence) on the array of reflecting elements 442 under the angle of 0° to 89.9° or 0.01° to 89° or 0.05° to 88° or 0.1° to 87° or 0.5° to 86° or 1° to 85°. The angle of reflection β (the angle between reflected narrow beams on a surface of the array of reflecting elements and the line perpendicular to the surface of the array of reflecting elements at the point of reflection) may be in the range of 0° to 89.9° or 0.01° to 89° or 0.05° to 88° or 0.1° to 87° or 0.5° to 86° or 1° to 85°. An arrangement of the non-planar reflecting layer may have an annular, semicircular, elliptical, oblong, square, rectangular, trapezoidal or polygonal shape and may correspond to the shape of the created MTZs on the tissue of the patient. Top views of some exemplary arrangements of reflecting element arrays 442 are depicted in Figs. 11B-J. The reflecting non-planar elements 442a may be created adjacent to each other leaving no space between the neighboring elements as shown in Figs. 11D, 11F and 11H or may have a non-reflecting space 442b between them as shown in Figs. 11B, 11C, 11E, 11G and 11J. The shortest distance between the two nearest neighboring reflecting elements may be in the range of 0 to 100 mm or in the range of 0 to 50 mm or in the range of 0 to 20 mm or in the range of 0 to 10 mm, wherein 0 mm means that two neighboring elements may have no space between them. The array of reflecting elements 442 may contain 2 to 30 000 reflecting elements or 2 to 10 000 reflecting elements or 2 to 5 000 reflecting elements or 2 to 3 000 reflecting elements. Alternatively, the array of reflecting elements 442 may contain 2 to 500 reflecting elements or 2 to 400 reflecting elements or 2 to 300 reflecting elements or 2 to 200 reflecting elements. The array of reflecting elements 442 may have a diameter of 0.02 to 2000 mm or 0.1 to 1000 mm or 0.15 to 700 mm or 0.2 mm to 550 mm. Alternatively, the length or width of the array of reflecting elements 442 may be in the range of 0.02 to 2000 mm or in the range of 0.1 to 1000 mm or in the range of 0.15 to 700 mm or in the range of 0.2 mm to 550 mm.

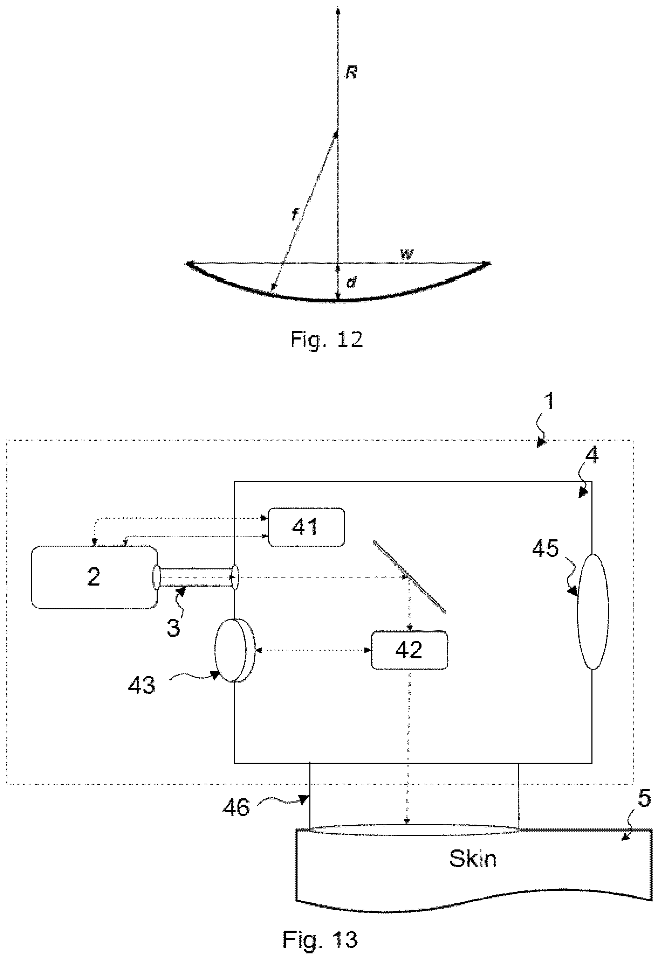

Attributes of one possible shape of the reflecting elements 444 used in the array of reflecting elements 442 are shown in Fig. 12. The reflecting element may be curved with a radius R in the range of 1 to 2000 mm or in the range of 5 to 1500 mm or in the range of 10 to 1000 mm or in the range of 20 to 500 mm. A width w of the curved reflecting element may be in the range of 0.01 to 100 mm or in the range of 0.05 to 50 mm or in the range of 0.08 to 20 mm or in the range of 0.1 to 10 mm and a depth of the reflecting element d may be in the range of 0.1 to 20 µm or in the range of 0.3 to 15 µm or in the range of 0.5 to 10 µm or in the range of 1 to 5 µm with the surface roughness of the surface which may be 0 µm to 1 µm or 0 µm to 0.5 µm or 0 µm to 0.2 µm or 0 µm to 0.1 µm. A focal length f of the reflecting element may be in the range of 1 to 2000 mm or in the range of 5 to 1500 mm or in the range of 10 to 1000 mm or in the range of 20 to 500 mm, wherein a width or length of the focal points in the focus f may be in the range of 1 to 1500 µm or in the range of 10 to 1000 µm or in the range of 100 to 700 µm with an area of focus points which may be in the range of 0.5 µm² to 5 mm² or in the range of 15 µm² to 3 mm² or in the range of 75 µm² to 2 mm² or in the range of 300 µm² to 1 mm² and a depth of focus of 0.1 to 200 mm or 0.3 to 180 mm or 0.5 to 150 mm or 1 to 120 mm. The absorption of the plurality of beams may be in the range of 0.01 mm to 20 mm under the skin surface or in the range of 0.02 mm to 15 mm or in the range of 0.03 to 10 mm under the surface of the skin.

The applicator 4 may also contain a movement sensor 446 which may track the movement of the applicator 4 in order to time the pulses to be properly spaced apart along the surface of the tissue in case of a movement of the applicator 4. One possible scenario is depicted in Fig. 10. The applicator creates the plurality of MTZs on the tissue which are represented by the full black circles. Then it is possible to move the applicator 4 in the preferred direction of movement which is depicted by the dashed bold arrow in Fig. 10. When the applicator 4 starts to move, the movement sensor may send a signal to the control unit 21 which may prevent the laser source 23 from providing the laser beam to the applicator 4, e.g. turn off the laser source 23 or close a shutter. When the sensor travels a predetermined distance (for example to the end of the dashed bold arrow in Fig. 10), a signal is sent to control unit 21 which allows the laser source 23 to provide another laser beam to the applicator 4 through the waveguide 3 and thus the second array of MTZs (dashed circles) may be created on the tissue with the same length L of untreated tissue between the two nearest spots. An alternative possible scenario is represented in Fig. 10. In this case the apparatus 1 is again set to create a plurality of MTZs and then when the applicator 4 partially moves (represented as solid arrow in Fig. 10) the apparatus 1 may create another plurality of MTZs (full white circles) on the tissue to concentrate the MTZs on the tissue, leaving the distance I of untreated portion between two MTZs shorter than the previous distance L between the original MTZs (I < L). It is also possible to set the apparatus 1 to combine both mentioned scenarios and thus it is possible to perform longitudinal movement of the applicator 4 with a higher concentration of the MTZs.

Furthermore, the device 1 may include a scanning unit that may contain a scanning control unit, movement assembly, scanning optics or sensor. The scanning unit may provide movement of the laser beam or the plurality of micro beams by changing one or more characteristics of the laser beam or the plurality micro beams e.g., the direction or intensity. The scanning unit may be controlled manually or automatically by the scanning control unit, by the control unit 21 or by the user interface 22. The scanning unit may be a part of the applicator 4 or may be connected externally to the applicator 4, waveguide 3 or the main case 2.

The scanning unit may move or tilt the laser beam with respect to the array of reflecting elements 442, thus changing the final direction of reflected micro beams and hence scanning the fractional pattern on the tissue.

Alternatively, the array of reflecting elements 442 may be a part of the scanning unit. In this configuration, the scanning unit may change the position of the array of reflecting elements 442 by rotational or translational motion, which may lead to scanning of the fractional pattern on the tissue of the patient without movement of the applicator 4.

The scanning unit may further comprise actuating components and mechanical structures, disposed at a different level from the array of reflecting elements 442 and configured to make the array of reflecting elements 442 have multiple degrees of freedom (DOFs) of motion. Alternatively, each reflecting element may be controlled by the scanning unit separately and may move or rotate together or independently of other reflecting elements.

The scanning optics may include one or more collimators, light deflecting elements (e.g. arrays of reflecting elements), focusing/defocusing elements (e.g. lenses) or filters to eliminate certain wavelengths of light (e.g. band pass filter).

In yet another aspect, the fractional applicator may comprise a scanning system employing mirrors mounted on galvanometers. In this aspect, a single combined laser beam is rapidly scanned over the target area by galvo mirrors with angular deflection ranges in a range of 0.01° to 30° or in a range of 2° to 25° or in a range of 5° to 20° and scanning frequencies in a range of 10 Hz to 500 Hz or in a range of 30 Hz to 350 Hz or in a range of 50 Hz to 200 Hz. This dynamic scanning technique may produce a fractional treatment pattern that is both adjustable and precisely controllable, ensuring consistent energy delivery while minimizing thermal buildup in adjacent tissues. However, this arrangement takes a longer time to treat the same area compared to the other arrangements mentioned above.

In yet another aspect, other ways of achieving a fractional arrangement may be used, such as mechanical masking and others.

Each of these aspects provides unique advantages, allowing the applicator 4 to be tailored to specific clinical needs. Whether using fixed micro-lens arrays for consistent MTZ patterns or dynamically adjustable micromirror or galvanometer systems for versatile treatment applications, the fractional treatment applicator enhances the precision and efficacy of laser-based tissue (e.g. skin) therapies. The fractional treatment applicator also helps with recovery, since not all of the irradiated tissue is left damaged.

Nevertheless, micro-lenses offer several advantages over other fractional laser beam-splitting methods, such as masks, diffractive optics, or scanning systems.

The micro-lens array has higher energy efficiency. Unlike diffractive optics or masks, which can cause significant energy loss due to diffraction or absorption, micro-lens arrays efficiently focus and redistribute laser energy with minimal loss. The micro-lens array creates highly uniform and well-defined micro beams, whereas masks or scanning systems may introduce irregularities due to mechanical limitations or beam distortions. The micro-lens array has no moving parts unlike scanning systems that rely on moving mirrors or galvanometers. The micro-lenses are static optical elements, making them more durable and reducing the risk of mechanical failure. The micro-lens array also has faster treatment speed - because micro-lenses simultaneously split the beam into multiple focal points, they can treat an entire area in a single (one) pulse, whereas scanning systems need to move point by point, increasing treatment time.

Additionally, a fractional applicator 4 may comprise other optical elements such as lenses (e.g. spherical or non-spherical lenses), apertures and other commonly used optical elements to manipulate the laser beam or micro beams such that they have the required shape and alignment necessary for effective treatment.

In one aspect, the fractional applicator 4 may comprise an aperture to change the shape of the laser beam entering the applicator. The aperture may have the shape of a square, triangle, rectangle, circle, ellipse, trapezoid, polygon (e.g. pentagon, hexagon, heptagon ...), star, semicircle, arbitrary shape or other shapes. The resulting fractional arrangement on the tissue of the patient may represent the shape of the aperture. The aperture reshapes the laser beam by selectively blocking part of the beam, thus the energy of the laser beam may be reduced in transiting the aperture. Based on the amount of energy that passes through the aperture, transmissivity may be associated with the aperture. Since the beam propagation ratio changes with any change of treatment parameters for each laser beam in the plurality of laser beams, due to the dependence of beam divergence on the beam propagation ratio (M²) of the laser source, the divergence angle of the laser beam may vary depending on the treatment parameters. Consequently, the radius of the laser beam at the location of the aperture may differ from its radius e.g. at the laser output. This variation in beam size at the aperture affects the effective transmissivity, i.e., the portion of the beam that passes through the aperture, thereby modulating the energy delivery in accordance with the selected laser parameters. Thus, the transmissivity through the aperture may change based on the treatment parameters of the laser sources.

In one aspect, a feedback system may be included such that the control unit 21 adjusts the treatment parameters for each laser source based on the transmissivity of the aperture for the given laser source, so that the resulting parameters of the laser beam interacting with the patient tissue are in accordance with the parameters set by the operator on the user interface 22.

In another aspect, the apparatus 1 may be pre-calibrated, such that the control unit adjusts the treatment parameters for each laser source without needing a feedback system. Thus, the resulting parameters of the laser beam interacting with the patient tissue are in accordance with the parameters set by the operator on the user interface 22. This approach is suitable for aspects where the number of combinations of the different treatment parameters for all of the lasers is finite (e.g. there are no continuous treatment parameters).

In one non-limiting example, when the operator sets a parameter related to an energy of the laser on the user interface, the laser could produce a laser beam having a circular cross section of a given energy. If a circular laser beam impacts on a square aperture it may lose e.g. 2 to 15 % of its energy as the aperture cuts off the sides of the circular laser beam and creates a square beam cross section. The system may automatically compensate for these losses and produce a laser beam that is 2 to 15 % stronger than set by the operator, thus the energy irradiating a tissue of the patient is the same as the energy set by the operator on the user interface.

In one aspect, the width of the MTZs on the tissue (e.g. skin) produced by the fractional arrangement may be adjusted.

In one aspect, where the MTZs may have the shape of a circle, the width of the MTZs may mean their diameter. In another aspect, wherein the MTZs may have the shape of a square, the width of the MTZs may mean the length of one side of the square. In yet another aspect, wherein the MTZs may have an arbitrary shape, the width of the MTZs may mean the characteristic parameter of the given shape (e.g. the side of a triangle, the length of the major axis of an ellipse, etc.).

Adjustment of the MTZ width (diameter) on the tissue (e.g. skin) using a fractional applicator may be achieved using either a continuous or a discrete mechanism. A continuous adjustment system allows the operator to finely vary the MTZ diameter in real time, providing smooth, uninterrupted control over the target zone in the treatment area. In contrast, a discrete adjustment system enables the operator to quickly switch between a series of predefined MTZ widths, thereby ensuring repeatable and consistent treatment settings. The MTZ widths may be changed using a controller 43. The controller 43 may comprise a button, a wheel, a keypad, a touchpad, a slider or other ways of changing a variable continuously or discretely. The controller 43 may be connected to a width changing element 42 inside the applicator 4. The width changing element 42 may comprise different optical elements to achieve the desired effect of changing the width of the laser beam traversing the width changing element 42.

In one aspect, the width changing element 42 may comprise a focusing lens (e.g. a spherical focusing lens) coupled to a moving mechanism (e.g. sliding mechanism) configured to move the focusing lens inside the applicator based on the controller 43. Thus, when the focusing lens is moved closer to the fractional element 44 the focusing lens bends the micro beams to a focal point and thus the MTZs have a first MTZ width on the tissue (e.g. skin) of the patient. When the focusing lens is moved to a second position, different from the first position, the position of the focal point relative to the tissue is changed and thus the MTZs have a second MTZ width on the tissue of the patient, wherein the second MTZ width is different from the first MTZ width.

In another aspect, the width changing element 42 may comprise a moving mechanism (e.g. sliding mechanism) configured to move the fractional element 44 inside the applicator based on the controller 43. For example, the array of micro-lenses may be moved during the adjustment of the

MTZ width. When the array of micro-lenses is moved to a first position inside the applicator 4, the micro-lenses focus the beams to a first focal point and thus the MTZs have a first MTZ width on the tissue (e.g. skin) of the patient. When the array of micro-lenses is moved to a second position inside the applicator 4, the micro-lenses focus the beams to a second focal point and thus the MTZs have a second MTZ width on the tissue (e.g. skin) of the patient, wherein the second MTZ width is different from the first MTZ width.

In yet another aspect, other mechanisms may be used to adjust the MTZ width. Thus the width changing element 42 may comprise other optical or non-optical elements configured to change the MTZ width.

The width changing element 42 may further comprise a magnetic sensor configured to obtain information about a position of the controller 43 that corresponds to the MTZ width of the micro laser beams on the tissue (e.g. skin) of the patient and provide this information to the control unit 21. The control unit 21 may further provide this information to the user interface 22 which may show the width of the MTZs to the operator.

In one aspect the width changing element 42 may be placed before the fractional element 44 on the optical path of the beam. In this arrangement, the focal point or width of the beam is changed before it is turned into a plurality of micro beams. In another aspect the width changing element 42 may be placed after the fractional element 44 on the optical path of the beam. In this arrangement, the width of the beams is adjusted after the main beam has been turned into a plurality of micro beams. In yet another aspect, the width changing element 42 may comprise the fractional element 44 and a moving mechanism.

In one aspect, the fractional treatment parameters can be tailored to meet a range of clinical needs. For example, the MTZ width (diameter) may be selected to be in a range of 100 µm to 1500 µm or in a range of 150 µm to 1400 µm or in a range of 200 µm to 1300 µm. Alternatively, the MTZ width may be selected in a range of 200 µm to 250 µm or in a range of 1100 µm to 1300 µm allowing for treatment customization based on the extent of the skin condition. The distance between individual MTZs may be adjusted to be in a range of 500 µm to 2000 µm or in a range of 750 µm to 1500 µm or in a range of 1000 µm to 1200 µm, which helps optimize the balance between treatment efficacy and tissue recovery. In one aspect, the distance between individual MTZs may mean the closest distance between the borders of the neighboring MTZs. In another aspect, the distance between individual MTZs may mean the distance between centers of neighboring MTZs.

In one aspect, the width-changing element 42, under the control of the controller 43, may adjust the width of the MTZ between two positions: a first position (e.g. minimum MTZ width) and a second position (e.g., maximum MTZ width). For the first position (e.g., minimum MTZ width), the MTZ width may be in a range of 100 µm to 400 µm or in a range of 150 µm to 350 µm or in a range of 165 µm to 295 µm or in a range of 180 µm to 270 µm. For the second position (e.g. a maximum MTZ width), the MTZ width may be in a range of 750 µm to 1650 µm or in a range of 900 µm to 1500 µm or in a range of 1000 µm to 1400 µm or in a range of 1050 µm to 1350 µm.

In another aspect, the width adjusting element 42 may be configured to change the MTZ width between a minimum MTZ width (e.g. first position) and a maximum MTZ width (e.g. second position) such that the maximum MTZ width is bigger than the minimum MTZ width in a range of 1.875 to 16.5 times or in a range of 3.8 to 7.5 times.

In one aspect, when delivering a plurality of laser beams in a spatially coordinated manner through the fractional applicator, the centers of each MTZ for each of the laser beams may be aligned. In this aspect, the MTZ width or diameter for each of the plurality of laser beams impacting the patient tissue may be different.

In one aspect, the widths or diameters of the MTZs produced by different laser beams may be in a range of 50 % to 100 % or in a range of 60 % to 100 % or in a range of 70 % to 100 % of each other.

In another aspect, the widths or diameters of the MTZs produced by different laser beams may be in a ratio ranging from 1:2 to 1:1, or from 3:5 to 1:1, or from 7:10 to 1:1 relative to each other.

In another aspect, the widths or diameters of the MTZs may be different by a value of in a range of 1 µm to 1000 µm or in a range of 5 µm to 600 µm or in a range of 10 µm to 300 µm or in a range of 15 µm to 200 µm or in a range of 20 µm to 80 µm.

In another aspect, when delivering the plurality of laser beams in a spatially coordinated manner through the fractional applicator, the centers as well as the widths or diameters of the MTZs may be the same.

Additional parameters that may be controlled include the number of MTZs and the fluence delivered per MTZ. The number of MTZs in a fractional pattern can be configured to be in a range of 10 to 200 or in a range of 20 to 150 or in a range of 30 to 100 or in a range of 10 to 1000 or in a range of 9 to 100 or in a range of 20 to 90 or in a range of 30 to 80, thus accommodating various treatment densities. In one aspect the MTZs may be arranged in a matrix of spots in the range of 3 x 3 to 10 x 10 or in the range of 4 x 4 to 9 x 9 or in the range of 5 x 5 to 8 x 8. In another aspect, the MTZs may be arranged in a matrix of spots in the range of 3 x 3 to 30 x 30 or in a matrix of 8 x 8.

In one aspect, when using the fractional applicator, the fluence delivered to each MTZ by any (an individual one) of the plurality of laser sources in one laser beam pulse may be in a range of 0.1 J/cm² to 650 J/cm² or in a range of 0.2 J/cm² to 500 J/cm² or in a range of 0.3 J/cm² to 400 J/cm² or in a range of 0.1 J/cm² to 100 J/cm².

In one example, the fluence delivered to each MTZ when using the fractional applicator by the ablative laser beam in one pulse may be in a range of 0.1 J/cm² to 650 J/cm² or in a range of 0.5 J/cm² to 300 J/cm² or in a range of 1 J/cm² to 70 J/cm² or in a range of 3 J/cm² to 38 J/cm² or in a range of 4 J/cm² to 33 J/cm² or in a range of 5 J/cm² to 30 J/cm² or in a range of 0.1 J/cm² to 3 J/cm² or in a range of 0.2 J/cm² to 1.5 J/cm² or in a range of 0.3 J/cm² to 1.2 J/cm².

In one example, the fluence delivered to each MTZ when using the fractional applicator by the non-ablative laser beam in one pulse may be in a range of 0.1 J/cm² to 650 J/cm² or in a range of 0.5 J/cm² to 300 J/cm² or in a range of 1 J/cm² to 100 J/cm² or in a range of 15 J/cm² to 70 J/cm² or in a range of 18 J/cm² to 65 J/cm² or in a range of 20 J/cm² to 60 J/cm² or in a range of 1 J/cm² to 3 J/cm² or in a range of 1.5 J/cm² to 2 J/cm² or in a range of 1.3 J/cm² to 1.8 J/cm².

In another aspect, when using the fractional applicator, the energy delivered to each MTZ by any (an individual one) of the plurality of laser sources in one laser beam pulse may be in a range of 1 mJ to 50 mJ or in a range of 2 mJ to 45 mJ or in a range of 3 mJ to 35 mJ. Thus, the energy delivered to the entire treatment zone (i.e. all of the MTZs together) by any of the plurality of laser sources in one laser beam pulse when using the fractional applicator may be in a range of 0.01 J to 10 J or in a range of 0.1 J to 9 J or in a range of 0.2 J to 7 J. Furthermore, the maximum energy of one laser beam pulse of any of the plurality of laser sources may be in a range of 1.2 J to 2.3 J.

In one example the energy delivered to each MTZ by the ablative laser beam in one pulse when using the fractional applicator may be in a range of 1 mJ to 35 mJ or in a range of 2 mJ to 30 mJ or in a range of 3 mJ to 25 mJ. Thus, the energy delivered to the entire treatment zone when using the fractional applicator by the ablative laser beam in one laser beam pulse may be in a range of 0.01 J to 7 J or in a range of 0.1 J to 6 J or in a range of 0.2 J to 5 J. Furthermore, the maximum energy of one pulse of ablative laser source may be in a range of 1.2 J to 1.6 J.

In another example, the energy delivered to each MTZ when using the fractional applicator by the non-ablative laser beam in one pulse may be in a range of 1 mJ to 50 mJ or in a range of 2 mJ to 40 mJ or in a range of 3 mJ to 35 mJ. Thus, the energy delivered to the entire treatment zone when using the fractional applicator by the non-ablative laser beam in one laser beam pulse may be in a range of 0.01 J to 10 J or in a range of 0.2 J to 8 J or in a range of 0.3 J to 7 J. Furthermore, the maximum energy may be in a range of 1.9 J to 2.3 J.

Additionally, as mentioned above, the plurality of laser sources may produce respective laser beam pulse stacks, each stack of a given wavelength. The number of pulses of the given laser source in the respective laser beam pulse stack may be in a range of 1 to 100 or in a range of 1 to 50 or in a range of 1 to 10 or 1 to 5. Thus, the energy and fluence delivered by the given laser source to the target zone in the treatment area may be 1 to 100 times or 1 to 50 times or 1 to 10 or 1 to 5 times higher, when considering the entire laser beam pulse stack.

Furthermore, laser beam pulse stacks produced by the plurality of laser sources may be applied in a simultaneous, successive or overlapping method. For example, when using the simultaneous method, the energy and fluence delivered to the treatment are by the plurality of laser sources may be the sum of the energies and fluences delivered to the target zone in the treatment area by all of the individual laser sources.

In one aspect, the area (e.g. of the target zone) irradiated during a single trigger (i.e. at least one laser beam pulse stack fired from at least one laser source) of the apparatus 1 using the fractional applicator 4 may be in a range of 10 mm² to 1000 mm² or in a range of 25 mm² to 500 mm² or in a range of 50 mm² to 100 mm². Additionally, the area irradiated during a single trigger of the apparatus 1 (area of the target zone) using the fractional applicator 4 may be in a range of 1 mm² to 25 cm².

In one aspect, the applicator may also comprise a mask to change the pattern of the created MTZs or to limit the number of MTZs created to change the size of the target zone in the treatment area. The mask may comprise a physical mask or a different type of mask. For example, an interchangeable mask may be attached to the end of the applicator proximal to the tissue of the patient (e.g. skin). In another aspect, the applicator may comprise a shutter configured to change the pattern of the created MTZs or to limit the number of MTZs.

In another aspect, a full field applicator is configured to deliver a continuous, uniform laser beam across an extended target zone in the treatment area to create a continuous treatment spot. An exemplary diagram of the layout of a fractional applicator is shown in Fig. 13.

Similar to the fractional treatment approach, the full field applicator may incorporate mechanisms for adjusting the treatment spot width (diameter) of the delivered beam. Adjustment of the treatment spot width on the tissue (e.g. skin) using the full field applicator may be achieved using either a continuous or a discrete mechanism. A continuous adjustment system allows the operator to finely vary the treatment spot width in real time, providing smooth, uninterrupted control over the target zone in the treatment area. In contrast, a discrete adjustment system enables the operator to quickly switch between a series of predefined treatment spot widths, thereby ensuring repeatable and consistent treatment settings. The treatment spot width may be changed using a controller 43. The controller 43 may comprise a button, a wheel, a keypad, a touchpad, a slider or other ways of changing a variable continuously or discreetly. The controller 43 may be connected to a width changing element 42 inside the applicator 4. The width changing element 42 may comprise different optical elements to achieve the desired effect of changing the width of the laser beam traversing the width changing element 42.

In one aspect, the width changing element 42 may comprise a focusing lens (e.g. a spherical focusing lens) coupled to a moving mechanism (e.g. sliding mechanism) configured to move the focusing lens inside the applicator based on the controller 43. Thus, when the focusing lens is moved to a first position, the focusing lens bends the laser beam (or beams) to a focal point and thus the treatment spot has a first spot width on the tissue of the patient. When the focusing lens is moved to a second position, different from the first position, the position of the focal point relative to the tissue is changed and thus the treatment spot has a second spot width on the tissue of the patient, wherein the second spot width is different from the first spot width.

In another aspect, other mechanisms may be used to adjust the treatment spot width. Thus, the width changing element 42 may comprise other optical or non-optical elements configured to change the treatment spot width.

In one aspect, the minimum treatment spot width on the tissue of a patient (e.g. skin) may be in a range of 0.1 mm to 2 mm or in a range of 0.5 mm to 1.5 mm or in a range of 0.7 mm to 1.3 mm and the maximum treatment spot width on the tissue of a patient may be in a range of 7.5 mm to 12.5 mm or in a range of 8.5 mm to 11.5 mm or in a range of 9 mm to 11 mm or in a range of 9.5 mm to 10.5 mm.

In another aspect, the width changing element 42 may be configured to change the treatment spot width between a minimum spot width and a maximum spot width such that the maximum spot width is bigger than the minimum spot width in a range of 3.75 to 125 times or in a range of 7.3 to 15 times.

In one aspect, when delivering a plurality of laser beams in a spatially coordinated manner through a full field applicator, their centers may be aligned. In this aspect, the treatment spot width (diameter) of one or more of the plurality of laser beams impacting the patient tissue may be different than the others.

In one aspect, the widths (diameters) of the treatment spots produced by different laser beams may be in a range of 50 % to 100 % or in a range of 60 % to 100 % or in a range of 70 % to 100 % of each other.

In another aspect, the widths (diameters) of the treatment spots produced by different laser beams may be in a ratio ranging from 1:2 to 1:1, or from 3:5 to 1:1, or from 7:10 to 1:1 relative to each other.

In another aspect, the widths (diameters) of the treatment spots may be different by a value of in a range of 0.03 mm to 7 mm or in a range of 0.2 mm to 5 mm or in a range of 0.25 mm to 4 mm.

In another aspect, when delivering the plurality of laser beams in a spatially coordinated manner through the full field applicator, their centers as well as their widths (diameters) may be the same.

The width changing element 42 may further comprise a magnetic sensor configured to obtain information about a position of the controller 43 that corresponds to the treatment spot width on the tissue of the patient (e.g. skin) and provide this information to the control unit 21.

Additionally, the full field applicator 4 may comprise other optical elements such as lenses (e.g. spherical or non-spherical lenses), apertures and other commonly used optical elements to manipulate the laser beam such that they have the required shape and alignment necessary for effective treatment.

In one aspect, the fluence delivered to the treatment spot when using the full field applicator by any of the plurality of laser sources in a single laser beam pulse may be in a range of 0.1 J/cm² to 500 J/cm² or in a range of 0.5 J/cm² to 400 J/cm² or in a range of 1 J/cm² to 360 J/cm². Additionally, the fluence delivered to the treatment spot when using the full field applicator by any of the plurality of laser sources in a single laser beam pulse may be in a range of 1 J/cm² to 51000 J/cm² or in a range of 0.1 J/cm² to 3.6 J/cm², ensuring that the energy delivery is optimized for different tissue (e.g. skin) types and treatment goals. By integrating these adjustable features, the full field applicator provides a flexible platform that can be easily adapted to various clinical scenarios, balancing ease of use with precise control over treatment parameters.

In one example, the fluence delivered to the treatment spot when using the full field applicator by the ablative laser beam in a single pulse may be in a range of 0.1 J/cm² to 500 J/cm² or in a range of 0.5 J/cm² to 400 J/cm² or in a range of 1 J/cm² to 360 J/cm². Additionally, the fluence delivered to the treatment spot when using the full field applicator by any of the plurality of laser sources in a single laser beam pulse may be in a range of 0.1 J/cm² to 3.6 J/cm².

In another example, the fluence delivered to the treatment spot when using the full field applicator by the non-ablative laser beam in a single pulse may be in a range of 0.1 J/cm² to 500 J/cm² or in a range of 0.5 J/cm² to 400 J/cm² or in a range of 1 J/cm² to 310 J/cm². Additionally, the fluence delivered to the treatment spot when using the full field applicator by any of the plurality of laser sources in a single laser beam pulse may be in a range of 0.1 J/cm² to 3.1 J/cm².

In another aspect, the energy delivered to the treatment spot when using the full field applicator by any of the plurality of laser sources in a single laser beam pulse may be in a range of 0.1J to 5 J or in a range of 0.2 J to 4 J or in a range of 0.3 J to 3 J.

In one example, the energy delivered to the treatment spot when using the full field applicator by the ablative laser beam in a single pulse may be in a range of 0.1 J to 5 J or in a range of 0.2 J to 4 J or in a range of 0.3 J to 3 J. Additionally, the maximum energy delivered to the treatment spot when using the full field applicator by the ablative laser beam in a single pulse may be in a range of 2.7 J to 2.9 J.

In another example, the energy delivered to the treatment spot when using the full field applicator by the non-ablative laser beam in a single pulse may be in a range of 0.1 J to 5 J or in a range of 0.2 J to 4 J or in a range of 0.3 J to 3 J. Additionally, the maximum energy delivered to the treatment spot when using the full field applicator by the ablative laser beam in a single pulse may be in a range of 2.3 J to 2.5 J.

Additionally, as mentioned above, the plurality of laser sources may produce laser beam pulse stacks, each with a single wavelength. The number of pulses of the given laser source in the laser beam pulse stack may be in a range of 1 to 100 or in a range of 1 to 50 or in a range of 1 to 10. Thus, the energy and fluence delivered by the given laser source to the target zone in the treatment area may be 1 to 100 times or 1 to 50 times or 1 to 10 times higher, when considering the entire laser beam pulse stack.

Furthermore, laser beam pulse stacks produced by the plurality of laser sources may be applied in a simultaneous, successive or overlapping method. For example, when using the simultaneous method, the energy and fluence delivered to the treatment area by the plurality of laser sources may be the sum of the energies and fluences delivered to the target zone in the treatment area by all of the individual laser sources.

In one aspect, the applicator 4 may contain additional optical components for focusing or defocusing the single laser beam or plurality of narrow beams or for changing the attributes of the single laser beam or plurality of narrow beams, e.g. the width, focus, intensity, shape or spatial distribution of narrow laser beams. For deeper depth coordinates, the optical system may focus to a selected depth below the at least one depth of the treatment portions in order to achieve concentration at the desired depth coordinate in the patient's tissue (e.g. skin). In another aspect, the applicator 4 may contain a cover glass at the end proximal to the tissue 5, such that the optical components inside the applicator do not get damaged and last longer working in perfect condition.

In one aspect, a spacer 46, which may be transparent to the used wavelengths, may be coupled between the applicator 4 and the tissue (e.g. skin) 5. In one aspect, the spacer 46 may have the same function as the additional optical components as described above, so it may focus or defocus the single laser beam or plurality of narrow beams or change the attributes of the single laser beam or plurality of narrow beams, e.g. the width, focus, intensity, shape or spatial distribution. The thickness of the spacer 46 (between the applicator 4 and the tissue 5) may be in a range of 1 to 2000 mm or in a range of 5 to 1500 mm or in a range of 10 to 1000 mm or in a range of 10 to 500 mm. Because of mechanical, structural, physical or chemical properties of this spacing object, the spacing object may provide or improve attachment of any parts of the device (e.g. applicator 4) or some parts of the device and patient tissue together. Additionally, the spacer 46 may provide cooling of the tissue or any part of the applicator.

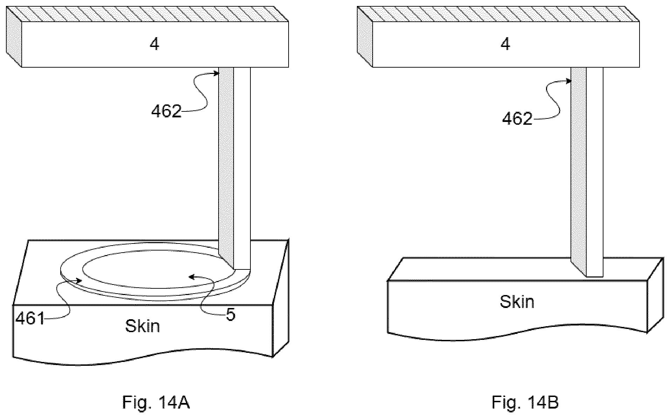

In another aspect, the spacer 46 may comprise a contact pad 461 and a spacing agent 462. The arrangement may be such that the spacing agent 462 is coupled to the end of the applicator proximal to the tissue of the patient (e.g. skin). An exemplary diagram of such an arrangement is shown in Fig. 14A. The spacing agent may have an oblong shape with a height in a range of 1 mm to 100 mm or in a range of 10 mm to 80 mm or in a range of 20 mm to 50 mm. The contact pad 461 may be coupled to the end of the spacing agent proximal to the tissue of the patient. The contact pad 461 may be a piece of material with thickness in a range of 0.1 mm to 5 mm or in a range of 0.5 mm to 3 mm or in a range of 1 mm to 2.5 mm.

In one aspect, the contact pad 461 may have the shape of a horseshoe or a section of an annulus. The section of the annulus may comprise 1% to 99% of the annulus or 10% to 80% of the annulus or 20% to 70% of the annulus. The annulus comprises two concentric circles with material in-between. The radius of the smaller circle may be in a range of 1 mm to 20 mm or in a range of 1.5 mm to 15 mm or in a range of 2 mm to 10 mm. The radius of the larger circle may be in a range of 2.0 mm to 25 mm or in a range of 2.5 mm to 17.5 mm or in a range of 3.0 mm to 12.5 mm.

In another aspect, the contact pad 461 may comprise a full annulus. The area of the empty space inside the smaller circle, where treatment may occur may be in a range of 3 mm² to 1300 mm² or in a range of 7 mm² to 710 mm² or in a range of 12 mm² to 400 mm².

In yet another aspect, the contact pad 461 may have different shapes at the inner and outer edges. The different shapes at the edges may be a square, a circle, a triangle, a polygon, an ellipse or other shapes. The area of the empty space within the inner edge may be in a range of 3 mm² to 1300 mm² or in a range of 7 mm² to 710 mm² or in a range of 12 mm² to 400 mm².

In yet another aspect, the spacer may only comprise the spacing agent 462. Thus, the contact with the patient's tissue (e.g. skin) is only through the side of the spacing agent proximal to the tissue of the patient 5. An exemplary diagram of such an arrangement is shown in Fig. 14B.

In one aspect, the full height of the spacer 46 may be in a range of 1 mm to 105 mm or in a range of 10 mm to 85 mm or in a range of 20 mm to 55 mm.

In one aspect, a spacer 46 may be coupled permanently to the applicator 4. In this arrangement, the applicator 4 may be changed to alter the size of the spacer 46. This arrangement may have the negative effect of having to swap the applicators, however the advantage it brings is that it reduces the number of moveable parts on the applicator 4. The fixed spacer 46 may comprise a spacer as described in the paragraphs above.

In another aspect, the applicator 4 may have multiple interchangeable spacers 46. The interchangeable spacers 46 may comprise spacers as described in the paragraphs above.

In one aspect, the applicator 4 may be equipped with an integrated printed circuit board (PCB) or flexible circuit that may be connected to the control unit 21 using a dedicated data connection. This integrated circuit may be designed to communicate the specific type of applicator 4 that is connected, whether it is a full field, fractional, spot, or another variant. Upon establishing the connection, the control unit 21 receives the applicator 4 identification data and promptly displays this information on the user interface 22.

Furthermore, the control unit 21 may utilize the received data to suggest optimized treatment protocols tailored to the connected applicator 4. Based on the applicator type, the control unit 21 can recommend specific parameters that are ideally suited for the intended clinical application. For example, the control unit may recommend specific laser parameters and treatment area configurations. This smart integration not only simplifies the overall setup process but also enhances treatment efficacy and safety by ensuring that the operator is guided with applicator-specific protocols throughout the procedure.

Alternatively, the control unit 21 may utilize the received data to instruct the operator on how to set up the applicator, so that the protocol selected reaches its full treatment potential.

In one aspect, the applicator may further comprise an attachment point 45 for a vacuum pump system to facilitate the continuous removal of ablated tissue debris from the treatment site. The vacuum pump attachment 45 may be positioned adjacent to the laser beam exit, ensuring that any residual ablated tissue is efficiently evacuated during the procedure. Or it may be positioned in the proximal end of the applicator to the waveguide, so that the quick connect system may ensure the connection of the vacuum pump hose as well. In this configuration, the applicator may comprise a removable part that comprises the airway. Such removable part facilitates the necessary cleaning of the vacuum pump air way.

The removal process not only enhances the visual clarity of the treatment area for the operator but also helps maintain high treatment efficiency by preventing the accumulation of debris that could scatter or absorb the laser energy, thereby ensuring consistent and effective energy delivery throughout the entire treatment session.

The vacuum pump system may comprise a vacuum pump configured to create a pressure lower than an ambient pressure or atmospheric pressure. The vacuum pump may be coupled to a vacuum pump hose, which may be coupled to the applicator.

In another aspect, the vacuum pump hose may be permanently attached to the applicator.

In one aspect, the vacuum pump hose may be integrally attached to the articulated arm or coupled alongside the fiber bundle. This secure attachment simplifies the overall design, minimizes the risk of hose entanglement, and ensures consistent alignment with the applicator, thereby enhancing ease of use and workflow during treatment.

In one aspect, the vacuum pump may be external to the main unit 2. In another aspect, the vacuum pump may be integrated into the main unit 2 to simplify the setup procedure and reduce the number of devices necessary to treat patients.

In one aspect, the applicator may further comprise an attachment point for a cooling hose of a patient cooling system to facilitate the delivery of cold air directly to the treatment area. The cooling hose attachment may be positioned adjacent to the laser beam exit, ensuring effective and continuous cooling of the patient's tissue during treatment. Additionally, the cooling hose may be a part of the quick connect system and thus simplify the cold air delivery system. In this configuration, the applicator may comprise a removable component that incorporates the airflow channel, thereby simplifying cleaning and maintenance procedures.

The direct application of cold air not only significantly enhances patient comfort during the procedure but also helps maintain optimal treatment efficiency by minimizing thermal buildup in the treated tissue, thus preventing unintended thermal damage. Additionally, the cooling system reduces potential tissue swelling or inflammation, thereby facilitating clearer visualization and allowing for more precise and controlled treatment.

In another aspect, the cooling hose may be permanently attached to the applicator.

In one aspect, the cooling hose may be integrally attached to the articulated arm or coupled alongside the fiber bundle or waveguide. Such integration simplifies overall system design, reduces the risk of hose interference or entanglement, and ensures consistent alignment with the applicator, thereby improving operator convenience and procedural workflow.

In one aspect, the patient cooling system may be external to the main unit 2. In another aspect, the patient cooling system may be integrated into the main unit 2 to simplify the setup procedure and reduce the number of devices necessary to treat patients.

In one aspect, the applicator 4 may comprise a lighting system configured to illuminate the treated patient tissue. The inclusion of a lighting system on the applicator may be beneficial, since lighting up the treated patient tissue may improve the visibility of the treatment area. Thus, it may help the operator and improve the overall treatment outcome.

In one aspect, the applicator 4 may be fixed to the waveguide 3. In one aspect, only a single applicator 4 may be used for the entirety of the therapy.

In another aspect the applicator 4 may be interchangeable. A quick release system, as described previously in this invention, may be used between the distal end of the waveguide 3 and the applicator 4. Different applicators 4 may be used during the therapy. The different applicators 4 may include a fractional treatment applicator, or a full field applicator, or other types of applicators.

Additionally, the apparatus 1 may include one or more sensors. A sensor may provide information about at least one physical quantity and its measurement may lead to feedback which may be displayed by the user interface 22. The one or more sensors may be used for sensing the energy of delivered laser energy or backscattered laser beams from the tissue (e.g. skin), impedance of the tissue, resistance of the tissue, temperature of the treated tissue, temperature of the untreated tissue, temperature of at least one layer of the tissue, water content of the apparatus, the phase angle of delivered or reflected energy, the position of the apparatus 1, the position of the applicator 4, temperature of the cooling media, temperature of the laser chambers 231a and 231b or contact with the tissue or force applied to the tissue. The sensor may be a temperature, acoustic, vibration, electric, magnetic, flow, positional, optical, imaging, pressure, force, energy flux, impedance, current, Hall or proximity sensor. The sensor may be a capacitive displacement sensor, acoustic proximity sensor, gyroscope, accelerometer, magnetometer, infrared camera or thermographic camera. The sensor may be invasive or contactless. The sensor may be located on the applicator 4 or in the main unit 2. For example, a temperature sensor may be located on the applicator 4, while the impedance sensor may be located in the main unit 2. One sensor may measure more than one physical quantity. For example, the sensor may include a combination of a gyroscope, an accelerometer or a magnetometer. Additionally, the sensor may measure one or more physical quantities of the tissue skin or untreated tissue.

The present invention significantly advances aesthetic medicine and dermatological treatment methodologies through a unique, multiple wavelength laser apparatus 1. The plurality of laser beams of different wavelengths may be used individually or in a simultaneous method or in an overlapping method or in a successive method. In one aspect, the plurality of laser beams may comprise the ablative (e.g. 2940 nm) and the non-ablative (e.g. 1540 nm) laser beams.

A non-ablative laser beam (e.g. generated by the first laser source and having a wavelength of approximately 1540 nm) may primarily induce controlled thermal coagulation within the dermal layer. Due to its selective absorption properties, the non-ablative laser beam may penetrate the skin effectively without causing surface vaporization, thereby preserving epidermal integrity while achieving significant therapeutic effects in deeper dermal layers. The controlled thermal coagulation produced by the non-ablative beam may lead to fibroblast activation, collagen and elastin synthesis, and initiation of a natural wound-healing cascade. A treatment by the non-ablative laser beam may therefore be particularly suitable for applications such as improving mild to moderate skin laxity, reducing fine lines, treating acne scars, and enhancing skin elasticity and tightness. Additionally, the controlled inflammation induced by the non-ablative beam may promote the release of beneficial cytokines and heat shock proteins, which assist in moderating inflammation, improving skin elasticity, and contributing to overall rejuvenation and restoration of youthful skin properties. The non-ablative laser beam may heat the target portion of the skin of the patient to a desirable temperature in a range of 37.5 °C to 99°C or in a range of 40°C to 95°C or in a range of 45°C to 80°C.

The ablative laser beam (e.g. generated by the second laser source and having a wavelength of approximately 2940 nm) may be specifically configured to precisely remove superficial skin layers via controlled vaporization. Due to its high absorption in water, the ablative beam efficiently ablates epidermal and superficial dermal tissues, enabling effective treatment of superficial lesions, keratoses, fine lines, wrinkles, and aged or sun damaged skin. The ablative laser beam may induce neocollagenesis through an acute wound response, leading to substantial improvements in skin texture and appearance. The ablative laser beam may be especially beneficial in procedures aimed at deep skin resurfacing, skin texture improvement, lesion removal, and advanced scar revision. The ablative laser beam may heat the target portion of the skin of the patient to a desirable temperature in a range of 99°C to 300°C or in a range of 100°C to 250°C or in a range of 101°C to 200°C to achieve ablation of the desired portion of the skin.

A notable and innovative aspect of the invention involves the simultaneous or overlapping application of the plurality of laser beams (e.g. the ablative and non-ablative laser beams), which may provide synergistic therapeutic effects that significantly enhance clinical outcomes.

In one aspect, the system may be configured to deliver an initial pulse of the non-ablative (e.g. 1540 nm) wavelength to prime the target zone in the treatment area. This pre-exposure may create distinct microthermal zones within the dermal layers, inducing targeted mild inflammation, fibroblast activation, enzymatic modulation, and/or a controlled redistribution of water content within the extracellular matrix (ECM). By preconditioning the skin in this manner, the overlapping ablative pulse (e.g. at 2940 nm) may achieve a more uniform and controlled vaporization effect. The priming effect from the non-ablative beam may also minimize undesirable localized thermal injury and promote deeper, more uniform ablation, ultimately enhancing the safety profile of the treatment by significantly reducing risks such as post-inflammatory hyperpigmentation (PIH) and prolonged erythema.

The invention may further employ a thermal buffering effect, wherein the controlled preheating of patient tissue by the non-ablative laser beam facilitates deeper, more uniform penetration and efficacy of the ablative laser beam. This synergistic application may lead to enhanced collagen remodeling and improved quality of the extracellular matrix components due to the combined thermal stimulation and controlled ablation. The dual-wavelength approach may thus significantly improve both immediate and long-term clinical outcomes, maximizing collagen synthesis, tissue regeneration, and contraction, ultimately enhancing skin rejuvenation and textural improvement.

In another aspect, more than two laser beams may be applied simultaneously or in overlap to potentially achieve an even more advanced therapeutic effect.

Additionally, the simultaneous or overlapping application of a plurality of laser beams may advantageously contribute to the selective removal of senescent cells (cells that can no longer divide, which may be created by aging or by environmental factors) within the skin. In one aspect, the thermal gradients created by overlapping or simultaneous laser beam exposure-wherein deeper layers experience controlled heating and superficial layers experience controlled ablation-may selectively target senescent cells due to their inherent reduced thermal stability compared to healthy cells. The preferential removal of senescent cells may effectively reduce chronic dermal inflammation, enhancing skin quality, appearance, and reducing signs of aging caused by environmental damage and oxidative stress.

Moreover, the overlapping or simultaneous emission of a plurality of laser beams may provide controlled thermal activation of skin regenerative pathways, including activation of dermal stem cells. Precise modulation of dermal temperatures by the plurality of beams may activate regenerative signaling pathways, promoting the proliferation and differentiation of dermal stem cells, thus enhancing skin regenerative potential, improving wound healing efficiency, and potentially counteracting cellular aging mechanisms. In another aspect, controlled thermal stress produced by the laser apparatus may activate cellular protective mechanisms, notably upregulating longevity-associated biomarkers such as klotho proteins. Additionally, controlled ablative action may physically remove superficial epidermal layers containing oxidative stress by-products, further enhancing skin's resilience and rejuvenation potential.

Additionally, the combination treatment provided by the invention may offer enhanced removal of advanced glycation end-products (AGEs) in the dermal extracellular matrix. In one aspect, pre-exposure by a non-ablative laser beam may induce controlled unfolding of dermal proteins, increasing enzymatic breakdown of AGEs. Following this step, the ablative laser beam may physically remove the glycation-damaged components of the extracellular matrix, thus reducing collagen stiffness, improving skin elasticity, and restoring youthful skin function.

In one aspect, an applicator specialized for dentistry may be used. Such an applicator may be used for treatments including, but not limited to: hard tissue ablation (cavity preparation, enamel and dentin treatment), soft tissue procedures (gingivectomy, periodontal treatments), bone surgery (osteotomy, implant site preparation), soft tissue surgeries (frenectomy, lesion removal), peri-implantitis treatment, or endodontic disinfection.

In another aspect, an applicator specialized for surgical procedures may be used. Such an applicator may be used for treatments including, but not limited to: ablation of soft tissues with minimal thermal damage, microsurgery (ENT, ophthalmology), general soft tissue surgery (neurosurgery, gynecology, urology) and treatment of tumors and dysplastic lesions.

In yet another aspect, an applicator specialized for gynecology may be used. Such an applicator may be used for treatments including, but not limited to: vaginal rejuvenation (improving elasticity and hydration), vaginal atrophy and stress, urinary incontinence treatment, or cervical lesion removal (HPV-related conditions).

In yet another aspect, an applicator specialized for ophthalmology may be used. Such an applicator may be used for treatments including, but not limited to: eyelid surgery (blepharoplasty) or pterygium removal.

Additionally, the apparatus 1 may be used for other treatments, including but not limited to treatments for: deep acne scars, boxcar scars, icepick scars, rolling scars, actinic keratosis, seborrheic keratosis, solar lentigines, age spots, freckles, melasma, café-au-lait macules, hyperpigmentation disorders, sebaceous hyperplasia, syringomas, xanthelasma, warts, verrucae, skin tags, cherry angiomas, telangiectasias, spider veins, poikiloderma of Civatte, rhinophyma, HPV-related lesions, genital warts, laser-assisted drug delivery (LADD) preparation, keloid prevention post-excision, post-inflammatory hyperpigmentation, skin tightening, rosacea (select cases), epidermal nevi, dermatosis papulosa nigra, porokeratosis, scar contractures, nevus sebaceous, trichoepitheliomas, basal cell carcinoma (superficial cases), Bowen's disease, leukoplakia, lichen simplex chronicus, hypertrophic actinic damage, seborrheic warts, fibrous papules, milia, lip lines, smoker's lines, perioral rhytids, crow's feet, forehead lines, neck wrinkles, décolletage aging, hands rejuvenation, traumatic tattoos, benign fibromas, epidermoid cysts, angiofibromas, dermal melanocytosis, Becker's nevus, venous lakes, elastosis, Morbus Darier, keratoacanthoma, mucosal pigmentation disorders, extragenital lichen sclerosus.

In one aspect, the operator may have the option to finely adjust treatment parameters, including pulse length, fluence, MTZ width, and sequence or timing of the plurality of laser beams via the user interface 22 and control unit 21. This tailored approach permits optimal energy distribution for a wide array of dermatological treatments, such as skin rejuvenation, improvement of texture and tone, acne scar revision, wrinkle reduction, pigmentation correction, lesion removal, and comprehensive scar management.

Furthermore, the disclosed device may have the convenience of the operator being able to adjust the treatment parameters for each of the plurality of laser beams individually.

In one aspect, the fluence of any of the plurality of laser beams may be adjusted to a value in a range of 0.1 J/cm² to 500 J/cm² or in a range of 0.5 J/cm² to 400 J/cm² or in a range of 1 J/cm² to 360 J/cm². Additionally, the fluence of any of the plurality of laser beams may be adjusted to a value in a range of 1 J/cm² to 51000 J/cm².

In another aspect, the pulse length of any of the plurality of laser beams may be adjusted to a value in a range of 0.01 ms to 30 ms or in a range of 0.05 ms to 20 ms or in a range of 0.1 ms to 10 ms.

In one aspect, when the foot switch 28 is activated by the operator, the apparatus 1 may fire laser beam pulse stacks made by individual laser sources with a set frequency. Laser beam pulse stacks created by different laser sources may be fired in a simultaneous, successive or overlapping method.

In one aspect, the number of times the laser source fires in a single laser beam pulse stack may be adjusted to a value in a range of 1 to 100 or in a range of 1 to 50 or in a range of 1 to 10.

In another aspect, the frequency of the firing of laser beam pulse stacks created by a single laser source may be adjusted to a value in a range of 0.1 Hz to 50 Hz or in a range of 0.3 Hz to 30 Hz or in a range of 0.5 Hz to 20 Hz in a range of 0.1 Hz to 10 Hz or 0.5 Hz to 7 Hz or 1 Hz to 5 Hz. As a non-limiting example, when the single laser source comprises a stack of four laser beam pulses and a repetition frequency of 0.5 Hz to 20 Hz, it means that the stack of four pulses is repeated with this repetition frequency. The individual pulses within the stack have higher frequency.

For example, when using the fractional applicator, with the minimum MTZ width, the operator may have the option to adjust the fluence of the ablative laser beam in a range of 3 J/cm² to 132 J/cm², or the option to adjust the pulse length of the ablative laser beam in a range of 0.2 ms to 1.5 ms. Additionally, the operator may be able to adjust the number of pulses of the ablative laser beam in its laser beam pulse stack. The number of pulses of the ablative laser beam in one laser beam pulse stack may be in a range of 1 to 4. The operator may also be able to adjust the fluence of the non-ablative laser beam in a range of 10 J/cm² to 60 J/cm². Additionally, the operator may adjust the frequency of the ablative laser beam pulse stacks and also the non-ablative laser beam in a range of 1.5 Hz to 10 Hz.

In another example, the apparatus 1 may be used in a single wavelength mode, where a single laser beam pulse stack is fired repeatedly. If the ablative laser beam is used individually, while using the full field applicator with a minimal spot width, the operator may be able to adjust the fluence of the ablative laser beam to be in a range of 40 J/cm² to 355 J/cm². The operator may also be able to adjust the pulse length of the ablative laser beam to be in a range of 0.2 ms to 1.5 ms. If the non-ablative laser beam is used individually, the operator may be able to adjust the fluence of the non-ablative laser beam to be in a range of 1 J/cm² to 6 J/cm². The operator may also be able to adjust the pulse length of the non-ablative laser beam to be in a range of 2 ms to 4 ms. Additionally, the operator may adjust the frequency of the laser beam pulse stacks to a value in a range of 1.5 Hz to 20 Hz.

As is mentioned above, the present invention further benefits from integrated treatment protocols stored within the memory of the control unit, providing the operator with comprehensive guidelines tailored to various clinical applications. The treatment protocols may be configured for aesthetic (e.g. improvement of visual appearance), dentistry, surgery, ophthalmology and other. By way of example, and without limitation, the device's versatility can be illustrated through representative treatment scenarios as follows.

In one exemplary protocol, which may be configured for mild skin rejuvenation and textural improvement, the fractional applicator, with larger MTZ widths (e.g. 1000 µm to 1300 µm) may be used. The first laser source (non-ablative, approximately 1540 nm wavelength) may be operated with a fluence setting in a range of 1 J/cm² to 2 J/cm² or in a range of 1.3 J/cm² to 1.7 J/cm². In overlap or successively, the second laser source (ablative, approximately 2940 nm wavelength) may be activated at a fluence in a range of 0.1 J/cm² to 0.5 J/cm² or in a range of 0.2 J/cm² to 0.4 J/cm², a pulse length in a range of 0.1 ms to 0.5 ms or in a range of 0.2 ms to 0.4 ms with a single pulse in each laser beam pulse stack. The laser beam pulse stacks may be repeated with a frequency in a range of 1 Hz to 2 Hz or in the range 1.3Hz to 1.7 Hz. Such a protocol achieves controlled dermal heating coupled with precise superficial ablation, thus promoting effective collagen remodeling while minimizing patient discomfort and recovery downtime.

For more intensive resurfacing treatments targeting advanced signs of skin aging, a different exemplary protocol may involve operating the non-ablative laser source (approximately 1540 nm) with a fluence in a range of 10 J/cm² to 50 J/cm² or in a range of 20 J/cm² to 40 J/cm². Simultaneously, or following a brief interval, the ablative laser (approximately 2940 nm) is activated at increased fluence levels in a range of 2 J/cm² to 30 J/cm² or in a range of 5 J/cm² to 25 J/cm ², and a pulse length in a range of 0.1 ms to 0.5 ms or in a range of 0.2 ms to 0.4 ms with no stacking to maximize superficial precision and efficacy. The laser beam pulse stacks may have a frequency in a range of 0.5 Hz to 5 Hz or in a range of 1.5 Hz to 4 Hz. This approach provides synergistic benefits, balancing aggressive epidermal renewal with deep dermal restructuring to markedly improve skin texture and elasticity.

A protocol specifically adapted for the treatment of acne scars exemplifies further clinical utility. Here, treatment may only utilize the non-ablative laser source and laser beam, the parameters might involve a pulse fluence in a range of 30 J/cm² to 70 J/cm² or in a range of 40 J/cm² to 60 J/cm² at a frequency in a range of 0.5 Hz to 2.5 Hz or in a range of 1 Hz to 2 Hz, thereby fostering robust dermal remodeling, enhancing fibroblast activity, and improving overall skin structure with minimal side effects.

The integrated user interface facilitates precise adjustment of these parameters, allowing the operator to tailor treatment protocols dynamically according to individual patient needs and clinical objectives. Stored protocol presets offer foundational guidelines, from which customization is both intuitive and precise, underscoring the versatility and adaptability of the apparatus across a comprehensive range of dermatological and aesthetic procedures.

While the preceding description primarily refers to a single combined laser beam comprising multiple wavelengths, it is understood and considered as part of the present disclosure that the described aspects of systems and methods are equally applicable to configurations utilizing a single laser beam of a single wavelength, including combinations of such single laser beam of a single wavelength with respective features and embodiments as outlined above.

## Claims

1. A laser apparatus for treatment of patient tissue, comprising:
a plurality of laser sources for generating a plurality of laser beams, the plurality of laser sources comprising:
a first laser source configured to generate a first laser beam of a first wavelength configured to provide non-ablation of a tissue of the patient; and
a second laser source configured to generate a second laser beam of a second wavelength configured to provide ablation of the tissue of the patient,
wherein the first wavelength is different than the second wavelength, and
wherein each laser source of the plurality of the laser sources is configured to generate a pulsed laser beam;
a laser beam combiner coupled to the plurality of the laser sources, said laser beam combiner being configured to combine the plurality of laser beams of the predetermined wavelengths into a single combined laser beam of multiple wavelengths;
a collimator, configured to collimate the plurality of laser beams of the single combined laser beam;
a movable waveguide coupled to the collimator on a first side and configured to deliver the single combined laser beam to an applicator coupled to a second side of the waveguide;
the applicator configured to deliver the single combined laser beam to a target zone of the patient tissue,
wherein the collimator, the laser beam combiner and the applicator are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit in communication with the plurality of laser sources, said control unit being configured to control the plurality of laser sources in a manner that the plurality of laser beams combined into the single combined laser beam are delivered to the target zone of tissue in a simultaneous or overlapping method,
wherein the delivery of the plurality of laser beams in the simultaneous or overlapping method facilitates a combined effect of the effects of each individual laser beam of the plurality of laser beams.

2. The laser apparatus of claim 1,
wherein the applicator further comprises a fractional element configured to split the single combined laser beam delivered to the applicator into a predetermined fractional pattern of spatially separated micro beams, and
wherein the fractional element comprises a micro-lens element configured to split the single combined laser beam into a matrix of micro beams in the range of 3 x 3 to 30 x 30,
wherein the micro beams create micro thermal zones on impact with the patient tissue having their centers apart in a range of 500 µm to 2000 µm, and wherein each micro thermal zone has a width in a range of 100 µm to 1500 µm, and
wherein the target zone has a size in the range of 1 mm² to 25 cm².

3. The laser apparatus of one of the preceding claims,
wherein the waveguide comprises an articulated arm comprising 5 to 10 joints, wherein each joint comprises a 45 degree mirror, and
wherein the articulated arm has a length in a range of 1 m to 2.5m.

4. The laser apparatus of one of the preceding claims,
wherein the collimator is a single collimator configured to align the plurality of laser beams inside the single combined laser beam such that the differences in their radii at the end of the articulated arm are in a range of 0.2 % to 25 %.

5. The laser apparatus of one of the preceding claims,
wherein optical distances from the plurality of laser sources to the collimator are adjusted such that the differences in beam radii at the output of the articulated arm is in a range of 0.3% to 20%,
wherein the optical distance from each laser source to the collimator is in a range of 200 mm to 700 mm, and
wherein the optical distance from the first laser source to the collimator is shorter by an amount in a range of 1 mm to 100 mm relative to the optical distance from the second laser source to the collimator.

6. The laser apparatus of one of the preceding claims,
further comprising a user interface in communication with the control unit,
wherein the user interface is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

7. The laser apparatus of claim 1,
wherein the first laser source is configured to generate a non-ablative laser beam in a range of 1000 nm to 1800 nm, and
wherein the second laser source is configured to generate an ablative laser beam in a range of 2500 nm to 3500 nm.

8. The laser apparatus of one of the preceding claims,
wherein the overlapping method comprises operating the first laser source to generate the first laser beam pulse during a first time interval and operating the second laser source to generate the second laser beam pulse during a second time interval that overlaps with at least a portion of the first time interval, and
wherein both the first and second laser beams are simultaneously active during an overlapping subinterval of said overlapping method time interval.

9. The laser apparatus of claim 8,
wherein a duration of the overlapping subinterval is in a range of 1% to 90% of the shortest pulse length among the first laser beam pulse and the second laser beam pulse.

10. The laser apparatus of one of the preceding claims,
wherein the first laser source comprises:
a solid state laser rod configured to generate a laser beam; and
a flash lamp configured to produce a broad-spectrum light flash configured to energize the solid state laser rod;
wherein the control unit is configured to control the first laser source such that:
(i) the flash lamp energizes the solid state laser rod with a therapeutic flash that is sufficient to pass a laser activation threshold and the solid state laser rod produces the first laser beam for the treatment of the patient tissue; and
(ii) the flash lamp energizes the solid state laser rod with a heat flash that is not sufficient to pass the laser activation threshold and the solid-state laser rod does not produce the first laser beam for the treatment of the patient tissue, thus keeping the solid state laser rod thermally primed,
wherein the solid state laser rod is kept thermally primed while not producing laser pulses for the treatment of the patient tissue.

11. The laser apparatus of claim 10,
wherein the first laser source further comprises a flash lamp charger electrically coupled to the flash lamp,
wherein the flash lamp is configured to generate the heat flash and the therapeutic flash, when energized by the first flash lamp charger, and
wherein the control unit configured to control the flash lamp charger to deliver:
(i) a heat current pulse to the flash lamp configured to energize the flash lamp to generate the heat flash, and
(ii) a therapeutic current pulse to the flash lamp configured to energize the flash lamp to generate the therapeutic flash,
wherein the control unit is configured to control the flash lamp charger such that a plurality of heat current pulses precede one or more therapeutic current pulses.

12. The laser apparatus of claim 11,
wherein a frequency of heat current pulses is in a range of 1 Hz to 200 Hz.

13. A method for irradiating a target zone, preferably by a laser apparatus of one of the preceding claims, the method comprising:
generating a plurality of laser beams from a plurality of laser sources, wherein the plurality of laser beams comprises:
a first laser beam having a first wavelength configured to provide a non-ablative laser beam; and
a second laser beam having a second wavelength configured to provide ablative laser beam,
wherein the first wavelength is different from the second wavelength;
combining the plurality of laser beams of different wavelengths into a single combined laser beam of multiple wavelengths by a laser beam combiner;
collimating the single combined laser beam to maintain the beam in a substantially parallel form by a collimator;
transmitting the collimated single combined laser beam via a movable waveguide to an applicator positioned adjacent to a target zone; and
irradiating the target zone in a simultaneous or overlapping method, such that all of the plurality of the laser beams in the single combined laser beam:
(i) irradiate the same area of the target zone, or
(ii) irradiate the target zone such that the centers of the plurality of laser beams are aligned to a common point on the target zone.

14. The method of claim 13, further comprising:
splitting the single combined laser beam into a plurality of spatially separated micro beams using a fractional element and delivering them to the target zone in a matrix pattern, wherein the splitting maintains effective overlap of the plurality of laser beams in time and space,
wherein the fractional element comprises a micro-lens element configured to split the single combined laser beam into a matrix of micro beams in the range of 3 x 3 to 30 x 30,
wherein the micro beams create micro thermal zones on impact having their centers 500 µm to 2000 µm apart, each micro thermal zone has a width in the range of 100 µm to 1500 µm, and wherein the target zone has a size in the range of 1 mm² to 25 cm².

15. The method of one of claims 13 or 14,
wherein the first laser beam has a wavelength in a range of 1000 nm to 1800 nm and the second laser beam has a wavelength in a range of 2500 nm to 3500 nm.

## Amended claims

### Amended claims in accordance with Rule 137(2) EPC.

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
an applicator (4) configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the apparatus (1) further comprises an articulated arm (3) coupled to the collimator (27) on a first side and to the applicator (4) on a second side; [par. [0027], [0194], [0223]]
wherein the articulated arm (3) comprises 3 to 15 joints, wherein each joint comprises a 45 degree mirror, and wherein the articulated arm (3) has a length in a range of 1 m to 2.5 m and is configured to deliver the single combined laser beam to the applicator (4). [par. [0221], [0223]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the articulated arm are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the articulated arm is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21), wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of anyone of the preceding claims,
wherein the first laser source (23a) is configured to generate a non-ablative laser beam in a range of 1000 nm to 1800 nm, and
wherein the second laser source (23b) is configured to generate an ablative laser beam in a range of 2500 nm to 3500 nm.

**8.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**9.** The laser apparatus (1) of claim 8,
wherein a duration of the overlapping subinterval is in a range of 1% to 90% of the shortest pulse length among the first laser beam pulse and the second laser beam pulse.

**10.** The laser apparatus (1) of anyone of the preceding claims,
wherein the first laser source (23a) comprises:
a solid state laser rod (234) configured to generate a laser beam; and
a flash lamp (233) configured to produce a broad-spectrum light flash configured to energize the solid state laser rod (234);
wherein the control unit (21) is configured to control the first laser source (23a) such that:
the flash lamp (233) energizes the solid state laser rod (234) with a therapeutic flash that is sufficient to pass a laser activation threshold and the solid state laser rod (234) produces the first laser beam for the treatment of the patient tissue (5); and
the flash lamp (233) energizes the solid state laser rod (234) with a heat flash that is not sufficient to pass the laser activation threshold and the solid-state laser rod does not produce the first laser beam for the treatment of the patient tissue (5), thus keeping the solid state laser rod (234) thermally primed,
wherein the solid state laser rod (234) is kept thermally primed while not producing laser pulses for the treatment of the patient tissue (5).

**11.** The laser apparatus (1) of claim 10,
wherein the first laser source (23a) further comprises a flash lamp charger (232) electrically coupled to the flash lamp (233),
wherein the flash lamp (233) is configured to generate the heat flash and the therapeutic flash, when energized by the first flash lamp charger (232), and
wherein the control unit (21) configured to control the flash lamp charger (232) to deliver:
(i) a heat current pulse to the flash lamp (233) configured to energize the flash lamp (233) to generate the heat flash, and
(ii) a therapeutic current pulse to the flash lamp (233) configured to energize the flash lamp (233) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** A method of operation of a laser apparatus (1), the apparatus comprising a plurality of laser sources (23), a laser beam combiner (26), a collimator (27), an applicator (4), a control unit (21), and an articulated arm (3) coupled to the collimator (27) on a first side and to the applicator (4) on a second side, wherein the articulated arm (3) comprises 3 to 15 joints, each joint comprising a 45-degree mirror, and wherein the articulated arm (3) has a length in a range of 1 m to 2.5 m, the method comprising steps of:
generating, by a first laser source (23a) of the plurality of laser sources (23), a pulsed first non-ablative laser beam of a first wavelength;
generating by a second laser source (23b) of the plurality of laser sources (23), a pulsed second ablative laser beam of a second wavelength, wherein the first wavelength is different than the second wavelength;
combining, by the laser beam combiner (26), the first and second laser beams into a single combined laser beam of multiple wavelengths;
collimating, by the collimator (27), the single combined laser beam to maintain the beam in a substantially parallel form;
guiding the single combined laser beam through the articulated arm (3) to the applicator (4);
directing, via the applicator (4), the single combined laser beam; and
controlling, by the control unit (21), the plurality of laser sources (23) such that the first and second laser beams combined into the single combined laser beam are emitted in a simultaneous or overlapping manner;
wherein the single combined laser beam is directed such that the individual laser beams have a common cross-sectional area, or are directed such that the beam axes of the individual laser beams intersect at a common focal point and their cross-sectional areas overlap.

**14.** The method of claim 13, further comprising:
splitting the single combined laser beam into a plurality of spatially separated micro beams using a fractional element (44); and
delivering the plurality of spatially separated micro-beams in a patterned array of micro beams,
wherein the splitting maintains effective overlap of the plurality of laser beams within each micro-beam in time and space,
wherein the fractional element (44) comprises a micro-lens element configured to split the single combined laser beam into a matrix of micro beams in the range of 3 x 3 to 30 x 30,
wherein the micro beams create micro thermal zones on impact having their centers 500 µm to 2000 µm apart, each micro thermal zone has a width in the range of 100 µm to 1500 µm, and
wherein the total area containing the matrix of micro beams has a size in the range of 1 mm² to 25 cm².

**15.** The method of one of claims 13 or 14,
wherein the first laser beam has a wavelength in a range of 1000 nm to 1800 nm and the second laser beam has a wavelength in a range of 2500 nm to 3500 nm.

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
an applicator (4) configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the apparatus (1) further comprises an articulated arm (3) coupled to the collimator (27) on a first side and to the applicator (4) on a second side; [par. [0027], [0194], [0223]]
wherein the articulated arm (3) comprises 5 to 10 joints, wherein each joint comprises a 45 degree mirror, and wherein the articulated arm (3) has a length in a range of 1 m to 2.5 m and is configured to deliver the single combined laser beam to the applicator (4). [par. [0221], [0223]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the articulated arm are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the articulated arm is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21),
wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of anyone of the preceding claims,
wherein the first laser source (23a) is configured to generate a non-ablative laser beam in a range of 1000 nm to 1800 nm, and
wherein the second laser source (23b) is configured to generate an ablative laser beam in a range of 2500 nm to 3500 nm.

**8.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**9.** The laser apparatus (1) of claim 8,
wherein a duration of the overlapping subinterval is in a range of 1% to 90% of the shortest pulse length among the first laser beam pulse and the second laser beam pulse.

**10.** The laser apparatus (1) of anyone of the preceding claims,
wherein the first laser source (23a) comprises:
a solid state laser rod (234) configured to generate a laser beam; and
a flash lamp (233) configured to produce a broad-spectrum light flash configured to energize the solid state laser rod (234);
wherein the control unit (21) is configured to control the first laser source (23a) such that:
the flash lamp (233) energizes the solid state laser rod (234) with a therapeutic flash that is sufficient to pass a laser activation threshold and the solid state laser rod (234) produces the first laser beam for the treatment of the patient tissue (5); and
the flash lamp (233) energizes the solid state laser rod (234) with a heat flash that is not sufficient to pass the laser activation threshold and the solid-state laser rod does not produce the first laser beam for the treatment of the patient tissue (5), thus keeping the solid state laser rod (234) thermally primed,
wherein the solid state laser rod (234) is kept thermally primed while not producing laser pulses for the treatment of the patient tissue (5).

**11.** The laser apparatus (1) of claim 10,
wherein the first laser source (23a) further comprises a flash lamp charger (232) electrically coupled to the flash lamp (233),
wherein the flash lamp (233) is configured to generate the heat flash and the therapeutic flash, when energized by the first flash lamp charger (232), and
wherein the control unit (21) configured to control the flash lamp charger (232) to deliver:
(i) a heat current pulse to the flash lamp (233) configured to energize the flash lamp (233) to generate the heat flash, and
(ii) a therapeutic current pulse to the flash lamp (233) configured to energize the flash lamp (233) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the articulated arm (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength in a range of 2500 nm to 3500 nm configured to provide ablation of the tissue of the patient, [par. [0090]]
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
an applicator (4) configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the apparatus (1) further comprises an articulated arm (3) coupled to the collimator (27) on a first side and to the applicator (4) on a second side; [par. [0027], [0194], [0223]]
wherein the articulated arm (3) comprises 5 to 10 joints, wherein each joint comprises a 45 degree mirror, and wherein the articulated arm (3) has a length in a range of 1 m to 2.5 m and is configured to deliver the single combined laser beam to the applicator (4). [par. [0221], [0223]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the articulated arm are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the articulated arm is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21),
wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of anyone of the preceding claims,
wherein the first laser source (23a) is configured to generate a non-ablative laser beam in a range of 1000 nm to 1800 nm.

**8.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**9.** The laser apparatus (1) of claim 8,
wherein a duration of the overlapping subinterval is in a range of 1% to 90% of the shortest pulse length among the first laser beam pulse and the second laser beam pulse.

**10.** The laser apparatus (1) of anyone of the preceding claims,
wherein the first laser source (23a) comprises:
a solid state laser rod (234) configured to generate a laser beam; and
a flash lamp (233) configured to produce a broad-spectrum light flash configured to energize the solid state laser rod (234);
wherein the control unit (21) is configured to control the first laser source (23a) such that:
the flash lamp (233) energizes the solid state laser rod (234) with a therapeutic flash that is sufficient to pass a laser activation threshold and the solid state laser rod (234) produces the first laser beam for the treatment of the patient tissue (5); and
the flash lamp (233) energizes the solid state laser rod (234) with a heat flash that is not sufficient to pass the laser activation threshold and the solid-state laser rod does not produce the first laser beam for the treatment of the patient tissue (5), thus keeping the solid state laser rod (234) thermally primed,
wherein the solid state laser rod (234) is kept thermally primed while not producing laser pulses for the treatment of the patient tissue (5).

**11.** The laser apparatus (1) of claim 10,
wherein the first laser source (23a) further comprises a flash lamp charger (232) electrically coupled to the flash lamp (233),
wherein the flash lamp (233) is configured to generate the heat flash and the therapeutic flash, when energized by the first flash lamp charger (232), and
wherein the control unit (21) configured to control the flash lamp charger (232) to deliver:
(i) a heat current pulse to the flash lamp (233) configured to energize the flash lamp (233) to generate the heat flash, and
(ii) a therapeutic current pulse to the flash lamp (233) configured to energize the flash lamp (233) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the articulated arm (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
a movable waveguide (3) coupled to the collimator (27) on a first side; [par. [0027]] an applicator (4) coupled to a second side of the waveguide (3),
wherein the applicator (4) is configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the second laser source (23b) comprises a solid state laser rod (234) configured to generate the second laser beam in a range of 2500 nm to 3500 nm. [par. [0024], [0079], [0090]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the waveguide (3) are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the waveguide (3) is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21),
wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of anyone of the preceding claims,
wherein the first laser source (23a) is configured to generate a non-ablative laser beam in a range of 1000 nm to 1800 nm.

**8.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**9.** The laser apparatus (1) of claim 8,
wherein a duration of the overlapping subinterval is in a range of 1% to 90% of the shortest pulse length among the first laser beam pulse and the second laser beam pulse.

**10.** The laser apparatus (1) of anyone of the preceding claims,
wherein the first laser source (23a) comprises:
a solid state laser rod (234) configured to generate a laser beam; and
a flash lamp (233) configured to produce a broad-spectrum light flash configured to energize the solid state laser rod (234);
wherein the control unit (21) is configured to control the first laser source (23a) such that:
the flash lamp (233) energizes the solid state laser rod (234) with a therapeutic flash that is sufficient to pass a laser activation threshold and the solid state laser rod (234) produces the first laser beam for the treatment of the patient tissue (5); and
the flash lamp (233) energizes the solid state laser rod (234) with a heat flash that is not sufficient to pass the laser activation threshold and the solid-state laser rod does not produce the first laser beam for the treatment of the patient tissue (5), thus keeping the solid state laser rod (234) thermally primed,
wherein the solid state laser rod (234) is kept thermally primed while not producing laser pulses for the treatment of the patient tissue (5).

**11.** The laser apparatus (1) of claim 10,
wherein the first laser source (23a) further comprises a flash lamp charger (232) electrically coupled to the flash lamp (233),
wherein the flash lamp (233) is configured to generate the heat flash and the therapeutic flash, when energized by the first flash lamp charger (232), and
wherein the control unit (21) configured to control the flash lamp charger (232) to deliver:
(i) a heat current pulse to the flash lamp (233) configured to energize the flash lamp (233) to generate the heat flash, and
(ii) a therapeutic current pulse to the flash lamp (233) configured to energize the flash lamp (233) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the waveguide (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
an applicator (4) configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the second laser source (23b) comprises a solid state laser rod (234) configured to generate the second laser beam in a range of 2500 nm to 3500 nm [par. [0024], [0079], [0090]]; and
the apparatus (1) further comprises an articulated arm (3) coupled to the collimator (27) on a first side and to the applicator (4) on a second side; [par. [0027], [0194], [0223]]
wherein the articulated arm (3) comprises 5 to 10 joints, wherein each joint comprises a 45 degree mirror, and wherein the articulated arm (3) has a length in a range of 1 m to 2.5 m and is configured to deliver the single combined laser beam to the applicator (4). [par. [0221], [0223]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the articulated arm are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the articulated arm is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21),
wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of anyone of the preceding claims,
wherein the first laser source (23a) is configured to generate a non-ablative laser beam in a range of 1000 nm to 1800 nm.

**8.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**9.** The laser apparatus (1) of claim 8,
wherein a duration of the overlapping subinterval is in a range of 1% to 90% of the shortest pulse length among the first laser beam pulse and the second laser beam pulse.

**10.** The laser apparatus (1) of anyone of the preceding claims,
wherein the first laser source (23a) comprises:
a solid state laser rod (234) configured to generate a laser beam; and
a flash lamp (233) configured to produce a broad-spectrum light flash configured to energize the solid state laser rod (234);
wherein the control unit (21) is configured to control the first laser source (23a) such that:
the flash lamp (233) energizes the solid state laser rod (234) with a therapeutic flash that is sufficient to pass a laser activation threshold and the solid state laser rod (234) produces the first laser beam for the treatment of the patient tissue (5); and
the flash lamp (233) energizes the solid state laser rod (234) with a heat flash that is not sufficient to pass the laser activation threshold and the solid-state laser rod does not produce the first laser beam for the treatment of the patient tissue (5), thus keeping the solid state laser rod (234) thermally primed,
wherein the solid state laser rod (234) is kept thermally primed while not producing laser pulses for the treatment of the patient tissue (5).

**11.** The laser apparatus (1) of claim 10,
wherein the first laser source (23a) further comprises a flash lamp charger (232) electrically coupled to the flash lamp (233),
wherein the flash lamp (233) is configured to generate the heat flash and the therapeutic flash, when energized by the first flash lamp charger (232), and
wherein the control unit (21) configured to control the flash lamp charger (232) to deliver:
(i) a heat current pulse to the flash lamp (233) configured to energize the flash lamp (233) to generate the heat flash, and
(ii) a therapeutic current pulse to the flash lamp (233) configured to energize the flash lamp (233) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the articulated arm (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
a movable waveguide (3) coupled to the collimator (27) on a first side; [par. [0027]] an applicator (4) coupled to a second side of the waveguide (3),
wherein the applicator (4) is configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the first wavelength is in a range of 1000 nm to 1800 nm [par. [0086]]; and the second laser source (23b) comprises a solid state laser rod (234) configured to generate the second laser beam in a range of 2500 nm to 3500 nm. [par. [0079], [0090]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the waveguide (3) are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the waveguide (3) is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21),
wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of anyone of the preceding claims,
wherein the first laser source is configured to generate a non-ablative laser beam in a range of 1520 nm to 1550 nm, and
wherein the second laser source is configured to generate an ablative laser beam in a range of 2900 nm to 2980 nm. [par. [0086],[0090]]

**8.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**9.** The laser apparatus (1) of claim 8,
wherein a duration of the overlapping subinterval is in a range of 1% to 90% of the shortest pulse length among the first laser beam pulse and the second laser beam pulse.

**10.** The laser apparatus (1) of anyone of the preceding claims,
wherein the first laser source (23a) comprises:
a solid state laser rod (234) configured to generate a laser beam; and
a flash lamp (233) configured to produce a broad-spectrum light flash configured to energize the solid state laser rod (234);
wherein the control unit (21) is configured to control the first laser source (23a) such that:
the flash lamp (233) energizes the solid state laser rod (234) with a therapeutic flash that is sufficient to pass a laser activation threshold and the solid state laser rod (234) produces the first laser beam for the treatment of the patient tissue (5); and
the flash lamp (233) energizes the solid state laser rod (234) with a heat flash that is not sufficient to pass the laser activation threshold and the solid-state laser rod does not produce the first laser beam for the treatment of the patient tissue (5), thus keeping the solid state laser rod (234) thermally primed,
wherein the solid state laser rod (234) is kept thermally primed while not producing laser pulses for the treatment of the patient tissue (5).

**11.** The laser apparatus (1) of claim 10,
wherein the first laser source (23a) further comprises a flash lamp charger (232) electrically coupled to the flash lamp (233),
wherein the flash lamp (233) is configured to generate the heat flash and the therapeutic flash, when energized by the first flash lamp charger (232), and
wherein the control unit (21) configured to control the flash lamp charger (232) to deliver:
(i) a heat current pulse to the flash lamp (233) configured to energize the flash lamp (233) to generate the heat flash, and
(ii) a therapeutic current pulse to the flash lamp (233) configured to energize the flash lamp (233) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the waveguide (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
an applicator (4) configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the first wavelength is in a range of 1000 nm to 1800 nm [par. [0086]];
the second laser source (23b) comprises a solid state laser rod (234) configured to generate the second laser beam in a range of 2500 nm to 3500 nm [par. [0079], [0090]]; and
the apparatus (1) further comprises an articulated arm (3) coupled to the collimator (27) on a first side and to the applicator (4) on a second side; [par. [0027], [0194], [0223]]
wherein the articulated arm (3) comprises 5 to 10 joints, wherein each joint comprises a 45 degree mirror, and wherein the articulated arm (3) has a length in a range of 1 m to 2.5 m and is configured to deliver the single combined laser beam to the applicator (4). [par. [0221], [0223]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the articulated arm are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the articulated arm is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21),
wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of anyone of the preceding claims,
wherein the first laser source is configured to generate a non-ablative laser beam in a range of 1520 nm to 1550 nm, and
wherein the second laser source is configured to generate an ablative laser beam in a range of 2900 nm to 2980 nm. [par. [0086],[0090]]

**8.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**9.** The laser apparatus (1) of claim 8,
wherein a duration of the overlapping subinterval is in a range of 1% to 90% of the shortest pulse length among the first laser beam pulse and the second laser beam pulse.

**10.** The laser apparatus (1) of anyone of the preceding claims,
wherein the first laser source (23a) comprises:
a solid state laser rod (234) configured to generate a laser beam; and
a flash lamp (233) configured to produce a broad-spectrum light flash configured to energize the solid state laser rod (234);
wherein the control unit (21) is configured to control the first laser source (23a) such that:
the flash lamp (233) energizes the solid state laser rod (234) with a therapeutic flash that is sufficient to pass a laser activation threshold and the solid state laser rod (234) produces the first laser beam for the treatment of the patient tissue (5); and
the flash lamp (233) energizes the solid state laser rod (234) with a heat flash that is not sufficient to pass the laser activation threshold and the solid-state laser rod does not produce the first laser beam for the treatment of the patient tissue (5), thus keeping the solid state laser rod (234) thermally primed,
wherein the solid state laser rod (234) is kept thermally primed while not producing laser pulses for the treatment of the patient tissue (5).

**11.** The laser apparatus (1) of claim 10,
wherein the first laser source (23a) further comprises a flash lamp charger (232) electrically coupled to the flash lamp (233),
wherein the flash lamp (233) is configured to generate the heat flash and the therapeutic flash, when energized by the first flash lamp charger (232), and
wherein the control unit (21) configured to control the flash lamp charger (232) to deliver:
(i) a heat current pulse to the flash lamp (233) configured to energize the flash lamp (233) to generate the heat flash, and
(ii) a therapeutic current pulse to the flash lamp (233) configured to energize the flash lamp (233) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the articulated arm (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
a movable waveguide (3) coupled to the collimator (27) on a first side;
an applicator (4) coupled to a second side of the waveguide (3),
wherein the applicator (4) is configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the second laser source (23b) comprises an Er:YAG laser rod (234) configured to generate the second laser beam at approximately 2940 nm. [par. [0093]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the waveguide (3) are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the waveguide (3) is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21),
wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of anyone of the preceding claims,
wherein the first laser source (23a) is configured to generate a non-ablative laser beam in a range of 1000 nm to 1800 nm.

**8.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**9.** The laser apparatus (1) of claim 8,
wherein a duration of the overlapping subinterval is in a range of 1% to 90% of the shortest pulse length among the first laser beam pulse and the second laser beam pulse.

**10.** The laser apparatus (1) of anyone of the preceding claims,
wherein the first laser source (23a) comprises:
a solid state laser rod (234) configured to generate a laser beam; and
a flash lamp (233) configured to produce a broad-spectrum light flash configured to energize the solid state laser rod (234);
wherein the control unit (21) is configured to control the first laser source (23a) such that:
the flash lamp (233) energizes the solid state laser rod (234) with a therapeutic flash that is sufficient to pass a laser activation threshold and the solid state laser rod (234) produces the first laser beam for the treatment of the patient tissue (5); and
the flash lamp (233) energizes the solid state laser rod (234) with a heat flash that is not sufficient to pass the laser activation threshold and the solid-state laser rod does not produce the first laser beam for the treatment of the patient tissue (5), thus keeping the solid state laser rod (234) thermally primed,
wherein the solid state laser rod (234) is kept thermally primed while not producing laser pulses for the treatment of the patient tissue (5).

**11.** The laser apparatus (1) of claim 10,
wherein the first laser source (23a) further comprises a flash lamp charger (232) electrically coupled to the flash lamp (233),
wherein the flash lamp (233) is configured to generate the heat flash and the therapeutic flash, when energized by the first flash lamp charger (232), and
wherein the control unit (21) configured to control the flash lamp charger (232) to deliver:
(i) a heat current pulse to the flash lamp (233) configured to energize the flash lamp (233) to generate the heat flash, and
(ii) a therapeutic current pulse to the flash lamp (233) configured to energize the flash lamp (233) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the waveguide (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
an applicator (4) configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the second laser source (23b) comprises an Er:YAG laser rod (234) configured to generate the second laser beam at approximately 2940 nm [par. [0093]]; and
the apparatus (1) further comprises an articulated arm (3) coupled to the collimator (27) on a first side and to the applicator (4) on a second side; [par. [0027], [0194], [0223]]
wherein the articulated arm (3) comprises 5 to 10 joints, wherein each joint comprises a 45 degree mirror, and wherein the articulated arm (3) has a length in a range of 1 m to 2.5 m and is configured to deliver the single combined laser beam to the applicator (4). [par. [0221], [0223]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the articulated arm are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the articulated arm is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21),
wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of anyone of the preceding claims,
wherein the first laser source (23a) is configured to generate a non-ablative laser beam in a range of 1000 nm to 1800 nm.

**8.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**9.** The laser apparatus (1) of claim 8,
wherein a duration of the overlapping subinterval is in a range of 1% to 90% of the shortest pulse length among the first laser beam pulse and the second laser beam pulse.

**10.** The laser apparatus (1) of anyone of the preceding claims,
wherein the first laser source (23a) comprises:
a solid state laser rod (234) configured to generate a laser beam; and
a flash lamp (233) configured to produce a broad-spectrum light flash configured to energize the solid state laser rod (234);
wherein the control unit (21) is configured to control the first laser source (23a) such that:
the flash lamp (233) energizes the solid state laser rod (234) with a therapeutic flash that is sufficient to pass a laser activation threshold and the solid state laser rod (234) produces the first laser beam for the treatment of the patient tissue (5); and
the flash lamp (233) energizes the solid state laser rod (234) with a heat flash that is not sufficient to pass the laser activation threshold and the solid-state laser rod does not produce the first laser beam for the treatment of the patient tissue (5), thus keeping the solid state laser rod (234) thermally primed,
wherein the solid state laser rod (234) is kept thermally primed while not producing laser pulses for the treatment of the patient tissue (5).

**11.** The laser apparatus (1) of claim 10,
wherein the first laser source (23a) further comprises a flash lamp charger (232) electrically coupled to the flash lamp (233),
wherein the flash lamp (233) is configured to generate the heat flash and the therapeutic flash, when energized by the first flash lamp charger (232), and
wherein the control unit (21) configured to control the flash lamp charger (232) to deliver:
(i) a heat current pulse to the flash lamp (233) configured to energize the flash lamp (233) to generate the heat flash, and
(ii) a therapeutic current pulse to the flash lamp (233) configured to energize the flash lamp (233) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the articulated arm (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
a movable waveguide (3) coupled to the collimator (27) on a first side;
an applicator (4) coupled to a second side of the waveguide (3),
wherein the applicator (4) is configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the first laser source (23a) comprises an Er:glass laser rod (234a) configured to generate the first laser beam at approximately 1540 nm [par. [0089]]; and
the second laser source (23b) comprises an Er:YAG laser rod (234b) configured to generate the second laser beam at approximately 2940 nm. [par. [0093]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the waveguide (3) are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the waveguide (3) is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21),
wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**8.** The laser apparatus (1) of claim 7,
wherein a duration of the overlapping subinterval is in a range of 1% to 90% of the shortest pulse length among the first laser beam pulse and the second laser beam pulse.

**9.** The laser apparatus (1) of anyone of the preceding claims,
wherein the first laser source (23a) comprises a first flash lamp (233a) configured to produce a broad-spectrum light flash configured to energize the Er:glass laser rod (234a);
wherein the control unit (21) is configured to control the first laser source (23a) such that:
the first flash lamp (233a) energizes the Er:glass laser rod (234a) with a therapeutic flash that is sufficient to pass a laser activation threshold and the Er:glass laser rod (234) produces the first laser beam for the treatment of the patient tissue (5); and
the first flash lamp (233a) energizes the Er:glass laser rod (234a) with a heat flash that is not sufficient to pass the laser activation threshold and the solid-state laser rod does not produce the first laser beam for the treatment of the patient tissue (5), thus keeping the Er:glass laser rod (234a) thermally primed,
wherein the Er:glass laser rod (234a) is kept thermally primed while not producing laser pulses for the treatment of the patient tissue (5).

**10.** The laser apparatus of claim 9, wherein the first laser source (23a) comprises a first laser chamber (231a) comprising a first flowtube assembly (235a) configured to hold the Er:glass laser rod (234a) and the first flash lamp (233a) such that they are cooled by a cooling liquid flowing in their proximity, and
the second laser source (23b) comprises a second laser chamber (231b) comprising a second flowtube assembly (235bn) configured to hold the Er:YAG laser rod (234b) and a second flash lamp (233b) such that they are cooled by the cooling liquid flowing in their proximity. [par. [0107]]

**11.** The laser apparatus (1) of claim 9 or 10,
wherein the first laser source (23a) further comprises a flash lamp charger (232) electrically coupled to the first flash lamp (233a),
wherein the first flash lamp (233a) is configured to generate the heat flash and the therapeutic flash, when energized by the first flash lamp charger (232), and
wherein the control unit (21) configured to control the flash lamp charger (232) to deliver:
(i) a heat current pulse to the first flash lamp (233a) configured to energize the first flash lamp (233a) to generate the heat flash, and
(ii) a therapeutic current pulse to the first flash lamp (233a) configured to energize the first flash lamp (233a) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the waveguide (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
an applicator (4) configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** - the first laser source (23a) comprises an Er:glass laser rod (234a) configured to generate the first laser beam at approximately 1540 nm [par. [0089]];
the second laser source (23b) comprises an Er:YAG laser rod (234b) configured to generate the second laser beam at approximately 2940 nm; and [par. [0093]]
the apparatus (1) further comprises an articulated arm (3) coupled to the collimator (27) on a first side and to the applicator (4) on a second side; [par. [0027], [0194], [0223]]
wherein the articulated arm (3) comprises 5 to 10 joints, wherein each joint comprises a 45 degree mirror, and wherein the articulated arm (3) has a length in a range of 1 m to 2.5 m and is configured to deliver the single combined laser beam to the applicator (4). [par. [0221], [0223]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the articulated arm are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the articulated arm is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21), wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**8.** The laser apparatus (1) of claim 7,
wherein a duration of the overlapping subinterval is in a range of 1% to 90% of the shortest pulse length among the first laser beam pulse and the second laser beam pulse.

**9.** The laser apparatus (1) of anyone of the preceding claims,
wherein the first laser source (23a) comprises a first flash lamp (233a) configured to produce a broad-spectrum light flash configured to energize the Er:glass laser rod (234a);
wherein the control unit (21) is configured to control the first laser source (23a) such that:
the first flash lamp (233a) energizes the Er:glass laser rod (234a) with a therapeutic flash that is sufficient to pass a laser activation threshold and the Er:glass laser rod (234) produces the first laser beam for the treatment of the patient tissue (5); and
the first flash lamp (233a) energizes the Er:glass laser rod (234a) with a heat flash that is not sufficient to pass the laser activation threshold and the solid-state laser rod does not produce the first laser beam for the treatment of the patient tissue (5), thus keeping the Er:glass laser rod (234a) thermally primed,
wherein the Er:glass laser rod (234a) is kept thermally primed while not producing laser pulses for the treatment of the patient tissue (5).

**10.** The laser apparatus of claim 9, wherein the first laser source (23a) comprises a first laser chamber (231a) comprising a first flowtube assembly (235a) configured to hold the Er:glass laser rod (234a) and the first flash lamp (233a) such that they are cooled by a cooling liquid flowing in their proximity, and
the second laser source (23b) comprises a second laser chamber (231b) comprising a second flowtube assembly (235bn) configured to hold the Er:YAG laser rod (234b) and a second flash lamp (233b) such that they are cooled by the cooling liquid flowing in their proximity. [par. [0107]]

**11.** The laser apparatus (1) of claim 9 or 10,
wherein the first laser source (23a) further comprises a flash lamp charger (232) electrically coupled to the first flash lamp (233a),
wherein the first flash lamp (233a) is configured to generate the heat flash and the therapeutic flash, when energized by the first flash lamp charger (232), and
wherein the control unit (21) configured to control the flash lamp charger (232) to deliver:
(i) a heat current pulse to the first flash lamp (233a) configured to energize the first flash lamp (233a) to generate the heat flash, and
(ii) a therapeutic current pulse to the first flash lamp (233a) configured to energize the first flash lamp (233a) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the articulated arm (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
a movable waveguide (3) coupled to the collimator (27) on a first side;
an applicator (4) coupled to a second side of the waveguide (3),
wherein the applicator (4) is configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the collimator (27) is a single collimator configured to collimate all laser beams from all laser sources simultaneously, and an axial displacement of each laser source (23a, 23b) from the single collimator (27) is parametrically determined by the product of a beam propagation ratio (M²) and a waist radius of the respective laser source (23a, 23b) [par. [0194], [0197], [0201]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the waveguide (3) are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the waveguide (3) is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21),
wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of claim 1,
wherein the first laser source (23a) is configured to generate a non-ablative laser beam in a range of 1000 nm to 1800 nm.

**8.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**9.** The laser apparatus (1) of claim 8,
wherein a duration of the overlapping subinterval is in a range of 1% to 90% of the shortest pulse length among the first laser beam pulse and the second laser beam pulse.

**10.** The laser apparatus (1) of anyone of the preceding claims,
wherein the first laser source (23a) comprises:
a solid state laser rod (234) configured to generate a laser beam; and
a flash lamp (233) configured to produce a broad-spectrum light flash configured to energize the solid state laser rod (234);
wherein the control unit (21) is configured to control the first laser source (23a) such that:
the flash lamp (233) energizes the solid state laser rod (234) with a therapeutic flash that is sufficient to pass a laser activation threshold and the solid state laser rod (234) produces the first laser beam for the treatment of the patient tissue (5); and
the flash lamp (233) energizes the solid state laser rod (234) with a heat flash that is not sufficient to pass the laser activation threshold and the solid-state laser rod does not produce the first laser beam for the treatment of the patient tissue (5), thus keeping the solid state laser rod (234) thermally primed,
wherein the solid state laser rod (234) is kept thermally primed while not producing laser pulses for the treatment of the patient tissue (5).

**11.** The laser apparatus (1) of claim 10,
wherein the first laser source (23a) further comprises a flash lamp charger (232) electrically coupled to the flash lamp (233),
wherein the flash lamp (233) is configured to generate the heat flash and the therapeutic flash, when energized by the first flash lamp charger (232), and
wherein the control unit (21) configured to control the flash lamp charger (232) to deliver:
(i) a heat current pulse to the flash lamp (233) configured to energize the flash lamp (233) to generate the heat flash, and
(ii) a therapeutic current pulse to the flash lamp (233) configured to energize the flash lamp (233) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the waveguide (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
a movable waveguide (3) coupled to the collimator (27) on a first side;
an applicator (4) coupled to a second side of the waveguide (3),
wherein the applicator (4) is configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the first laser source (23a) comprises an Er:glass laser rod (234a) configured to generate the first laser beam at approximately 1540 nm; [par. [0089]]
the second laser source (23b) comprises an Er:YAG laser rod (234b) configured to generate the second laser beam at approximately 2940 nm; [par. [0093]]
the first laser source (23a) comprises a first laser chamber (231a) comprising a single flowtube assembly (235) holding the Er:glass laser rod (234a) and a first flash lamp (233a); and the second laser source (23b) comprises a second laser chamber (231b) comprising a multiple flowtube assembly holding the Er:YAG laser rod (234b) and a second flash lamp (233b). [par. [0105], [0107], [0108]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the waveguide (3) are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the waveguide (3) is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21),
wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of nay of the preceding claims,
wherein the multiple flowtube assembly of the second laser chamber (231b) comprises a first flowtube (235a) configured to hold the Er:YAG laser rod (234b) and a second flowtube (235b) configured to hold the second flash lamp (233b),
wherein the first flowtube (235a) comprises a space for a cooling-flow (237) adjacent to the Er:YAG laser rod and the second flowtube (235b) comprises a space for a cooling-flow (237) adjacent to the second flash lamp;
wherein a cavity (238) of the second laser chamber (231b) outside of the multiple flowtube assembly is filled with air, gas, or is evacuated in an amount of 10% to 90% of a volume of the second laser chamber; and
wherein a cavity of the first laser chamber (231a) outside of the single flowtube assembly (235) is filled with air, gas, or is evacuated in an amount of 0.1% to 40% of a volume of the first laser chamber [par. [0105]-[0109]].

**8.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**9.** The laser apparatus (1) of claim 8,
wherein a duration of the overlapping subinterval is in a range of 1% to 90% of the shortest pulse length among the first laser beam pulse and the second laser beam pulse.

**10.** The laser apparatus (1) of anyone of the preceding claims,
wherein the first flash lamp (233a) is configured to produce a broad-spectrum light flash configured to energize the Er:glass laser rod (234a);
wherein the control unit (21) is configured to control the first laser source (23a) such that:
the first flash lamp (233a) energizes the Er:glass laser rod (234a) with a therapeutic flash that is sufficient to pass a laser activation threshold and the Er:glass laser rod (234a) produces the first laser beam for the treatment of the patient tissue (5); and
the first flash lamp (233a) energizes the Er:glass laser rod (234a) with a heat flash that is not sufficient to pass the laser activation threshold and the solid-state laser rod does not produce the first laser beam for the treatment of the patient tissue (5), thus keeping the Er:glass laser rod (234a) thermally primed,
wherein the Er:glass laser rod (234a) is kept thermally primed while not producing laser pulses for the treatment of the patient tissue (5).

**11.** The laser apparatus (1) of claim 10,
wherein the first laser source (23a) further comprises a flash lamp charger (232) electrically coupled to the first flash lamp (233a),
wherein the first flash lamp (233a) is configured to generate the heat flash and the therapeutic flash, when energized by the first flash lamp charger (232), and
wherein the control unit (21) is configured to control the flash lamp charger (232) to deliver:
(i) a heat current pulse to the first flash lamp (233a) configured to energize the first flash lamp (233a) to generate the heat flash, and
(ii) a therapeutic current pulse to the first flash lamp (233a) configured to energize the first flash lamp (233a) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the waveguide (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
a movable waveguide (3) coupled to the collimator (27) on a first side;
an applicator (4) coupled to a second side of the waveguide (3),
wherein the applicator (4) is configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the first laser source (23a) comprises a first laser chamber (231a) and the second laser source (23b) comprises a second laser chamber (231b), and wherein a laser chamber cavity (238) of at least one of the first laser chamber (231a) and the second laser chamber (231b) is made of polytetrafluoroethylene (PTFE) (236) or is coated with PTFE (236). [par. [0024], [0077], [0078], [0090], [0098]]

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the waveguide (3) are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the waveguide (3) is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21),
wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of anyone of the preceding claims,
wherein the first laser source (23a) is configured to generate a non-ablative laser beam in a range of 1000 nm to 1800 nm.

**8.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**9.** The laser apparatus (1) of claim 8,
wherein a duration of the overlapping subinterval is in a range of 1% to 90% of the shortest pulse length among the first laser beam pulse and the second laser beam pulse.

**10.** The laser apparatus (1) of anyone of the preceding claims,
wherein the first laser source (23a) comprises:
a solid state laser rod (234) configured to generate a laser beam; and
a flash lamp (233) configured to produce a broad-spectrum light flash configured to energize the solid state laser rod (234);
wherein the control unit (21) is configured to control the first laser source (23a) such that:
the flash lamp (233) energizes the solid state laser rod (234) with a therapeutic flash that is sufficient to pass a laser activation threshold and the solid state laser rod (234) produces the first laser beam for the treatment of the patient tissue (5); and
the flash lamp (233) energizes the solid state laser rod (234) with a heat flash that is not sufficient to pass the laser activation threshold and the solid-state laser rod does not produce the first laser beam for the treatment of the patient tissue (5), thus keeping the solid state laser rod (234) thermally primed,
wherein the solid state laser rod (234) is kept thermally primed while not producing laser pulses for the treatment of the patient tissue (5).

**11.** The laser apparatus (1) of claim 10,
wherein the first laser source (23a) further comprises a flash lamp charger (232) electrically coupled to the flash lamp (233),
wherein the flash lamp (233) is configured to generate the heat flash and the therapeutic flash, when energized by the first flash lamp charger (232), and
wherein the control unit (21) configured to control the flash lamp charger (232) to deliver:
(i) a heat current pulse to the flash lamp (233) configured to energize the flash lamp (233) to generate the heat flash, and
(ii) a therapeutic current pulse to the flash lamp (233) configured to energize the flash lamp (233) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the waveguide (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]

**1.** A laser apparatus (1) for treatment of patient tissue (5), comprising:
a plurality of laser sources (23) for generating a plurality of laser beams, the plurality of laser sources comprising:
a first laser source (23a) configured to generate a first laser beam of a first wavelength configured to provide non-ablation of a tissue of the patient; and
a second laser source (23b) configured to generate a second laser beam of a second wavelength configured to provide ablation of the tissue of the patient,
wherein the first wavelength is different than the second wavelength, and
wherein each laser source of the plurality of the laser sources (23) is configured to generate a pulsed laser beam;
a laser beam combiner (26) coupled to the plurality of the laser sources (23), said laser beam combiner (26) being configured to combine the plurality of laser beams of the predetermined wavelengths into a single combined laser beam of multiple wavelengths;
a collimator (27), configured to collimate the plurality of laser beams of the single combined laser beam;
a movable waveguide (3) coupled to the collimator (27) on a first side;
an applicator (4) coupled to a second side of the waveguide (3),
wherein the applicator (4) is configured to deliver the single combined laser beam to a target zone of the patient tissue (5);
wherein the collimator (27), the laser beam combiner (26) and the applicator (4) are configured such that the plurality of the laser beams in the single combined laser beam:
irradiates the same area of the target zone, or
irradiates the target zone such that the centers of the plurality of laser beams are aligned to a common point on the target zone and their cross-sections areas overlaps on the target zone; and
a control unit (21) in communication with the plurality of laser sources (23), said control unit being configured to control the plurality of laser sources (23) in a manner that the plurality of laser beams combined into the single combined laser beam are delivered to the target zone of tissue (5) in a simultaneous or overlapping method,
wherein the delivery of the plurality of laser beams in the simultaneous or overlapping method facilitates a combined effect of the effects of each individual laser beam of the plurality of laser beams, **characterized in that** the first laser source (23a) comprises an Er:glass laser rod (234a) configured to generate the first laser beam at approximately 1540 nm; [par. [0089]]
the second laser source (23b) comprises an Er:YAG laser rod (234b) configured to generate the second laser beam at approximately 2940 nm; [par. [0093]]
the first laser source (23a) comprises a first laser chamber (231a) comprising a single flowtube assembly (235) holding the Er:glass laser rod (234a) and a first flash lamp (233a);
the second laser source (23b) comprises a second laser chamber (231b) comprising a multiple flowtube assembly holding the Er:YAG laser rod (234b) and a second flash lamp (233b) [par. [0105], [0107], [0108]],
wherein a laser chamber cavity (238) of at least one of the first laser chamber (231a) and the second laser chamber (231b) is made of polytetrafluoroethylene (PTFE) (236) or is coated with PTFE (236); [par. [0077], [0096]]
wherein the apparatus (1) further comprises an articulated arm (3) coupled to the collimator (27) on a first side and to the applicator (4) on a second side; [par. [0027], [0194], [0223]]
wherein the articulated arm (3) comprises 5 to 10 joints, wherein each joint comprises a 45 degree mirror, and wherein the articulated arm (3) has a length in a range of 1 m to 2.5 m and is configured to deliver the single combined laser beam to the applicator (4); and [par. [0221], [0223]]
wherein the collimator (27) is a single collimator configured to collimate all laser beams from all laser sources simultaneously, and an axial displacement of each laser source (23a, 23b) from the single collimator (27) is parametrically determined by the product of a beam propagation ratio (M²) and a waist radius of the respective laser source (23a, 23b)

**2.** The laser apparatus (1) of claim 1,
wherein the applicator (4) further comprises a fractional element (44) configured to split the single combined laser beam delivered to the applicator (4) into a predetermined fractional pattern of spatially separated micro beams, and
wherein the fractional element (44) comprises a micro-lens element (441) configured to split the single combined laser beam into a matrix of micro beams in the range of 3 x 3 to 30 x 30,
wherein the micro beams create micro thermal zones on impact with the patient tissue (5) having their centers apart in a range of 500 µm to 2000 µm, and wherein each micro thermal zone has a width in a range of 100 µm to 1500 µm, and
wherein the target zone has a size in the range of 1 mm² to 25 cm².

**3.** The laser apparatus (1) of anyone of the preceding claims, wherein the applicator (4) further comprises an attachment point (45) for a vacuum pump system configured to facilitate continuous removal of ablated tissue (5) debris [par. [0343]].

**4.** The laser apparatus (1) of one of the preceding claims, wherein the collimator (27) is a single collimator configured to align the plurality of laser beams inside the single combined laser beam such that the differences in their radii at the end of the waveguide (3) are in a range of 0.2 % to 25 %.

**5.** The laser apparatus (1) of anyone of the preceding claims,
wherein optical distances from the plurality of laser sources (23) to the collimator (27) are adjusted such that the differences in beam radii at the output of the waveguide (3) is in a range of 0.3% to 20%,
wherein the optical distance from each laser source to the collimator (27) is in a range of 200 mm to 700 mm, and
wherein the optical distance from the first laser source (23a) to the collimator (27) is shorter by an amount in a range of 1 mm to 100 mm relative to the optical distance from the second laser source (23b) to the collimator (27).

**6.** The laser apparatus (1) of anyone of the preceding claims,
further comprising a user interface (22) in communication with the control unit (21),
wherein the user interface (22) is configured to allow an operator of the laser apparatus to select a treatment protocol and to adjust at least one treatment parameter of each laser source independently, the treatment parameter comprising at least one of pulse duration, energy level, and a fluence; and
wherein the treatment protocol is configured for improvement of a visual appearance of a patient.

**7.** The laser apparatus (1) of nay of the preceding claims,
wherein the multiple flowtube assembly of the second laser chamber (231b) comprises a first flowtube (235a) configured to hold the Er:YAG laser rod (234b) and a second flowtube (235b) configured to hold the second flash lamp (233b),
wherein the first flowtube (235a) comprises a space for a cooling-flow (237) adjacent to the Er:YAG laser rod and the second flowtube (235b) comprises a space for a cooling-flow (237) adjacent to the second flash lamp;
wherein a cavity (238) of the second laser chamber (231b) outside of the multiple flowtube assembly is filled with air, gas, or is evacuated in an amount of 10% to 90% of a volume of the second laser chamber; and
wherein a cavity of the first laser chamber (231a) outside of the single flowtube assembly (235) is filled with air, gas, or is evacuated in an amount of 0.1% to 40% of a volume of the first laser chamber [par. [0105]-[0109]].

**8.** The laser apparatus (1) of anyone of the preceding claims,
wherein the overlapping method comprises operating the first laser source (23a) to generate the first laser beam pulse during a first time interval and operating the second laser source (23b) to generate the second laser beam pulse during a second time interval that overlaps with at least a portion of the first time interval, and
wherein both the first and second laser beams are simultaneously active during an overlapping subinterval of said overlapping method time interval.

**9.** The laser apparatus (1) of claim 8,
wherein a duration of the overlapping subinterval is in a range of 1% to 90% of the shortest pulse length among the first laser beam pulse and the second laser beam pulse.

**10.** The laser apparatus (1) of anyone of the preceding claims,
wherein the first flash lamp (233a) is configured to produce a broad-spectrum light flash configured to energize the Er:glass laser rod (234a);
wherein the control unit (21) is configured to control the first laser source (23a) such that:
the first flash lamp (233a) energizes the Er:glass laser rod (234a) with a therapeutic flash that is sufficient to pass a laser activation threshold and the Er:glass laser rod (234a) produces the first laser beam for the treatment of the patient tissue (5); and
the first flash lamp (233a) energizes the Er:glass laser rod (234a) with a heat flash that is not sufficient to pass the laser activation threshold and the solid-state laser rod does not produce the first laser beam for the treatment of the patient tissue (5), thus keeping the Er:glass laser rod (234a) thermally primed,
wherein the Er:glass laser rod (234a) is kept thermally primed while not producing laser pulses for the treatment of the patient tissue (5).

**11.** The laser apparatus (1) of claim 10,
wherein the first laser source (23a) further comprises a flash lamp charger (232) electrically coupled to the first flash lamp (233a),
wherein the first flash lamp (233a) is configured to generate the heat flash and the therapeutic flash, when energized by the first flash lamp charger (232), and
wherein the control unit (21) is configured to control the flash lamp charger (232) to deliver:
(i) a heat current pulse to the first flash lamp (233a) configured to energize the first flash lamp (233a) to generate the heat flash, and
(ii) a therapeutic current pulse to the first flash lamp (233a) configured to energize the first flash lamp (233a) to generate the therapeutic flash,
wherein the control unit (21) is configured to control the flash lamp charger (232) such that a plurality of heat current pulses precede one or more therapeutic current pulses.

**12.** The laser apparatus (1) of claim 11, wherein a frequency of heat current pulses is in a range of 1 Hz to 200 Hz.

**13.** The laser apparatus (1) of any one of the preceding claims, wherein a quick-connect system is used between a distal end of the waveguide (3) and the applicator (4) [par. [0228]],
wherein the applicator (4) is equipped with an integrated printed circuit board (PCB) or flexible circuit connected to the control unit (21) using a dedicated data connection [par. [0340]],
wherein the integrated circuit is designed to communicate the specific type of applicator (4) that is connected to the control unit (21), and wherein the control unit (21) is configured to utilize the received data to suggest optimized treatment protocols tailored to the connected applicator (4) [par. [0341]].

**14.** The laser apparatus (1) of any one of the preceding claims, wherein the applicator (4) comprises an aperture to change the shape of the laser beam entering the applicator (4) [par. [0278]], and wherein the aperture has shape of a square, triangle, rectangle, ellipse, trapezoid, polygon, star, or semicircle.

**15.** The laser apparatus (1) of claim 14, wherein the apparatus further comprises a feedback system configured such that the control unit (21) adjusts the treatment parameters for each laser source (23a, 23b) based on a transmissivity of the aperture for the given laser source, so that resulting parameters of the laser beam interacting with the patient tissue (5) are in accordance with the parameters set by an operator on the user interface (22). [par. [0279]]
